(19) Europäisches Patentamt
European Patent Office
Office européen des brevets

(11) EP 0 761 219 A1

# (12) EUROPEAN PATENT APPLICATION

(43) Date of publication:
**12.03.1997 Bulletin 1997/11**

(51) Int Cl.6: **A61K 31/445**, A61K 31/495

(21) Application number: **96305917.5**

(22) Date of filing: **14.08.1996**

(84) Designated Contracting States:
**AT BE CH DE DK ES FI FR GB GR IE IT LI LU NL PT SE**
Designated Extension States:
**AL LT LV SI**

(30) Priority: **21.08.1995 US 2581**

(71) Applicant: **ELI LILLY AND COMPANY**
**Indianapolis, Indiana 46285 (US)**

(72) Inventors:
- **Dodge, Jeffrey Alan**
  **Indianapolis, Indiana 46236 (US)**
- **Hipskind, Philip Arthur**
  **New Palestine, Indiana 46163 (US)**

(74) Representative: **Tapping, Kenneth George et al**
**Lilly Industries Limited**
**European Patent Operations**
**Erl Wood Manor**
**Windlesham Surrey GU20 6PH (GB)**

## (54) 2-Acylaminopropanamines as growth hormone secretagogues

(57) This invention provides a series of substituted propanamines which are useful in treating a physiological condition which may be modulated by an increase in growth hormone. This invention also provides methods for the treatment of such physiological conditions which comprise administering a growth hormone secretagogue as described in the present invention in combination with growth hormone releasing hormone.

EP 0 761 219 A1

## Description

Growth hormone is a secretory protein of the pituitary gland of animals having wide ranging developmental effects on the organism. Artificial manipulation of growth hormone levels has been demonstrated to have significant therapeutic utility. Human growth hormone supplementation has been shown to be an effective treatment for growth hormone deficiencies and their related disease states in humans. Apart from this application, studies have uncovered new and significant properties of growth hormone which lend further importance to the ability to control growth hormone levels. For example, recent clinical studies indicate that growth hormone supplementation may be useful in combating the maladies of aging in humans. Elevated growth hormone levels in animals have been shown to result in increased lean muscle mass. One application of this latter observation could result in higher production of leaner meat products or in the production of larger and/or stronger animals.

While growth hormone is naturally produced by the pituitary gland, the secretion of growth hormone into the bloodstream is controlled by a second protein, Growth Hormone Releasing Factor (GRF). This hormone is also commonly known in the art as somatocrinin, Growth Hormone Releasing Hormone (GHRH), and Growth Releasing Hormone (GRH).

There are two ways to approach the problem of increasing circulating levels of growth hormone: (1) increase the level of human growth hormone in the organism directly or (2) increase the organism's natural tendency to produce growth hormone. The latter strategy may be achieved via supplementation with GRF. GRF has been demonstrated to increase the circulatory levels of growth hormone in vivo. Rivier, et al., Nature (London), 300:276 (1982). The effect of GRF (and various structural analogs thereof) on growth hormone production has been widely studied. A primary obstacle to the use of GRF as a direct supplement is its short lifespan in vivo. L.A. Frohman, et al., Journal of Clinical Investiiation, 78:906 (1986). More potent and/or longer lasting GRF molecules are therefore desirable for the development of effective human therapeutic or animal husbandry agents.

The structure of GRF has been modified in numerous ways resulting in longer lasting and/or more potent GRF analogs. It has been demonstrated that the first 29 amino acids from the N-terminus are sufficient to retain full GRF activity. Speiss, et al., Biochemistry, 21:6037 (1982). One strategy has been the incorporation of novel D-amino acid residues in various regions of the GRF molecule. V.A. Lance, et al., Biochemical and Biophysical Research Communications, 119:265 (1984); D.H. Coy, et al., Peptides, 8(suppl. 1):49 (1986). Another strategy has modified the peptide backbone of GRF by the incorporation of peptide bond isosteres in the N-terminal region. D. Tourwe, Janssen. Chim. Acta, 3:3 (1985); S.J. Hocart, et al., Journal of Medicinal Chemistry, 33:1954-58 (1990). A series of very active analogs of GHRH is described in European Patent Publication 511,003, published October 28, 1992.

In addition to the actions of GHRH there are various ways known to release growth hormone. For example, chemicals such as arginine, L-3,4-dihydroxyphenylalanine (L-DOPA), glucagon, vasopressin, and insulin-induced hypoglycemia, as well as activities such as sleep and exercise, indirectly cause growth hormone to be released from the pituitary by acting in some fashion on the hypothalamus, perhaps either to decrease somatostatin secretion or to increase the secretion of GHRH.

In cases where increased levels of growth hormone are desired, the problem has generally been solved by providing exogenous growth hormone or by administering GHRH, or a related peptidyl compounds which stimulates growth hormone production or release. In either instance the peptidyl nature of the compound has necessitated that it be administered by injection.

Other compounds have been developed which stimulate the release of endogenous growth hormone, such as analogous peptidyl compounds related to GHRH. These peptides, while considerably smaller than growth hormones are still susceptible to metabolic instability.

Administration of the hexapeptide growth hormone releasing peptide-6 (GHRP-6) results in the secretion of growth hormone in many species, including humans. This peptide has the following sequence.

$$\text{His-D-Trp-Ala-Trp-D-Phe-Lys-NH}_2$$

This peptide is one of a series of synthetic peptides, the structures of which were based on the pentapeptide Met-enkephalin. It has been shown that GHRP binds specifically to the pituitary, although the binding does not involve the opioid, GHRH, or the somatostatin receptors.

In recent years significant efforts have been taken to develop nonpeptidyl analogs of this series of compounds. Such compounds, termed growth hormone secretagogues, should be orally bioavailable, induce the production or release of growth hormone, and act synergistically with GHRH.

Representative growth hormone secretagogues are disclosed in United States Patent 3,239,345; United States Patent 4,036,979; United States Patent 4,411,890; United States Patent 5,206,235; United States Patent 5,248,841; United States Patent 5,310,737; United States Patent 5,310,017; European Patent Publication 144,230; European

Patent Publication 513,974; Patent Cooperation Treaty Patent Publication WO 94/07486; Patent Cooperation Treaty Patent Publication WO 94/08583; Patent Cooperation Treaty Patent Publication WO 94/13696; and Science, 260: 1640-1643 (1993), the entire of all of which are herein incorporated by reference.

United States Patent 5,206,235, issued April 27, 1993, describes a series of benzolactam compounds typified by the following structure.

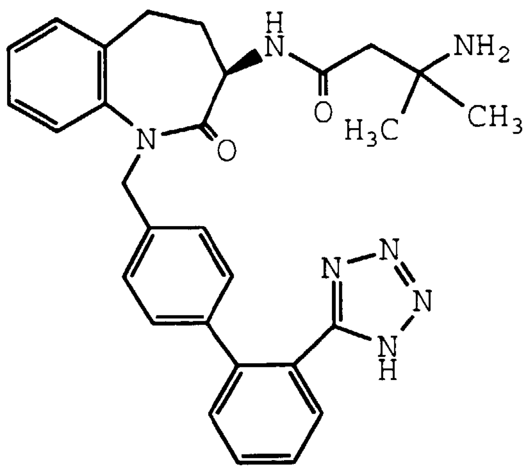

These compounds have demonstrated clinical activity in humans in raising the growth hormone secretory levels. B.J. Gertz, Journal of Clinical Endocrinology and Metabolism, 77:1393-1397 (1993).

A second generation of growth hormone secretagogues is described in Patent Cooperation Treaty Patent Publication WO 94/13696, published June 23, 1994. These compounds are typified by the following two structures.

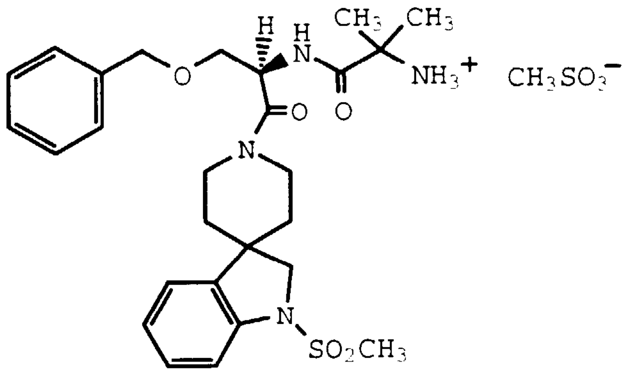

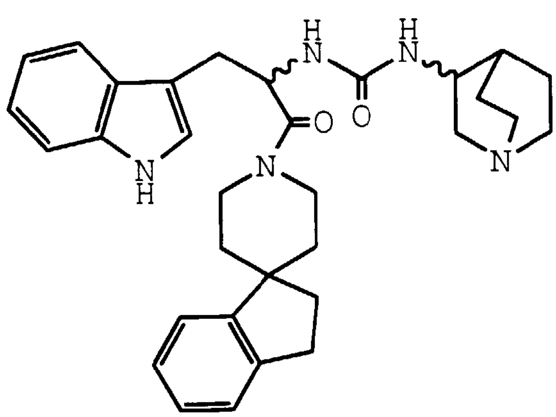

The present invention provides a series of compounds that have activity as growth hormone secretagogues. These compounds are orally active and non-peptidyl in nature and are, therefore, more metabolically stable than growth hormone, growth hormone releasing hormone, or analogs of either of these proteins. The compounds employed in the present invention are preferred for human pharmaceutical uses as well as veterinary uses, particularly in cattle, swine, sheep, poultry and fish.

This invention encompasses methods for the treatment or prevention of a physiological condition which may be modulated by an increase in growth hormone, which method comprises administering to an animal in need of said treatment an effective amount of a compound of Formula I

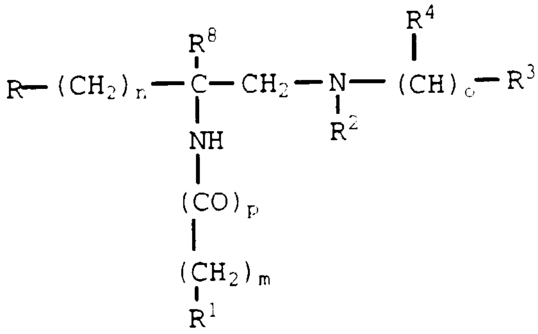

I

wherein

m is 0, 1, 2, or 3;
n is 0 or 1;
o is 0,1, or 2;
p is 0 or 1;
R is phenyl, 2- or 3-indolyl, 2- or 3-indolinyl, benzothienyl, benzofuranyl, or naphthyl;

which groups may be substituted with one or two halo, $C_1$-$C_3$ alkoxy, trifluoromethyl, $C_1$-$C_4$ alkyl, phenyl-$C_1$-$C_3$ alkoxy, or $C_1$-$C_4$ alkanoyl groups;

$R^1$ is trityl, phenyl, diphenylmethyl, phenoxy, phenylthio, piperazinyl, piperidinyl, pyrrolidinyl, morpholinyl, indolinyl, indolyl, benzothienyl, hexamethyleneiminyl, benzofuranyl, tetrahydropyridinyl, quinolinyl, isoquinolinyl, reduced quinolinyl, reduced isoquinolinyl, phenyl-($C_1$-$C_4$ alkyl)-, phenyl-($C_1$-$C_4$ alkoxy)-, quinolinyl-($C_1$-$C_4$ alkyl)-, isoquinolinyl-($C_1$-$C_4$ alkyl)-, reduced quinolinyl-($C_1$-$C_4$ alkyl)-, reduced isoquinolinyl-($C_1$-$C_4$ alkyl)-, benzoyl-($C_1$-$C_3$ alkyl)-, $C_1$-$C_4$ alkyl, or -NH- $CH_2$-$R^5$;

any one of which $R^1$ groups may be substituted with halo, $C_1$-$C_4$ alkyl, $C_1$-$C_4$ alkoxy, trifluoromethyl, amino, $C_1$-$C_4$ alkylamino, di($C_1$-$C_4$ alkyl)amino, or $C_2$-$C_4$ alkanoylamino;
or any one of which $R^1$ groups may be substituted with phenyl, piperazinyl, $C_3$-$C_8$ cycloalkyl, benzyl, $C_1$-$C_4$ alkyl, piperidinyl, pyridinyl, pyrimidinyl, $C_2$-$C_6$ alkanoylamino, pyrrolidinyl, $C_2$-$C_6$ alkanoyl, or $C_1$-$C_4$ alkoxycarbonyl;

any one of which groups may be substituted with halo, $C_1$-$C_4$ alkyl, $C_1$-$C_4$ alkoxy, trifluoromethyl, amino, $C_1$-$C_4$ alkylamino, di($C_1$-$C_4$ alkyl)amino, or $C_2$-$C_4$ alkanoylamino;

or $R^1$ is amino, a leaving group, hydrogen, $C_1$-$C_4$ alkylamino, or di($C_1$-$C_4$ alkyl)amino;
$R^5$ is pyridyl, anilino-($C_1$-$C_3$ alkyl)-, or anilinocarbonyl;
$R^2$ is hydrogen, $C_1$-$C_4$ alkyl, $C_1$-$C_4$ alkylsulfonyl, carboxy-($C_1$-$C_3$ alkyl)-, $C_1$-$C_3$ alkoxycarbonyl-($C_1$-$C_3$ alkyl)-, or -CO-$R^6$;
$R^6$ is hydrogen, $C_1$-$C_4$ alkyl, $C_1$-$C_3$ haloalkyl, phenyl, $C_1$-$C_3$ alkoxy, $C_1$-$C_3$ hydroxyalkyl, amino, $C_1$-$C_4$ alkylamino, di($C_1$-$C_4$ alkyl)amino, or -$(CH_2)_q$-$R^7$;
q is 0 to 3;
$R^7$ is carboxy, $C_1$-$C_4$ alkoxycarbonyl, $C_1$-$C_4$ alkylcarbonyloxy, amino, $C_1$-$C_4$ alkylamino, di($C_1$-$C_4$ alkyl)amino, $C_1$-$C_6$ alkoxycarbonylamino, or
phenoxy, phenylthio, piperazinyl, piperidinyl, pyrrolidinyl, morpholinyl, indolinyl, indolyl, benzothienyl, benzofuranyl, quinolinyl, isoquinolinyl, reduced quinolinyl, reduced isoquinolinyl, phenyl-($C_1$-$C_4$ alkyl)-, quinolinyl-($C_1$-$C_4$ alkyl)-, isoquinolinyl-($C_1$-$C_4$ alkyl)-, reduced quinolinyl-($C_1$-$C_4$ alkyl)-, reduced isoquinolinyl-($C_1$-$C_4$ alkyl)-, benzoyl-

$C_1$-$C_3$ alkyl;

any one of which $R^7$ groups may be substituted with halo, trifluoromethyl, $C_1$-$C_4$ alkoxy, $C_1$-$C_4$ alkyl, amino, $C_1$-$C_4$ alkylamino, di($C_1$-$C_4$ alkyl)amino, or $C_2$-$C_4$ alkanoylamino;

or any one of which $R^7$ groups may be substituted with phenyl, piperazinyl, $C_3$-$C_8$ cycloalkyl, benzyl, piperidinyl, pyridinyl, pyrimidinyl, pyrrolidinyl, $C_2$-$C_6$ alkanoyl, or $C_1$-$C_4$ alkoxycarbonyl;

any of which groups may be substituted with halo, trifluoromethyl, amino, $C_1$-$C_4$ alkoxy, $C_1$-$C_4$ alkyl, $C_1$-$C_4$ alkylamino, di($C_1$-$C_4$ alkyl)amino, or $C_2$-$C_4$ alkanoylamino;

$R^8$ is hydrogen or $C_1$-$C_6$ alkyl;
$R^3$ is phenyl, phenyl-($C_1$-$C_6$ alkyl)-, $C_3$-$C_8$ cycloalkyl, $C_5$-$C_8$ cycloalkenyl, $C_1$-$C_8$ alkyl, naphthyl, $C_2$-$C_8$ alkenyl, or hydrogen;

any one of which groups except hydrogen may be substituted with one or two halo, $C_1$-$C_3$ alkoxy, $C_1$-$C_3$ alkylthio, nitro, trifluoromethyl, or $C_1$-$C_3$ alkyl groups; and

$R^4$ is hydrogen or $C_1$-$C_3$ alkyl;
with the proviso that if $R^1$ is hydrogen or halo, $R^3$ is phenyl, phenyl-($C_1$-$C_6$ alkyl)-, $C_3$-$C_8$ cycloalkyl, $C_5$-$C_8$ cycloalkenyl, or naphthyl;
with the proviso that if $R^1$ is hydrogen or halo, $R^3$ is phenyl, phenyl-($C_1$-$C_6$ alkyl)-, $C_3$-$C_8$ cycloalkyl, $C_5$-$C_8$ cycloalkenyl, or naphthyl; or a pharmaceutically acceptable salt thereof.

In another embodiment, this invention encompasses the novel compounds of Formula I and the pharmaceutically acceptable salts, solvates, and prodrugs thereof, as well as pharmaceutical formulations comprising, as an active ingredient, a compound of Formula I in combination with a pharmaceutically acceptable carrier, diluent or excipient. This invention also encompasses novel processes for the synthesis of the compounds of Formula I.

All temperatures stated herein are in degrees Celsius (°C). All units of measurement employed herein are in weight units except for liquids which are in volume units.

As used herein, the term "$C_1$-$C_6$ alkyl" refers to straight or branched, monovalent, saturated aliphatic chains of 1 to 6 carbon atoms and includes, but is not limited to, methyl, ethyl, propyl, isopropyl, butyl, isobutyl, t-butyl, pentyl, isopentyl, and hexyl. The term "$C_1$-$C_6$ alkyl" includes within its definition the term "$C_1$-$C_4$ alkyl".

"Divalent($C_1$-$C_4$)alkyl" represents a straight or branched divalent saturated aliphatic chain having from one to four carbon atoms. Typical divalent($C_1$-$C_4$)alkyl groups include methylene, ethylene, propylene, 2-methylpropylene, butylene and the like.

"Halo" represents chloro, fluoro, bromo or iodo.

"Halo($C_1$-$C_4$)alkyl" represents a straight or branched alkyl chain having from one to four carbon atoms with 1, 2 or 3 halogen atoms attached to it. Typical halo($C_1$-$C_4$)alkyl groups include chloromethyl, 2-bromoethyl, 1-chloroisopropyl, 3-fluoropropyl, 2,3-dibromobutyl, 3-chloroisobutyl, iodo-*t*-butyl, trifluoromethyl and the like.

"Hydroxy($C_1$-$C_4$)alkyl" represents a straight or branched alkyl chain having from one to four carbon atoms with hydroxy group attached to it. Typical hydroxy($C_1$-$C_4$)alkyl groups include hydroxymethyl, 2-hydroxyethyl, 1-hydroxyisopropyl, 2-hydroxypropyl, 2-hydroxybutyl, 3-hydroxyisobutyl, hydroxy-*t*-butyl and the like.

"$C_1$-$C_6$ alkylthio" represents a straight or branched alkyl chain having from one to six carbon atoms attached to a sulfur atom. Typical $C_1$-$C_6$ alkylthio groups include methylthio, ethylthio, propylthio, isopropylthio, butylthio and the like. The term "$C_1$-$C_6$ alkylthio" includes within its definition the term "$C_1$-$C_4$ alkylthio".

The term "$C_2$-$C_8$ alkenyl" as used herein represents a straight or branched, monovalent, unsaturated aliphatic chain having from two to eight carbon atoms. Typical $C_2$-$C_6$ alkenyl groups include ethenyl (also known as vinyl), 1-methylethenyl, 1-methyl-1-propenyl, 1-butenyl, 1-hexenyl, 2-methyl-2-propenyl, 1-propenyl, 2-propenyl, 2-butenyl, 2-pentenyl, and the like.

"$C_5$-$C_8$ cycloalkenyl" represents a hydrocarbon ring structure containing from five to eight carbon atoms and having at least one double bond within that ring, which is unsubstituted or substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or $-(CH_2)_a-R^c$ where a is 1, 2, 3 or 4 and $R^c$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino.

"$C_1$-$C_4$ alkylamino" represents a straight or branched alkylamino chain having from one to four carbon atoms attached to an amino group. Typical $C_1$-$C_4$ alkyl-amino groups include methylamino, ethylamino, propylamino, isopropylamino, butylamino, *sec*-butylamino and the like.

"Di($C_1$-$C_4$ alkyl)amino" represents a straight or branched dialkylamino chain having two alkyl chains, each having independently from one to four carbon atoms attached to a common amino group. Typical di($C_1$-$C_4$)alkylamino groups include dimethylamino, ethylmethylamino, methylisopropylamino, *t*-butylisopropylanfino, di-*t*-butylamino and the like.

"Arylsulfonyl" represents an aryl moiety attached to a sulfonyl group. "Aryl" as used in this term represents a phenyl,

naphthyl, heterocycle, or unsaturated heterocycle moiety which is optionally substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or -$(CH_2)_a$-$R^b$ where a is 1, 2, 3 or 4; and $R^b$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$) alkylamino.

The term "heterocycle" represents an unsubstituted or substituted stable 5- to 7-membered monocyclic or 7- to 10-membered bicyclic heterocyclic ring which is saturated and which consists of carbon atoms and from one to three heteroatoms selected from the group consisting of nitrogen, oxygen or sulfur, and wherein the nitrogen and sulfur heteroatoms may optionally be oxidized, and the nitrogen heteroatom may optionally be quaternized and including a bicyclic group in which any of the above-defined heterocyclic rings is fused to a benzene ring. The heterocyclic ring may be attached at any heteroatom or carbon atom which affords a stable structure. The hetero-cycle is unsubstituted or substituted with 1, 2 or 3 substituents independently selected from halo, halo($C_1$-$C_4$)-alkyl, $C_1$-$C_4$ alkyl, $C_1$-$C_4$ alkoxy, carboxy, $C_1$-$C_4$ alkoxy-carbonyl, carbamoyl, N-($C_1$-$C_4$)-alkylcarbamoyl, amino, $C_1$-$C_4$ alkylamino, di($C_1$-$C_4$)alkylamino or -$(CH_2)_a$-$R^d$ where a is 1, 2, 3 or 4; and $R^d$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino.

The term "unsaturated heterocycle" represents an unsubstituted or substituted stable 5- to 7-membered monocyclic or 7- to 10-membered bicyclic heterocyclic ring which has one or more double bonds and which consists of carbon atoms and from one to three heteroatoms selected from the group consisting of nitrogen, oxygen or sulfur, and wherein the nitrogen and sulfur heteroatoms may optionally be oxidized, and the nitrogen heteroatom may optionally be quaternized and including a bicyclic group in which any of the above-defined heterocyclic rings is fused to a benzene ring. The unsaturated heterocyclic ring may be attached at any heteroatom or carbon atom which affords a stable structure. The unsaturated heterocycle is unsubstituted or substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or -$(CH_2)_a$-$R^e$ where a is 1, 2, 3 or 4; and $R^e$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino.

Examples of such heterocycles and unsaturated heterocycles include piperidinyl, piperazinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazolyl, benzopyranyl, benzothiazolyl, benzoazolyl, furyl, tetrahydrofuryl, tetrahydropyranyl, thienyl, benzothienyl, thiamorpholinyl, thiamorpholinyl-sulfoxide, thiamorpholinylsulfone, oxadiazolyl, triazolyl, tetrahydroquinolinyl, tetrahydrisoquinolinyl, 3-methylimidazolyl, 3-methoxypyridyl, 4-chloroquinolinyl, 4-aminothiazolyl, 8-methylquinolinyl, 6-chloroquinoxalinyl, 3-ethylpyridyl, 6-methoxybenzimidazolyl, 4-hydroxyfuryl, 4-methylisoquinolinyl, 6,8-dibromoquinolinyl, 4,8-dimethyl-naphthyl, 2-methyl-1,2,3,4-tetrahydroisoquinolinyl, N-methyl-quinolin-2-yl, 2-*t*-butoxycarbonyl-1,2,3,4-isoquinolin-7-yl and the like.

"$C_1$-$C_6$ alkoxy" represents a straight or branched alkyl chain having from one to six carbon atoms attached to an oxygen atom. Typical $C_1$-$C_6$ alkoxy groups include methoxy, ethoxy, propoxy, isopropoxy, butoxy, *t*-butoxy, pentoxy and the like. The term "$C_1$-$C_6$ alkoxy" includes within its definition the term "$C_1$-$C_4$ alkoxy".

"$C_2$-$C_6$ alkanoyl" represents a straight or branched alkyl chain having from one to five carbon atoms attached to a carbonyl moiety. Typical $C_2$-$C_6$ alkanoyl groups include ethanoyl, propanoyl, isopropanoyl, butanoyl, *t*-butanoyl, pentanoyl, hexanoyl, 3-methylpentanoyl and the like.

"$C_1$-$C_4$ alkoxycarbonyl" represents a straight or branched alkoxy chain having from one to four carbon atoms attached to a carbonyl moiety. Typical $C_1$-$C_4$ alkoxy-carbonyl groups include methoxycarbonyl, ethoxycarbonyl, propoxycarbonyl, isopropoxycarbonyl, butoxycarbonyl, *t*-butoxycarbonyl and the like.

"$C_3$-$C_8$ cycloalkyl" represents a saturated hydrocarbon ring structure containing from three to eight carbon atoms which is unsubstituted or substituted with 1, 2 or 3 substituents independently selected from halo, halo($C_1$-$C_4$)alkyl, $C_1$-$C_4$ alkyl, $C_1$-$C_4$ alkoxy, carboxy, $C_1$-$C_4$ alkoxycarbonyl, carbamoyl, N-($C_1$-$C_4$)alkylcarbamoyl, amino, $C_1$-$C_4$ alkylamino, di($C_1$-$C_4$)alkylamino or -$(CH_2)_a$-$R^f$ where a is 1, 2, 3 or 4 and $R^f$ is hydroxy, $C_1$-$C_4$ alkoxy, carboxy, $C_1$-$C_4$ alkoxycarbonyl, amino, carbamoyl, $C_1$-$C_4$ alkylamino or di($C_1$-$C_4$)alkylamino. Typical $C_3$-$C_8$ cycloalkyl groups include cyclopropyl, cyclopentyl, cyclohexyl, cycloheptyl, 3-methyl-cyclopentyl, 4-ethoxycyclohexyl, 4-carboxycycloheptyl, 2-chlorocyclohexyl, cyclobutyl, cyclooctyl, and the like.

The term "amino-protecting group" as used in the specification refers to substituents of the amino group commonly employed to block or protect the amino functionality while reacting other functional groups on the compound. Examples of such amino-protecting groups include formyl, trityl, phthalimido, trichloroacetyl, chloroacetyl, bromoacetyl, iodoacetyl, and urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, *t*-butoxycarbonyl, 1,1-diphenyleth-1-yloxycarbonyl, 1,1-diphenylprop-1-yloxycarbonyl, 2-phenylprop-2-yloxycarbonyl, 2-(*p*-toluyl)-prop-2-yloxycarbonyl, cyclopentanyloxycarbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfonyl)-ethoxycarbonyl, 2-(methylsulfonyl)ethoxycarbonyl, 2-(triphenylphosphino)-ethoxycarbonyl, fluorenylmethoxy-carbonyl ("FMOC"), 2-(trimethylsilyl )ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decyloxy)benzyloxycarbonyl, isobornyloxycarbonyl, 1-piperidyloxycarbonyl and the like; benzoylmethylsulfonyl group, 2-nitrophenylsulfenyl, diphenylphosphine oxide and like amino-protecting groups. The species of amino-protecting group employed is usually not critical so long as the derivatized amino group is stable to the condition of subsequent reactions on other positions of the intermediate molecule and can be selectively removed at the appropriate point without disrupting the remainder of the molecule including any other amino-protecting groups. Preferred amino-protecting groups are trityl, *t*-butoxycarbonyl (*t*-BOC), allyloxycarbonyl and benzyloxycarbonyl. Further examples of groups referred to by the above terms are described by E. Haslam, "Protective Groups in Organic Chemistry", (J.G.W. McOmie, ed., 1973), at Chapter 2; and T.W. Greene and P.G.M. Wuts, "Protective Groups in Organic Synthesis" (1991), at Chapter 7.

The term "carboxy-protecting group" as used in the specification refers to substituents of the carboxy group commonly employed to block or protect the carboxy functionality while reacting other functional groups on the compound. Examples of such carboxy-protecting groups include methyl, *p*-nitrobenzyl, *p*-methylbenzyl, *p*-methoxy-benzyl, 3,4-dimethoxybenzyl, 2,4-dimethoxybenzyl, 2,4,6-trimethoxybenzyl, 2,4,6-trimethylbenzyl, pentamethylbenzyl, 3,4-methylene-dioxybenzyl, benzhydryl, 4,4'-dimethoxy-benzhydryl, 2,2',4,4'-tetramethoxybenzhydryl, *t*-butyl, *t*-amyl, trityl, 4-methoxytrityl, 4,4'-dimethoxytrityl, 4,4',4''-trimethoxytrityl, 2-phenylprop-2-yl, trimethylsilyl, *t*-butyldimethylsilyl, phenacyl, 2,2,2-trichloroethyl, 2-(di(n-butyl)methylsilyl)ethyl, *p*-toluenesulfonylethyl, 4-nitrobenzylsulfonylethyl, allyl, cinnamyl, 1-(trimethylsilylmethyl)prop-1-en-3-yl and like moieties. Preferred carboxy-protecting groups are allyl, benzyl and *t*-butyl. Further examples of these groups are found in E. Haslam, supra, at Chapter 5, and T.W. Greene, et al., supra, at Chapter 5.

The term "leaving group" as used herein refers to a group of atoms that is displaced from a carbon atom by the attack of a nucleophile in a nucleophilic substitution reaction. The term "leaving group" as used in this document encompasses, but is not limited to, activating groups.

The term "activating group" as used herein refers a leaving group which, when taken with the carbonyl (-C=O) group to which it is attached, is more likely to take part in an acylation reaction than would be the case if the group were not present, as in the free acid. Such activating groups are well-known to those skilled in the art and may be, for example, succinimidoxy, phthalimidoxy, benzotriazolyloxy, benzenesulfonyloxy, methanesulfonyloxy, toluenesulfonyloxy, azido, or -O-CO-($C_4$-$C_7$ alkyl).

The compounds used in the method of the present invention have multiple asymmetric centers. As a consequence of these chiral centers, the compounds of the present invention occur as racemates, mixtures of enantiomers and as individual enantiomers, as well as diastereomers and mixtures of diastereomers. All asymmetric forms, individual isomers and combinations thereof, are within the scope of the present invention.

The terms "R" and "S" are used herein as commonly used in organic chemistry to denote specific configuration of a chiral center. The term "R" (*rectus*) refers to that configuration of a chiral center with a clockwise relationship of group priorities (highest to second lowest) when viewed along the bond toward the lowest priority group. The term "S" *(sinister)* refers to that configuration of a chiral center with a counterclockwise relationship of group priorities (highest to second lowest) when viewed along the bond toward the lowest priority group. The priority of groups is based upon their atomic number (in order of decreasing atomic number). A partial list of priorities and a discussion of stereochemistry is contained in "Nomenclature of Organic Compounds: Principles and Practice", (J.H. Fletcher, et al., eds., 1974) at pages 103-120.

In addition to the (R)-(S) system, the older D-L system is also used in this document to denote absolute configuration, especially with reference to amino acids. In this system a Fischer projection formula is oriented so that the number 1 carbon of the main chain is at the top. The prefix "D" is used to represent the absolute configuration of the isomer in which the functional (determining) group is on the right side of the carbon atom at the chiral center and "L", that of the isomer in which it is on the left.

As noted supra, this invention encompasses methods employing the pharmaceutically acceptable salts of the compounds defined by Formula I. A compound of this invention can possess a sufficiently acidic, a sufficiently basic, or both functional groups, and accordingly react with any of a number of organic and inorganic bases, and inorganic and organic acids, to form a pharmaceutically acceptable salt.

The term "pharmaceutically acceptable salt" as used herein, refers to salts of the compounds of the above formula which are substantially non-toxic to living organisms. Typical pharmaceutically acceptable salts include those salts prepared by reaction of the compounds of the present invention with a pharmaceutically acceptable mineral or organic acid or an organic or inorganic base. Such salts are known as acid addition and base addition salts.

Acids commonly employed to form acid addition salts are inorganic acids such as hydrochloric acid, hydrobromic acid, hydroiodic acid, sulfuric acid, phosphoric acid, and the like, and organic acids such as *p*-toluenesulfonic, methanesulfonic acid, oxalic acid, *p*-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprylate, acrylate, formate, hydrochloride, dihydrochloride, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, $\gamma$-hydroxybutyrate, glycolate, tartrate, methanesulfonate, propanesulfonate, naphthalene-1-sulfonate, napththalene-2-sulfonate, mandelate and the like. Preferred pharmaceutically acceptable acid addition salts are those formed with mineral acids such as hydrochloric acid and hydrobromic acid, and those formed with organic acids such as maleic acid and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counterion forming a part of any salt of this invention is usually not of a critical nature, so long as the salt as a whole is pharmacologically acceptable and as long as the counterion does not contribute undesired qualities to the salt as a whole.

This invention further encompasses methods employing the pharmaceutically acceptable solvates of the compounds of Formulas I. Many of the Formula I compounds can combine with solvents such as water, methanol, ethanol and acetonitrile to form pharmaceutically acceptable solvates such as the corresponding hydrate, methanolate, ethanolate and acetonitrilate.

The especially preferred compounds used in the methods of this invention are those of Formula I wherein

a) R is substituted or unsubstituted 2- or 3-indolyl, phenyl, or naphthyl;
b) n is 1;
c) $R^1$ is phenyl, substituted phenyl, piperidinyl, substituted piperidinyl, piperazinyl, substituted piperazinyl, pyrrolidinyl, pyridyl, benzoyl, or morpholinyl;
d) $R^2$ is -CO-$R^6$, $C_1$-$C_4$ alkylsulfonyl, or $C_1$-$C_3$ alkoxycarbonyl-($C_1$-$C_3$ alkyl)-;
e) $R^3$ is phenyl, substituted phenyl, $C_3$-$C_8$ cycloalkyl, substituted $C_3$-$C_8$ cycloalkyl, naphthyl or substituted naphthyl; and
f) $R^8$ is hydrogen or methyl.

A most preferred group of compounds used in the methods of this invention are those of Formula I wherein R is optionally substituted indolyl, $R^1$ is substituted piperidinyl or substituted piperazinyl, $R^8$ is hydrogen, and $R^2$ is acetyl or methylsulfonyl. Another preferred group of compounds used in the methods of this invention are those of Formula I wherein R is naphthyl, $R^1$ is optionally substituted phenyl, substituted piperidinyl or substituted piperazinyl, $R^2$ is acetyl or methylsulfonyl, and $R^3$ is phenyl or substituted phenyl.

The especially preferred compounds of this invention are those of Formula I wherein

a) R is substituted or unsubstituted 2- or 3-indolyl, phenyl, or naphthyl;
b) n is 1;
c) $R^1$ is trityl, phenyl, substituted phenyl, piperidinyl, substituted piperidinyl, piperazinyl, substituted piperazinyl, pyrrolidinyl, pyridyl, benzoyl, or morpholinyl;
d) $R^2$ is -CO-$R^6$, $C_1$-$C_4$ alkylsulfonyl, or $C_1$-$C_3$ alkoxycarbonyl-($C_1$-$C_3$ alkyl)-;
e) $R^3$ is phenyl, substituted phenyl, $C_3$-$C_8$ cycloalkyl, substituted $C_3$-$C_8$ cycloalkyl, naphthyl or substituted naphthyl; and
f) $R^8$ is hydrogen or methyl.

The single most preferred compound employed in the methods of the present invention is (R)-3-(1H-indol-3-yl)-1-[N-(2-methoxybenzyl)acetylamino]-2-[N-(2-(4-(piperidin-1-yl)piperidin-1-yl)acetyl)amino]propane dihydrochloride trihydrate

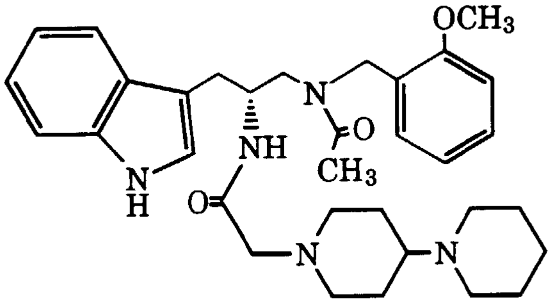

· 2 HCl

· 3 $H_2O$

the synthesis of which is described in United States Patent 5,530,009, issued June 25, 1996, the entire contents of which are herein incorporated by reference.

The compounds of the present invention can be prepared by a variety of procedures well known to those of ordinary skill in the art. The particular order of steps required to produce the compounds of Formula I is dependent upon the particular compound being synthesized, the starting compound, and the relative lability of the substituted moieties.

Examples of such protocols are depicted in Schemes I through IV. The coupling of the substituted amine to the compound of Formula II (**Method A**) can be performed by many means known in the art, the particular methods employed being dependent upon the particular compound of Formula II which is used as the starting material and the type of substituted amine used in the coupling reaction. These coupling reactions frequently employ commonly used coupling reagents such as 1,1-carbonyl diimidazole, dicyclohexylcarbodiimide, diethyl azodicarboxylate, 1-hydroxybenzotriazole, alkyl chloroformate and triethylamine, phenyldichlorophosphate, and chlorosulfonyl isocyanate. Examples of these methods are described infra. After deprotection of the amino group, the compounds of Formula III are obtained.

The compound of Formula III is then reduced, converting the amide into an amine **(Method B).** Amides can be reduced to amines using procedures well known in the art. These reductions can be performed using lithium aluminum hydride as well as by use of many other different aluminum-based hydrides. Alternatively, the amides can be reduced by catalytic hydrogenation, though high temperatures and pressures are usually required for this. Sodium borohydride in combination with other reagents may be used to reduce the amide. Borane complexes, such as a borane dimethylsulfide complex, are especially useful in this reduction reaction.

The next step in Scheme I (**Method C**) is the selective acylation of the primary amine using standard methods, as typified by Method C. Because of the higher steric demand of the secondary amine, the primary amine is readily available for selective substitution.

This acylation can be done using any of a large number of techniques regularly employed by those skilled in organic chemistry. One such reaction scheme is a substitution using an anhydride such as acetic anhydride. Another reaction scheme often employed to acylate a primary amine employs a carboxylic acid preferably with an activating agent as described for Method A, supra. An amino-de-alkoxylation type of reaction uses esters as a means of acylating the primary amine. Activated esters which are attenuated to provide enhanced selectivity are very efficient acylating agents.

Scheme I

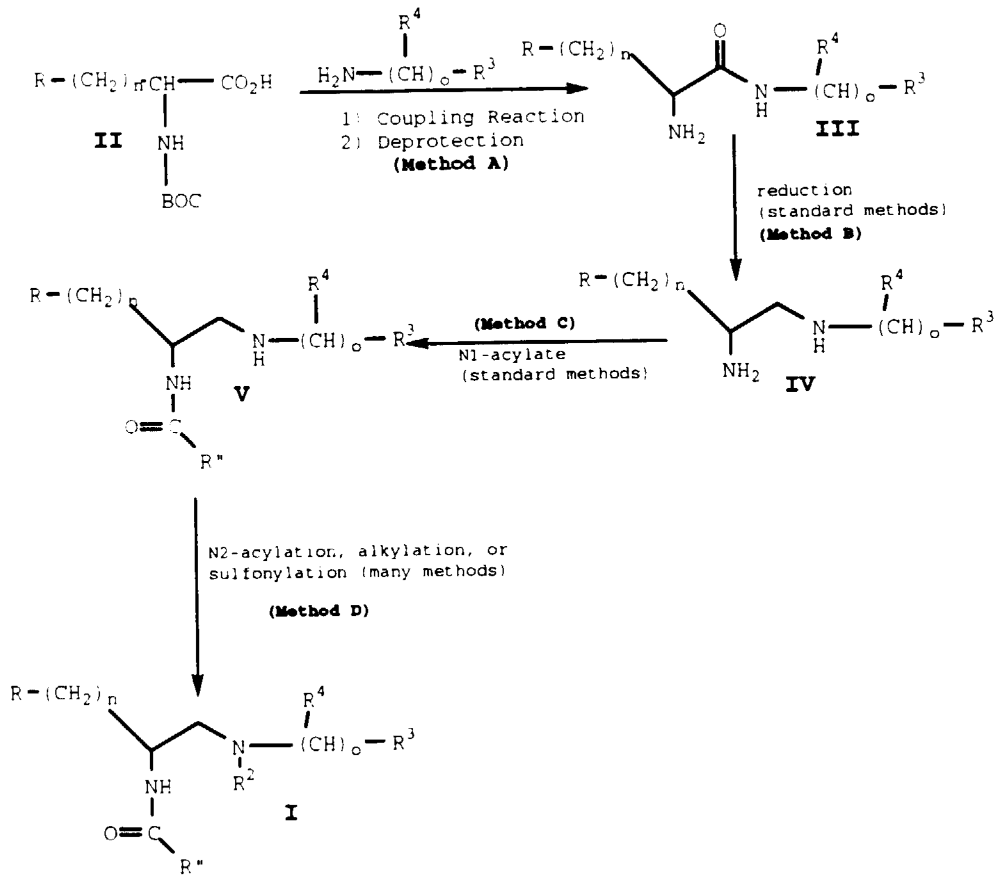


wherein:

R" is equal to $-(CH_2)_m-R^1$; and
$R^2$ is not hydrogen.

## Scheme II

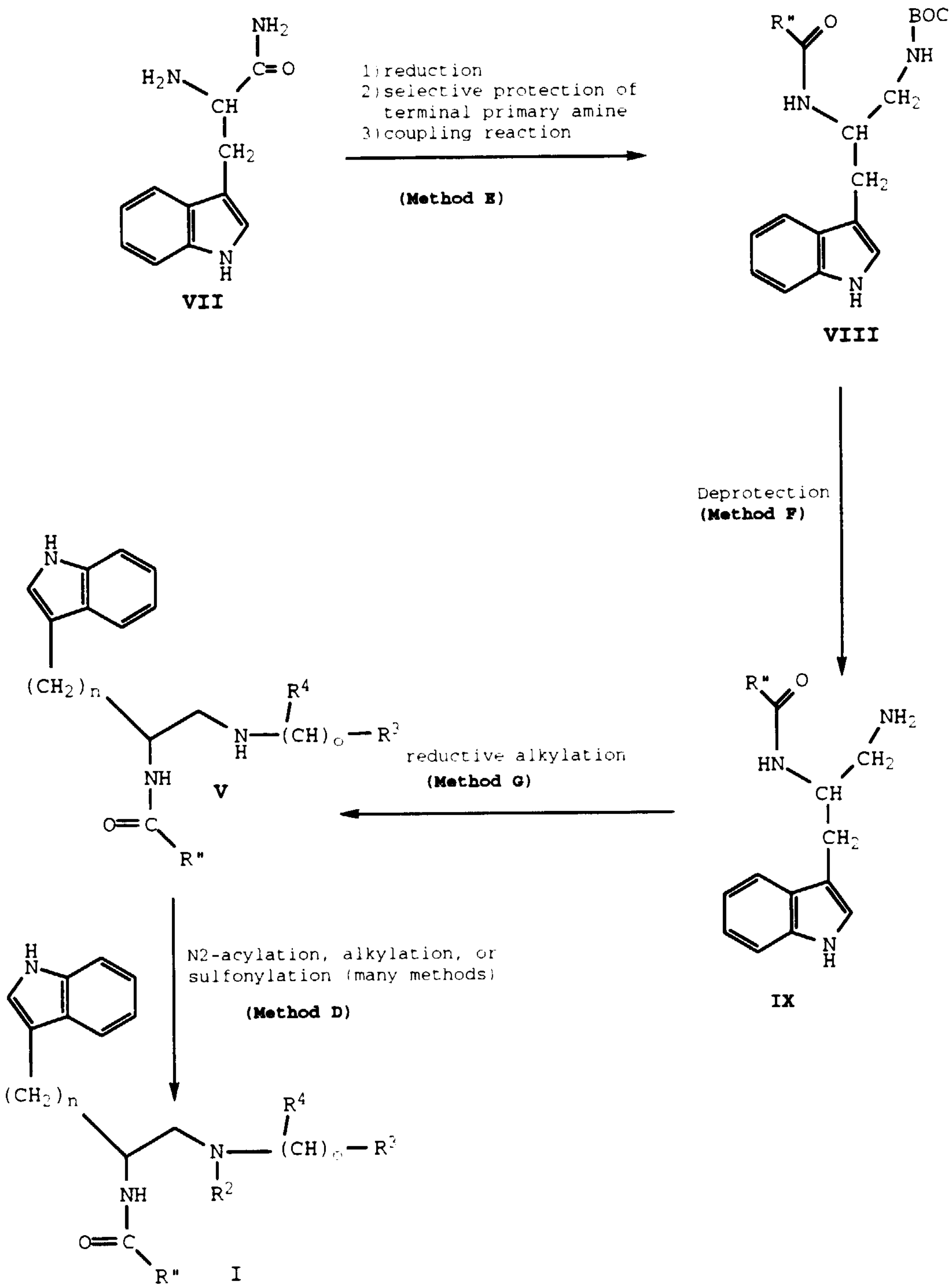


Primary amines can also be acylated using amides to perform what is essentially an exchange reaction. This reaction is usually carried out with the salt of the amine. Boron trifluoride, usually in the form of a boron trifluoride diethyl ether complex, is frequently added to this reaction to complex with the leaving ammonia.

The next procedure is one of substitution of the secondary amine (**Method D**). For most of the compounds of Formula I this substitution is one of alkylation, acylation, or sulfonation. This substitution is usually accomplished using well recognized means. Typically, alkylations can be achieved using alkyl halides and the like as well as the well-known reductive alkylation methods as seen in Method G, Scheme II, supra, employing aldehydes or ketones. Many of the acylating reaction protocols discussed supra efficiently acylate the secondary amine as well. Alkyl- and aryl-sulfonyl

chlorides can be employed to sulfonate the secondary amine.

In many instances one of the later steps in the synthesis of the compounds of Formula I is the removal of an amino- or carboxy-protecting group. Such procedures, which vary, depending upon the type of protecting group employed as well as the relative lability of other moieties on the compound, are described in detail in many standard references works such as T.W. Greene, et al., Protective Groups in Organic Synthesis (1991).

Schemes II and III depict alternative protocols and strategies for the synthesis of the compounds of Formula I. Many of the individual reactions are similar to those described in Scheme I but the reactions of Schemes II and III are done in a different, but yet well known to those skilled in the art, series of steps.

## Scheme III

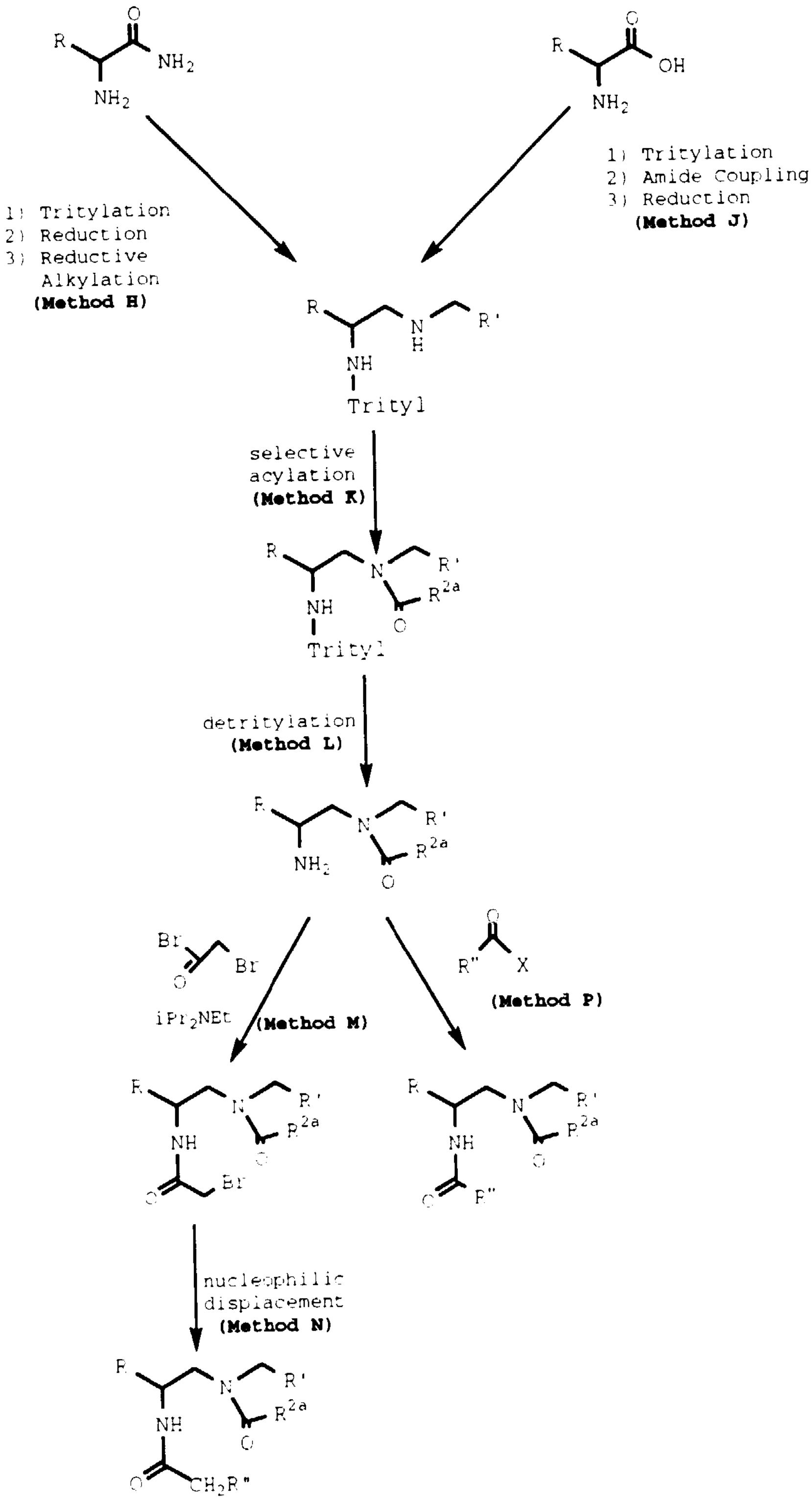


wherein $R^{2a}$ coupled with the carbonyl group to which it is attached is equal to $R^2$.

In order to preferentially prepare one optical isomer over its enantiomer, the skilled practitioner can proceed by one of two routes. The practitioner may first prepare the mixture of enantiomers and then separate the two enantiomers. A commonly employed method for the resolution of the racemic mixture (or mixture of enantiomers) into the individual enantiomers is to first convert the enantiomers to diastereomers by way of forming a salt with an optically active salt

or base. These diastereomers can then be separated using differential solubility, fractional crystallization, chromatography, or like methods. Further details regarding resolution of enantiomeric mixtures can be found in J. Jacques, et al., "Enantiomers, Racemates, and Resolutions", (1991).

In addition to the schemes described above, the practitioner of this invention may also choose an enantiospecific protocol for the preparation of the compounds of Formula I. Scheme IV, infra, depicts a typical such synthetic reaction design which maintains the chiral center present in the starting material in a desired orientation, in this case in the "R" configuration. These reaction schemes usually produce compounds in which greater than 95 percent of the title product is the desired enantiomer.

Many of the synthetic steps employed in Scheme IV are the same as used in other schemes, especially Scheme III.

## Scheme IV

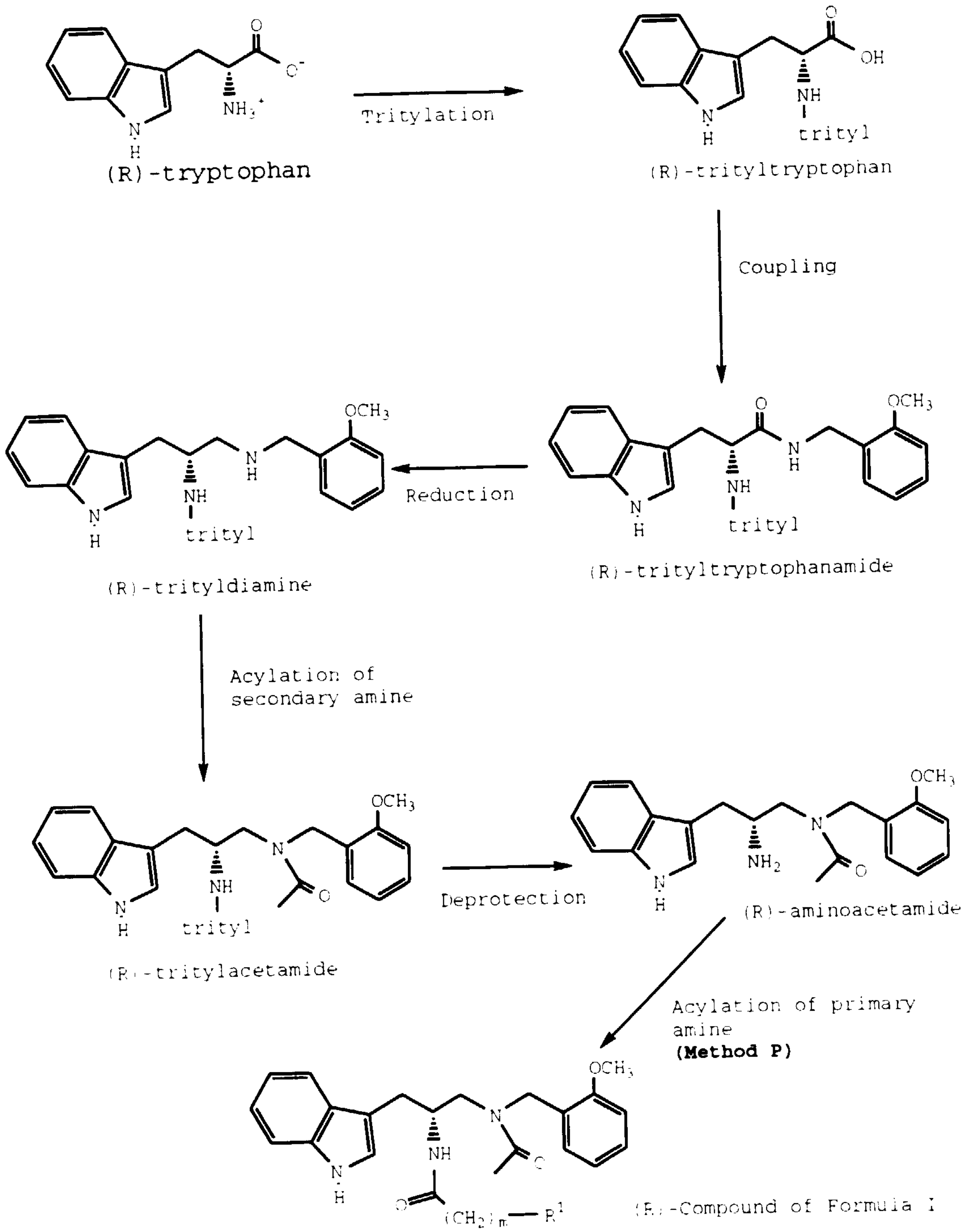


(R)-tryptophan

(R)-trityltryptophan

(R)-trityltryptophanamide

(R)-trityldiamine

(R)-tritylacetamide

(R)-aminoacetamide

(R)-Compound of Formula I

The following depicts representative examples of reaction conditions employed in the preparation of the compounds of Formula I.

**Method A**

**Coupling of carboxylic acid and primary amine to form amide**

Preparation of 2-*t*-butoxycarbonylamino-3-(1H-indol-3-yl)-N-(2-methoxybenzyl )propanamide

To a solution of N-(*t*-butoxycarbonyl)tryptophan (46.4 g, 152.6 mmoles) in 500 ml of dioxane was added carbonyl diimidazole (25.4 g, 156 mmoles) in a portionwise manner. The resulting mixture was stirred for about 2.5 hours at room temperature and then stirred at 45°C for 30 minutes. Next, 2-methoxybenzylamine (20.7 ml, 158.7 mmoles) was added and the reaction mixture was then stirred for 16 hours at room temperature.

The dioxane was removed under reduced pressure. The product was partitioned between ethyl acetate and water and was washed successively with 1 N hydrochloric acid, saturated sodium bicarbonate solution, water, and brine, followed by drying over sodium sulfate and removal of the solvent. Final crystallization from methanol yielded 52.2 g of homogeneous product as yellow crystals. Yield 80.8%. m.p. 157-160°C.

**Deprotection of primary amine**

Synthesis of 2-amino-3-(1H-indol-3-yl)-N-(2-methoxybenzyl )propanamide

To a mixture of the 2-*t*-butoxycarbonylamino-3-(1H-indol-3-yl)-N-(2-methoxybenzyl)propanamide prepared supra (25.1 g, 59.2 mmoles) and anisole (12 ml, 110.4 mmoles) at 0°C was added dropwise an aqueous solution of trifluoroacetic acid (118 ml, 1.53 moles) in 50 ml of water. This mixture was stirred for one hour at 0°C, followed by stirring for about 2.5 hours at ambient temperature. The mixture was then refrigerated for about 16 hours.

The volatiles were removed under reduced pressure. The product was partitioned between ethyl acetate and saturated sodium bicarbonate solution and was then washed with water followed by brine and then dried over sodium sulfate. The solvents were removed in vacuo. Recrystallization from a 1:1 diethyl ether/cyclohexane solution yielded 18.0 g (94.2%) of homogeneous product as an off-white powder. m.p. 104-108°C.

**Method B**

**Reduction of amide carbonyl**

Synthesis of 2-amino-3-(1H-indol-3-yl)-1-[N-(2-methoxybenzyl )amino]propane

To a refluxing solution of 2-amino-3-(1H-indol-3-yl)-N-(2-methoxybenzyl)propanamide (9.81 g, 30.3 mmoles), prepared as described supra, in 100 ml of anhydrous tetrahydrofuran was added dropwise a 10M borane-methyl sulfide complex (9.1 ml, 91.0 mmoles). The resulting mixture was refluxed for about 2 hours. The mixture was cooled to room temperature and the excess borane was quenched by the dropwise addition of 160 ml of methanol. The resulting mixture was refluxed for 15 minutes and the methanol was removed under reduced pressure.

The residue was dissolved in a saturated methanol solution of hydrochloric acid (250 ml) and the solution refluxed for about 1 hour. The methanol was removed in vacuo and the product was isolated the addition of 5 N sodium hydroxide followed by extraction with diethyl ether. The product was then dried over sodium sulfate. The solvents were removed in vacuo. Flash chromatography (silica gel, eluting with methanol:methylene chloride:ammonium hydroxide, 10:100: 0.5) provided 7.1 g of a mixture of the title compound (75%) and the indoline derivative of the title product (25%) as an amber oil.

**Method C**

**Acylation of primary amine**

Preparation of 3-(1H-indol-3-yl)-1-[N-(2-methoxybenzyl)amino]-2-[N-(2-((4-phenyl)piperazin-1-yl)acetyl)amino] propane

[Compound of Example 17]

A mixture of 2-((4-phenyl)piperazin-1-yl)acetic acid, sodium salt (1.64 g, 6.8 mmoles) and triethylamine hydrobromide (1.24 g, 6.8 mmoles) in 35 ml of anhydrous dimethylformamide was heated to 50°C and remained at that temperature for about 35 minutes. The mixture was allowed to cool to room temperature. 1,1-Carbonyl diimidazole (1.05

g, 6.5 mmoles) and 10 ml of anhydrous dimethylformamide were added to the mixture. The resulting mixture was stirred for about 3 hours at room temperature.

A solution of the 2-amino-3-(1H-indol-3-yl)-1-[N-(2-methoxybenzyl)amino]propane (75%) and the indoline derivative (25%) prepared supra, dissolved in 10 ml of anhydrous dimethylformamide was added to the previous reaction mixture. The resulting mixture was stirred for about 16 hours at room temperature. The dimethylformamide was removed under reduced pressure.

The title product and its indoline derivative were partitioned between ethyl acetate and water and then washed with brine, and dried over sodium sulfate. The solvents were removed in vacuo. This process yielded 3.2 g of a mixture of the title compound and its indoline derivative as a yellow oil. These two compounds were then separated using high performance liquid chromatography using a reverse phase column followed by a silica gel column to give the title product (5.2 % yield) as a yellow foam.

**Method D**

**Techniques of Acylation of Secondary Amine**

Preparation of 1-[N-ethoxycarbonyl-N-(2-methoxybenzyl)amino]-3-(1H-indol-3-yl)-2-[N-(2-((4-phenyl)piperazin-1-yl) acetyl)amino]propane

[Compound of Example 28]

To a solution of the 3-(1H-indol-3-yl)-1-[N-(2-methoxybenzyl)amino]-2-[N-(2-((4-phenyl)piperazin-1-yl)acetyl)amino]propane (0.43 g, 0.85 mmole) and triethylamine (130 µl, 0.93 mmole) in 5 ml of anhydrous tetrahydrofuran, was added dropwise ethylchloroformate (89 µl, 0.93 mmole). The resulting mixture was stirred for about 16 hours at room temperature. The tetrahydrofuran was removed under reduced pressure.

The acylated product was partitioned between ethyl acetate and 0.2 N sodium hydroxide, and was then washed with water and brine successively, then dried over sodium sulfate. Flash chromatography (silica gel, methanol:methylene chloride, 2.5:97.5) provided 390 mg of homogeneous title product as a white foam.

Preparation of 3-(1H-indol-3-yl)-1-[N-(2-methoxybenzyl)-N-(methylaminocarbonyl)amino]-2-[N-(2-((4-phenyl) piperazin-1-yl)acetyl)amino]propane

[Compound of Example 29]

To a room temperature solution of 3-(1H-indol-3-yl)-1-[N-(2-methoxybenzyl)amino]-2-[N-(2-((4-phenyl)piperazin-1-yl)acetyl)amino]propane (0.40 g, 0.78 mmole) in 10 ml of anhydrous tetrahydrofuran was added dropwise methyl isocyanate (140 µl, 2.3 mmoles). The resulting mixture was then stirred for 16 hours at room temperature. The tetrahydrofuran was removed in vacuo. The title product was isolated by consecutive washes with ethyl acetate, water, and brine, and then dried over sodium sulfate. Flash chromatography using silica gel and a methanol/methylene chloride (5/95) eluant provided 396 mg of the homogeneous product as a yellow oil.

**Alkylation of Secondary Amine**

Preparation of 1-[N-ethyl-N-(2-methoxybenzyl)amino]-3-(1H-indol-3-yl)-2-[N-(2-((4-phenyl)piperazin-1-yl)acetyl) amino]propane

[Compound of Example 9]

To a room temperature solution of 3-(1H-indol-3-yl)-1-[N-(2-methoxybenzyl)amino]-2-[N-(2-((4-phenyl)piperazin-1-yl)acetyl)amino]propane (0.41 g, 0.80 mmole) in 5 ml of anhydrous N,N-dimethylformamide were added ethyl iodide (120 µl, 1.5 mmoles) and potassium carbonate (120 mg, 0.87 mmole). This mixture was then heated to 50°C and maintained at that temperature for about 4 hours after which it was stirred at room temperature for about 16 hours. The N,N-dimethylformamide was then removed under reduced pressure. The product was partitioned between ethyl acetate and water, and then washed with brine, before drying over sodium sulfate. The solvents were removed in vacuo. Preparative thin layer chromatography provided 360 mg of the title product as a yellow foam.

## Method E

### Reduction of the carbonyl of an amide

Preparation of 1,2-diamino-3-(1H-indol-3-yl)propane

Boron trifluoride etherate (12.3 ml, 0.1 mmole) was added to a tetrahydrofuran (24.4 ml) solution of tryptophan amide (20.3 g, 0.1 mole) at room temperature with stirring. At reflux with constant stirring, borane methylsulfide (32.25 ml, 0.34 mole) was added dropwise. The reaction was heated at reflux with stirring for five hours. A tetrahydrofuran: water mixture (26 ml, 1:1) was carefully added dropwise. A sodium hydroxide solution (160 ml, 5N) was added and the mixture heated at reflux with stirring for sixteen hours.

The layers of the cooled mixture were separated and the aqueous was extracted twice with 40 ml each of tetrahydrofuran. These combined tetrahydrofuran extracts were evaporated. Ethyl acetate (800 ml) was added and this solution was washed three times with 80 ml saturated sodium chloride solution. The ethyl acetate extract was dried over sodium sulfate, filtered and evaporated to yield 18.4 g (97%) of the title compound.

### Protection of primary amine

Preparation of the 2-amino-1-*t*-butoxycarbonylamino-3-(1H-indol-3-yl)propane.

Di-*t*-butyldicarbonate (0.90 ml, 3.9 mmoles) in 10 ml of tetrahydrofuran was added dropwise at room temperature to the 1,2-diamino-3-(1H-indol-3-yl)propane (1.06 g, 5.6 mmoles) produced supra, which was dissolved in 28 ml of tetrahydrofuran. This dropwise addition occurred over a 5 hour period. The solvent was evaporated. Flash chromatography using ethanol/ammonium hydroxide/ethylacetate yielded 0.51 g (1.76 mmoles, 31%) of the desired carbamate.

### Acylation of the secondary amine

Preparation of 1-*t*-butoxycarbonylamino-3-(1H-indol-3-yl)-2-[N-(2-((4-phenyl)piperazin-1-yl)acetyl)amino]propane

[Compound of Example 151]

A slurry of 2-((4-phenyl)piperazin-1-yl)acetic acid (2.47 g, 11.2 mmoles) and triethylamine (3.13 ml, 22.5 mmoles) in acetonitrile (1200 ml) was heated to reflux briefly with stirring. While the resulting solution was still warm carbonyldiimidazole (1.82 g, 11.2 mmoles) was added and the mixture was heated at reflux for 10 minutes. The 2-amino- 1-*t*-butoxycarbonylamino-3-(1H-indol-3yl)-propane (3.25 g, 11.2 mmoles) in 50 ml of acetonitrile was then added to the reaction. The resulting mixture was refluxed with stirring for 30 minutes and was then stirred at room temperature overnight.

The reaction mixture was then refluxed with stirring for 5 hours and the solvent was then removed in vacuo. The resulting oil was washed with a sodium carbonate solution, followed by six washes with water, which was followed by a wash with a saturated sodium chloride solution. The resulting liquid was dried over sodium sulfate and filtered. The retained residue was then dried in vacuo. The filtrate was reduced in volume and then partially purified by chromatography. The sample from the chromatograaphy was pooled with the residue retained by the filter, combining for 3.94 grams (72% yield) of the title product.

## Method F

### Deprotection of Primary Amine

Synthesis of 1-amino-3-(1H-indol-3-yl)-2-[N-(2-((4-phenyl)piperazin-1-yl)acetyl)amino]propane

[Compound of Example 150]

To an ice cold soution of 70% aqueous trifluoroacetic acid (2.8 ml of trifluoroacetic acid in 4.0 ml total volume) were added 1-*t*-butoxycarbonylamino-3-(1H-indol-3-yl)-2-[N-(2-((4-phenyl)piperazin-1-yl)acetyl)amino]propane (0.80 g, 1.63 mmoles) and anisole (0.4 ml). This mixture was stirred for 35 minutes, resulting in a clear solution. The solution was then stirred for an additional hour and then evaporated.

Ethyl acetate was then added to the resulting liquid, followed by a wash with a sodium carbonate solution. This wash was then followed by three washes with a saturated sodium chloride solution. The resulting solution was then

dried over soldium sulfate, filtered and evaporated, resulting in 0.576 g (90% yield) of the title product.

**Method G**

**Reductive Alkylation of Primary Amine**

Preparation of 1-[N-(2-chlorobenzyl)amino]-3-(1H-indol-3-yl )-2-[N-(2-((4-phenyl)piperazin-1-yl)acetyl)amino] propane.

[Compound of Example 2]

2-Chlorobenzaldehyde (0.112 g, 0.8 mmole) was combined with the 1-amino-3-(1H-indol-3-yl)-2-[N-(2-((4-phenyl) piperazin-1-yl)acetyl)amino]propane (0.156 g, 0.398 mmole) in toluene. The resulting mixture was then stirred and warmed, and then evaporated. Toluene was then added to the residue and this mixture was again evaporated. Tetrahydrofuran was added to the residue and the mixture was then cooled in an ice bath.

Sodium cyanoborohydride (0.025 g, 0.4 mmole) was then added to the reaction mixture. Gaseous hydrogen chloride was periodically added above the liquid mixture. The mixture was stirred at room temperature for 16 hours and then reduced in volume in vacuo.

A dilute hydrochloric acid solution was then added to the residue and the solution was then extracted twice with ether. The acidic aqueous extract was basified by the dropwise addition of 5N sodium hydroxide. This basified solution was then extracted three times with ethyl acetate. The combined ethyl acetate washes were washed with a saturated sodium chloride solution, dried over sodium sulfate, filtered and evaporated. This process was followed by chromatography yielding 0.163 g (79% yield) of the title product.

**Method H**

**Tritylation**

Preparation of 3-(1H-indol-3-yl)-2-(N-triphenylmethylamino )propanamide.

Tryptophan amide (26.43 g, 0.130 mole) was suspended in 260 ml of methylene chloride and this mixture was flushed with nitrogen and then put under argon. Trityl chloride (38.06 g, 0.136 mole) was dissolved in 75 ml of methylene chloride. The trityl chloride solution was slowly added to the tryptophan amide solution which sat in an ice bath, the addition taking about 25 minutes. The reaction mixture was then allowed to stir ovenight.

The reaction mixture was then poured into a separation funnel and was washed with 250 ml of water, followed by 250 ml of brine. As the organic layer was filtering through sodium sulfate to dry, a solid precipitated. The filtrate was collected and the solvent was evaporated.

Ethyl acetate was then added to the pooled solid and this mixture was stirred and then refrigerated overnight. The next day the resulting solid was washed several times with cold ethyl acetate and then dried in vacuo. Yield 49.76 g (85.9%).

**Reduction of Carbonyl**

Preparation of 1-amino-3-(1H-indol-3-yl)-2-(N-triphenylmethylamino)propane

Under argon the 3-(1H-indol-3-yl)-2-(N-triphenylmethylamino)propanamide (48.46 g, 0.108 mole) was suspended in 270 ml of tetrahydrofuran. This mixture was then heated to reflux. Borane-methyl sulfide complex (41.3 g, 0.543 mole) was then slowly added to the reaction mixture. All of the starting amide dissolved during the addition of the borane-methyl sulfide complex. This solution was then stirred overnight in an 83°C oil bath.

After cooling a 1:1 mixture of tetrahydrofuran:water (75 ml total) was then added to the solution. Sodium hydroxide (5N, 230 ml) was then added to the mixture, which was then heated to reflux for about 30 minutes.

After partitioning the aqueous and organic layers, the organic layer was collected. The aqueous layer was then extracted with tetrahydrofuran. The organic layers were combined and the solvents were then removed by evaporation. The resulting liquid was then partitioned between ethyl acetate and brine and was washed a second time with brine. The solution was then dried over sodium sulfate and the solvents were removed in vacuo to yield 48.68 grams of the desired intermediate.

**Substitution of primary amine**

Preparation of 1-[N-(2-methoxybenzyl)amino]-3-(1H-indol-3-yl)-2-(N-triphenylmethylamino)propane

To a mixture of 1-amino-3-(1H-indol-3-yl)-2-(N-triphenylmethylamino)propane (48.68 g, 0.109 mole) dissolved in toluene (1.13 l) was added 2-methoxybenzaldehyde (23.12 g, 0.169 mole), the 2-methoxybenzaldehyde having been previously purified by base wash. The reaction mixture was stirred overnight. The solvents were then removed in vacuo.

The recovered solid was dissolved in 376 ml of a 1:1 tetrahydrofuran:methanol mixture. To this solution was added sodium borohydride (6.83 g, 0.180 mole). This mixture was stirred on ice for about 4 hours. The solvents were removed by evaporation. The remaining liquid was partitioned between 1200 ml of ethyl acetate and 1000 ml of a 1:1 brine:20N sodium hydroxide solution. This was extracted twice with 500 ml of ethyl acetate each and then dried over sodium sulfate. The solvents were then removed by evaporation overnight, yielding 67.60 grams (>99% yield) of the desired product.

**Method J**

**Tritylation**

Preparation of 3-(1H-indol-3-yl)-2-(N-triphenylmethylamino)propanoic acid [N-trityltryptophan]

Chlorotrimethylsilane (70.0 ml, 0.527 moles) was added at a moderate rate to a stirring slurry of tryptophan (100.0 g, 0.490 mole) in anhydrous methylene chloride (800 ml) under a nitrogen atmosphere. This mixture was continuously stirred for 4.25 hours. Triethylamine (147.0 ml, 1.055 moles) was added followed by the addition of a solution of triphenylmethyl chloride (147.0 g, 0.552 mole) in methylene chloride (400 ml) using an addition funnel. The mixture was stirred at room temperature, under a nitrogen atmosphere for at least 20 hours. The reaction was quenched by the addition of methanol (500 ml).

The solution was concentrated on a rotary evaporator to near dryness and the mixture was redissolved in methylene chloride and ethyl acetate. An aqueous work-up involving a 5% citric acid solution (2X) and brine (2X) was then performed. The organic layer was dried over anhydrous sodium sulfate, filtered, and concentrated to dryness on a rotary evaporator. The solid was dissolved in hot diethyl ether followed by the addition of hexanes to promote crystallization. By this process 173.6 g (0.389 mole) of analytically pure 3-(1H-indol-3-yl)-2-(N-triphenylmethylamino)propanoic acid was isolated as a light tan solid in two crops giving a total of 79% yield.

**Coupling**

Preparation of 3-(1H-indol-3-yl)-N-(2-methoxybenzyl)-2-(N-triphenylmethylamino)propanamide

To a stirring solution of 3-(1H-indol-3-yl)-2-(N-triphenylmethylamino)propanoic acid (179.8 g, 0.403 mole), 2-methoxybenzylamine (56.0 ml, 0.429 mole), and hydroxybenzotriazole hydrate (57.97 g, 0.429 mole) in anhydrous tetrahydrofuran (1.7 L) and anhydrous N,N-dimethylformamide (500 ml) under a nitrogen atmosphere at 0°C, were added triethylamine (60.0 ml, 0.430 mole) and 1-(3-dimethylaminopropyl)-3-ethoxycarbodiimide hydrochloride (82.25 g, 0.429 mole). The mixture was allowed to warm to room temperature under a nitrogen atmosphere for at least 20 hours. The mixture was concentrated on a rotary evaporator and then redissolved in methylene chloride and an aqueous work-up of 5% citric acid solution (2X), saturated sodium bicarbonate solution (2X), and brine (2X) was performed. The organic layer was dried over anhydrous sodium sulfate, filtered, and concentrated to dryness on a rotary evaporator. The title product was then filtered as a pink solid in two lots. Isolated 215.8 g (0.381 mole) of analytically pure material (95% yield).

**Reduction**

Preparation of 3-(1H-indol-3-yl)-1-[N-(2-methoxybenzyl)amino]-2-(N-triphenylmethylamino)propane

Red-Al®, [a 3.4 M, solution of sodium bis(2-methoxyethoxy)aluminum hydride in toluene] (535 ml, 1.819 moles), dissolved in anhydrous tetrahydrofuran (400 ml) was slowly added using an addition funnel to a refluxing solution of the acylation product, 3-(1H-indol-3-yl)-N-(2-methoxybenzyl)-2-(N-triphenylmethylamino)propanamide (228.6 g, 0.404 mols) produced supra, in anhydrous tetrahydrofuran (1.0 liter) under a nitrogen atmosphere. The reaction mixture became a purple solution. The reaction was quenched after at least 20 hours by the slow addition of excess saturated Rochelle salt solution (potassium sodium tartrate tetrahydrate). The organic layer was isolated, washed with brine (2X),

dried over anhydrous sodium sulfate, filtered, and concentrated to an oil on a rotary evaporator. No further purification was done and the product was used directly in the next step.

**Method K**

**Acylation**

Preparation of 3-(1H-indol-3-yl)-1-[N-(2-methoxybenzyl)-acetylamino]-2-(N-triphenylmethylamino)propane

To a stirring solution of 3-(1H-indol-3-yl)-1-[N-(2-methoxybenzyl)amino]-2-(N-triphenylmethylamino)propane (0.404 mole) in anhydrous tetrahydrofuran (1.2 liters) under a nitrogen atmosphere at 0°C was added triethylamine (66.5 ml, 0.477 mole) and acetic anhydride (45.0 ml, 0.477 mole). After 4 hours, the mixture was concentrated on a rotary evaporator, redissolved in methylene chloride and ethyl acetate, washed with water (2X) and brine (2X), dried over anhydrous sodium sulfate, filtered, and concentrated to a solid on a rotary evaporator. The resulting solid was dissolved in chloroform and loaded onto silica gel 60 (230-400 mesh) and eluted with a 1:1 mixture of ethyl acetate and hexanes. The product was then crystallized from an ethyl acetate/hexanes mixture. The resulting product of 3-(1H-indol-3-yl)-1-[N-(2-methoxybenzyl)acetylamino]-2-(N-triphenylmethylamino)propane was crystallized and isolated over three crops giving 208.97 grams (87% yield) of analytically pure material.

**Method L**

**Detritylation**

Preparation of 2-amino-3-(1H-indol-3-yl)-1-[N-(2-methoxybenzyl )acetylamino]propane

Formic acid (9.0 ml, 238.540 mmoles) was added to a stirring solution of 3-(1H-indol-3-yl)-1-[N-(2-methoxybenzyl) acetylamino]-2-(N-triphenylmethylamino)propane (14.11 g, 23.763 mmoles) in anhydrous methylene chloride under a nitrogen atmosphere at 0°C. After 4 hours, the reaction mixture was concentrated to an oil on a rotary evaporator and redissolved in diethyl ether and 1.0 N hydrochloric acid. The aqueous layer was washed twice with diethyl ether and basified with sodium hydroxide to a pH greater than 12. The product was extracted out with methylene chloride (4X). The organic extracts were combined, dried over anhydrous sodium sulfate, filtered, and concentrated on a rotary evaporator to a white foam. The compound 2-amino-3-(1H-indol-3-yl)-1-[N-(2-methoxybenzyl)acetylamino]propane (7.52 g, 21.397 mmols) was isolated giving a 90% yield. No further purification was necessary.

**Method M**

**Bromoacetylation**

Preparation of 2-[(2-bromo)acetyl]amino-3-(1H-indol-3-yl)-1-[N-(2-methoxybenzyl)acetylamino]propane

To a stirring solution of 2-amino-3-(1H-indol-3-yl)-1-[N-(2-methoxybenzyl)acetylamino]propane (7.51 g, 21.369 mmoles) in anhydrous tetrahydrofuran (100 ml) under a nitrogen atmosphere at 0°C was added diisopropylethylamine (4.1 ml, 23.537 mmoles) and bromoacetyl bromide (2.05 ml, 23.530 mmoles). After 2 hours, ethyl acetate was added and the reaction mixture washed with water twice, 1.0 N hydrochloric acid (2X), saturated sodium bicarbonate solution (2X), and brine. The organic layer was dried over anhydrous sodium sulfate, filtered, and concentrated to a tan foam on a rotary evaporator. In this manner the 2-[(2-bromo)acetyl]amino-3-(1H-indol-3-yl)-1-[N-(2-methoxybenzyl) acetylamino]propane was obtained in quantitative yield. No further purification was necessary.

**Method N**

**Nucleophilic Displacement**

Preparation of 1-[N-(2-methoxybenzyl)acetylamino]-3-(1H-indol-3-yl)-2-[N-(2-((4-cyclohexyl)piperazin-1-yl)acetyl ) amino]propane

[Compound of Example 74]

1-Cyclohexylpiperazine (3.65 g, 22.492 mmoles) was added to a stirring solution of 2-[(2-bromo)acetyl]amino-3-(1H-indol-3-yl)-1-[N-(2-methoxybenzyl)acetylamino]propane (21.369 mmoles) and powdered potassium carbonate (3.56 g, 25.758 mmols) in methylene chloride under a nitrogen atmosphere. The reaction mixture was stirred overnight at room temperature. The salts were filtered and the solution concentrated to a brown foam on a rotary evaporator. The desired product was purified on a Prep 500 column using a 10 L gradient starting with 100% methylene chloride and ending with 5% methanol/94.5% methylene chloride/0.5% ammonium hydroxide. Impure fractions were combined and purified further by reverse phase preparative high performance liquid chromatography (methanol/acetonitrile/water/ammonium acetate). After combining the material from both chromatographic purifications the title compound (10.43 g, 18.663 mmoles) was isolated (87% yield).

An alternative means of acylation of the primary amine as shown in the final step of the synthesis protocol of Scheme IV is by means of reacting a compound of the formula

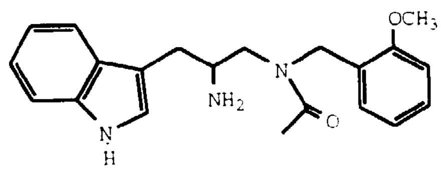

with a potassium carboxylate of the formula

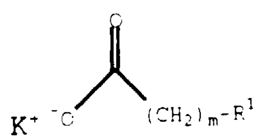

in the presence of isobutylchloroformate and N-methylmorpholine. This reaction is usually performed in the presence of a non-reactive solvent such as methylene chloride at cool temperatures, usually between -30°C and 10°C, more preferably at temperatures between -20°C and 0°C. In this reaction equimolar amounts of the two reactants are generally employed although other ratios are operable. An example of this preferred means of acylating the primary amine is shown in the following example.

**Method P**

Preparation of (R)-1-[N-(2-methoxybenzyl)acetylamino]-3-(1H-indol-3-yl)-2-[N-(2-((4-cyclohexyl)piperazin-1-yl)acetyl) amino]propane

[Compound of Example 75]

The title compound was prepared by first cooling 2-((4-cyclohexyl)piperazin-1-yl)acetic acid potassium salt to a temperature between -8°C and -15°C in 5 volumes of anhydrous methylene chloride. To this mixture was then added isobutylchloroformate at a rate such that the temperature did not exceed -8°C. This reaction mixture was then stirred for about 1 hour, the temperature being maintained between -8°C and -15°C.

To this mixture was then added (R)-2-amino-3-(1H-indol-3-yl)-1-[N-(2-methoxybenzyl)acetylamino]propane dihydrochloride at such a rate that the temperature did not exceed 0°C. Next added to this mixture was N-methyl morpholine at a rate such that the temperature did not exceed 0°C. This mixture was then stirred for about 1 hour at a temperature between -15°C and -8°C.

The reaction was quenched by the addition of 5 volumes of water. The organic layer was washed once with a saturated sodium bicarbonate solution. The organic phase was then dried over anhydrous potassium carbonate and filtered to remove the drying agent. To the filtrate was then added 2 equivalents of concentrated hydrochloric acid, followed by 1 volume of isopropyl alcohol. The methylene chloride was then exchanged with isopropyl alcohol under vacuum by distillation.

The final volume of isopropyl alcohol was then concentrated to three volumes by vacuum. The reaction mixture was cooled to 20°C to 25°C and the product was allowed to crystallize for at least one hour. The desired product was then recovered by filtration and washed with sufficient isopropyl alcohol to give a colorless filtrate. The crystal cake was then dried under vacuum at 50°C.

Preparation of (R)-3-(1H-indol-3-yl)-1-[N-(2-methoxybenzyl)acetylamino]-2-[N-(2-(4-(piperidin-1-yl)piperidin-1-yl) acetyl)amino]propane dihydrochloride trihydrate

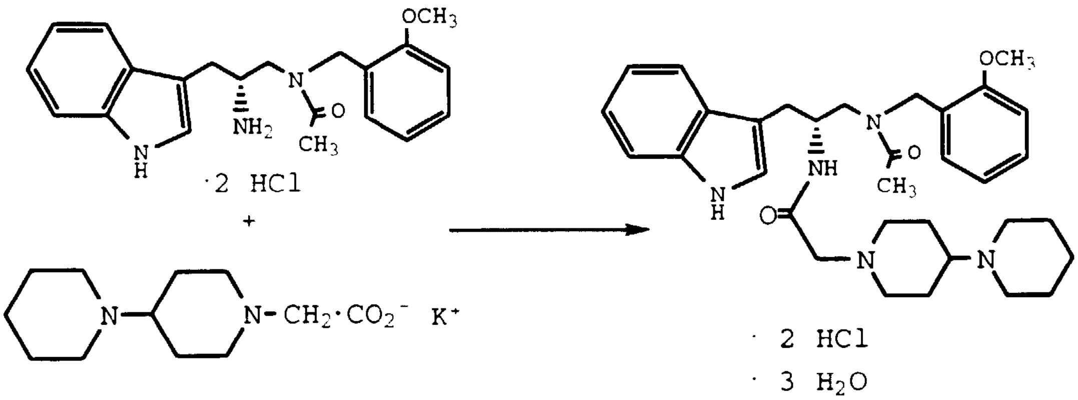

Under a nitrogen atmosphere 2-(4-(piperidin-1-yl)piperidin-1-yl)acetic acid, potassium salt (0.75 kg, 2.84 mol) was added to methylene chloride (7.5 L). The resulting mixture was cooled to -15 to -8°C and isobutyl chloroformate (0.29 kg, 2.12 mol) was added at such a rate so as to maintain the temperature of the reaction mixture below -8°C. After the addition the resulting reaction mixture was stirred for 90 minutes between -15 and -8°C.

The reaction mixture was then cooled to -35°C and solid (R)-2-amino-3-(1H-indol-3-yl)-1-[N-(2-methoxybenzyl) amino]propane dihydrochloride (0.60 kg, 1.14 mol) was added at such a rate that the reaction temperature was maintained at less than -20°C. After the addition, the reaction mixture was stirred for about one hour with the temperature being maintained between -37°C and -20°C. The reaction was quenched by the addition of deionized water (7.5 L). The reaction mixture was basified to pH 12.8-13.2 by the addition of 5 N sodium hydroxide. The aqueous fraction was removed and retained. Additional deionized water (3.75 L) was added to the organic fraction as was sufficient 5 N sodium hydroxide to re-adjust the pH to 12.8-13.2.

The two aqueous fractions were combined, back-extracted with methylene chloride (1.5 L) and then discarded. The organic fractions were combined and washed with deionized water (4 x 3.5 L). These extracts were combined, back-extracted with methylene chloride (1.5 L), and then discarded. The two organic layers were combined and washed with a saturated sodium chloride solution (3.7 L).

The organic fraction was dried over anhydrous magnesium sulfate, filtered, and solvent exchanged from methylene chloride to acetone (3.75 L) on a rotary evaporator. An aqueous solution of hydrochloric acid (0.48 L of 6 N solution, 2.88 mol) and seed crystals (2 g) were added and mixture was stirred for 30-90 minutes. Acetone (13.2 L) was then added and the slurry stirred for one hour. The resulting solid was then filtered, washed with acetone (2 x 1.4 L), and dried to yield 633 g (90%) of(R)-3-(1H-indol-3-yl)-1-[N-(2-methoxybenzyl)acetylamino]-2-[N-(2-(4-(piperidin-1-yl)piperidin-1-yl)acetyl)amino]propane dihydrochloride trihydrate.

An alternative process of preparing the most preferred compounds is as follows.

Preparation of (R)-3-(1H-indol-3-yl)-2-(N-triphenylmethylamino)propanoic acid, N-methylmorpholine salt (N-trityl-D-tryptophan N-methylmopholine salt).

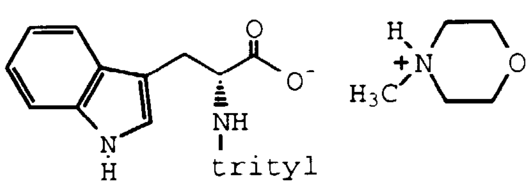

To a one liter 4 neck flask equipped with mechanical stirrer, condensor, probe, and stopper, were added D-tryptophan (40.0 g, 0.196 mol), acetonitrile (240 ml), and 1,1,1,3,3,3-hexamethyldisilazane (39.5 g, 0.245 mol). The resulting mixture was heated to 50-60°C and stirred until homogeneous. In a separate beaker trityl chloride (60.06 g, 0.215 mol) and acetonitrile (120 ml) were slurried. The slurry was added to the silylated tryptophan mixture and the beaker was rinsed with 40 ml of acetonitrile. To the reaction mixture N-methylmorpholine (23.7 ml, 21.8 g, 0.216 mol) was added and the resulting mixture was stirred for one hour. The progress of the reaction was monitored by chromatography.

After satisfactory progress, water (240 ml) was added dropwise to the reaction mixture and the resulting mixture was cooled to less than 10°C, stirred for thirty minutes, and filtered. The residue was washed with water, and then dried to obtain 108.15 grams (>99% yield) of the desired title product.

$^1H$ NMR (DMSO-$d_6$) δ 2.70 (m, 1H), 2.83 (m, 2H), 3.35 (m, 1H), 6.92-7.20 (m, 12H), 7.30-7.41 (m, 8H), 10.83 (s, 1H), 11.73 (br s, 1H).

| Analysis for $C_{30}H_{26}N_2O_2$: | | | |
|---|---|---|---|
| Theory: | C, 80.69; | H, 5.87; | N, 6.27 |
| Found: | C, 80.47; | H, 5.92; | N, 6.10. |

Preparation of (R)-3-(1H-indol-3-yl)-N-(2-methoxybenzyl)-2-(N-triphenylmethylamino )propanamide.

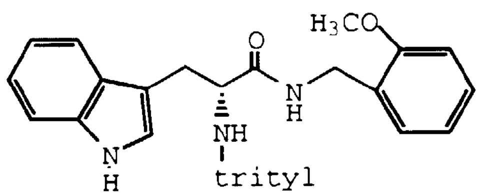

To a two liter 4 neck flask equipped with mechanical stirrer, condensor, and thermocouple, under a nitrogen atmosphere, were added N-trityl-D-tryptophan N-methylmopholine salt (108.0 g, 0.196 mol), acetonitrile (800 ml), 2-chloro-4,6-dimethoxy-1,3,5-triazine (38.63 g, 0.22 mol), and N-methylmorpholine (29.1 ml). The resulting mixture was stirred at ambient temperature until homogeneous (about ten minutes).

After about one hour, 2-methoxybenzylamine (29 ml) was added. The resulting mixture was heated to 35°C and maintained at that temperature overnight. The progress of the reaction was monitored by chromatography. Water (750 ml) was then added dropwise to the reaction mixture and the resulting mixture was cooled to less than 10°C, stirred for thirty minutes, and filtered. The residue was washed with water (about 100 ml), and then dried to obtain the desired title product. (Yield: 87% and 91% in two runs)

FDMS 565 ($M^+$).

$^1H$ NMR ($CDCl_3$) δ 2.19 (dd, J=6.4 Hz, Δυ=14.4 Hz, 1H), 2.64 (d, J=6.5 Hz, 1H), 3.19 (dd, J=4.3 Hz, Δυ=14.4 Hz, 1H), 3.49 (m, 1H), 3.63 (s, 3H), 3.99 (dd, J=5.4 Hz, Δυ=14.2 Hz, 1H), 4.25 (dd, J=7.1 Hz, Δυ=14.2 Hz, 1H), 6.64 (d, J=2.1 Hz, 1H), 6.80 (d, J=8.2 Hz, 1H), 6.91 (t, J=7.4 Hz, 1H), 7.06-7.38 (m, 21 H), 7.49 (d, J=7.9 Hz, 1H), 7.75 (s, 1H).

| Analysis for $C_{38}H_{35}N_3O_2$: | | | |
|---|---|---|---|
| Theory: | C, 80.68; | H, 6.24; | N, 7.43. |
| Found: | C, 80.65; | H, 6.46; | N, 7.50. |

**Reduction of Carbonyl**

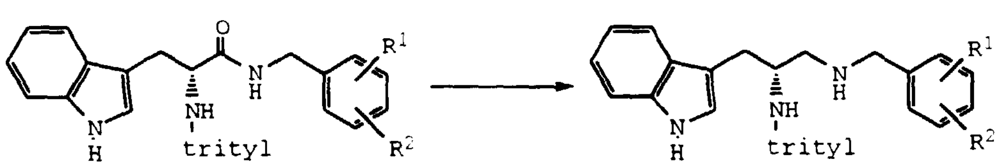

Preparation of (R)-3-(1H-indol-3-yl)-1-[N-(2-methoxybenzyl)amino]-2-(N-triphenylmethylamino)propane

RED-AL®, [a 3.4 M, solution of sodium bis(2-methoxyethoxy)aluminum hydride in toluene] (535 ml, 1.819 mol), dissolved in anhydrous tetrahydrofuran (400 ml) was slowly added using an addition funnel to a refluxing solution of the acylation product, (R)-3-(1H-indol-3-yl)-N-(2-methoxybenzyl)-2-(N-triphenylmethylamino)propanamide (228.6 g, 0.404 mols) produced supra, in anhydrous tetrahydrofuran (1.0 L) under a nitrogen atmosphere. The reaction mixture became a purple solution. The reaction was quenched after at least 20 hours by the slow addition of excess saturated

Rochelle's salt solution (potassium sodium tartrate tetrahydrate). The organic layer was isolated, washed with brine (2X), dried over anhydrous sodium sulfate, filtered, and concentrated to an oil on a rotary evaporator. No further purification was done and the product was used directly in the next step.

**Acylation of Secondary Amine**

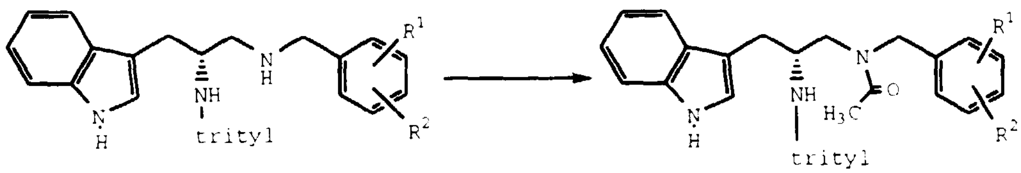

Preparation of (R)-3-(1H-indol-3-yl)-1-[N-(2-methoxybenzyl)-acetylamino]-2-(N-triphenylmethylamino)propane

To a stirring solution of (R)-3-(1H-indol-3-yl)-1-[N-(2-methoxybenzyl)amino]-2-(N-triphenylmethylamino)propane (0.404 mol) in anhydrous tetrahydrofuran (1.2 L) under a nitrogen atmosphere at 0°C was added triethylamine (66.5 ml, 0.477 mol) and acetic anhydride (45.0 ml, 0.477 mol). After 4 hours, the mixture was concentrated on a rotary evaporator, redissolved in methylene chloride and ethyl acetate, washed with water (2X) and brine (2X), dried over anhydrous sodium sulfate, filtered, and concentrated to a solid on a rotary evaporator. The resulting solid was dissolved in chloroform and loaded onto silica gel 60 (230-400 mesh) and eluted with a 1:1 mixture of ethyl acetate and hexanes. The product was then crystallized from an ethyl acetate/hexanes mixture. The resulting product of (R)-3-(1H-indol-3-yl)-1-[N-(2-methoxybenzyl )acetylamino]-2-(N-triphenylmethylamino)propane was crystallized and isolated over three crops giving 208.97 grams (87% yield) of analytically pure material.

| Analysis for $C_{40}H_{39}N_3O_2$: | | | |
|---|---|---|---|
| Theory: | C, 80.91; | H, 6.62; | N, 7.08. |
| Found: | C, 81.00; | H, 6.69; | N, 6.94. |

**Deprotection**

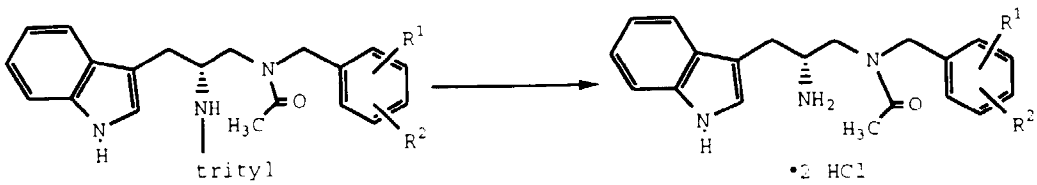

Preparation of (R)-2-amino-3-(1H-indol-3-yl)-1-[N-(2-methoxybenzyl)acetylamino]propane dihydrochloride

A stirring solution of (R)-3-(1H-indol-3-yl)-1-[N-(2-methoxybenzyl)acetylamino]-2-(N-triphenylmethylamino)propane in two volumes of methylene chloride was cooled to between -40°C and -50°C. Anhydrous hydrogen chloride gas was added at such a rate that the temperature of the reaction mixture did not exceed 0°C. The reaction mixture was stirred for 30 minutes to one hour at 0-10°C.

To this reaction mixture was added two volumes of methyl *t*-butyl ether and the resulting mixture was allowed to stir for 30 minutes to one hour at 0-10°C. The resulting crystalline solid was removed by filtration and then washed with methyl *t*-butyl ether. The reaction product was dried under vacuum at 50°C. (Yield >98%)

| Analysis for $C_{21}H_{25}N_3O_2 \bullet 2\,HCl$: | | | |
|---|---|---|---|
| Theory: | C, 59.44; | H, 6.41; | N, 9.90. |
| Found: | C, 60.40; | H, 6.60; | N, 9.99. |

Preparation of 2-((4-cyclohexyl)piperazin-1-yl)acetic acid potassium salt hydrate

Cyclohexylpiperazine (10.0 g, 0.059 mol) was added to ten volumes of methylene chloride at room temperature. To this mixture was added sodium hydroxide (36 ml of a 2N solution, 0.072 mol) and tetrabutylammonium bromide (1.3 g, 0.004 mol). After the addition of the sodium hydroxide and tetrabutylammonium bromide, methyl bromoacetate (7.0 ml, 0.073 mol) was added and the reaction mixture was stirred for four to six hours. The progress of the reaction was monitored by gas chromatography.

The organic fraction was separated and the aqueous phase was back-extracted with methylene chloride. The organic phases were combined and washed twice with deionized water, once with saturated sodium bicarbonate solution, and then with brine. The organic phase was dried over magnesium sulfate and the solvents were removed in vacuo to yield methyl 2-((4-cyclohexyl)piperazin-1-yl)acetate as a yellowish oil.

The title compound was prepared by dissolving the methyl 2-((4-cyclohexyl)piperazin-1-yl)acetate (10.0 g, 0.042 mol) in ten volumes of diethyl ether. This solution was cooled to 15°C and then potassium trimethylsilanoate (5.9 g, 0.044) was added. This mixture was then stirred for four to six hours. The reaction product was removed by filtration, washed twice with five volumes of diethyl ether, then washed twice with five volumes of hexanes, and then dried in a vacuum oven for 12-24 hours at 50°C.

| Analysis for $C_{12}H_{21}KN_2O_2 \bullet 1.5\ H_2O$: | | | |
|---|---|---|---|
| Theory: | C, 49.63; | H, 7.98; | N, 9.65. |
| Found: | C, 49.54; | H, 7.72; | N, 9.11. |

Preparation of (R)-2-[N-(2-((4-cyclohexyl)piperazin-1-yl)acetyl)amino]-3-(1H-indol-3-yl)-1-[N-(2-methoxybenzyl)acetylamino]propane

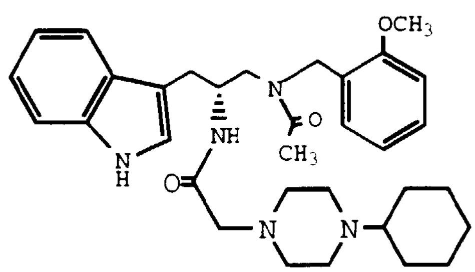

The title compound was prepared by first cooling 2-((4-cyclohexyl)piperazin-1-yl)acetic acid potassium salt to a temperature between -8°C and -15°C in 5 volumes of anhydrous methylene chloride. To this mixture was added isobutylchloroformate at a rate such that the temperature did not exceed -8°C. The resulting reaction mixture was stirred for about 1 hour, the temperature being maintained between -8°C and -15°C.

To this mixture was then added (R)-2-amino-3-(1H-indol-3-yl)-1-[N-(2-methoxybenzyl)acetylamino]propane dihydrochloride at such a rate that the temperature did not exceed 0°C. Next added to this mixture was N-methyl morpholine at a rate such that the temperature did not exceed 0°C. This mixture was then stirred for about 1 hour at a temperature between -15°C and -8°C.

The reaction was quenched by the addition of 5 volumes of water. The organic layer was washed once with a saturated sodium bicarbonate solution. The organic phase was then dried over anhydrous potassium carbonate and filtered to remove the drying agent. To the filtrate was then added 2 equivalents of concentrated hydrochloric acid, followed by 1 volume of isopropyl alcohol. The methylene chloride was then exchanged with isopropyl alcohol under vacuum by distillation.

The final volume of isopropyl alcohol was then concentrated to three volumes by vacuum. The reaction mixture was cooled to 20°C to 25°C and the product was allowed to crystallize for at least one hour. The desired product was then recovered by filtration and washed with sufficient isopropyl alcohol to give a colorless filtrate. The crystal cake was then dried under vacuum at 50°C. MS 560 ($M+1^+$).

$^1H$ NMR ($CDCl_3$) $\delta$ 1.09-1.28 (m, 5H), 1.64 (d, J=10 Hz, 1H), 1.80-1.89 (m, 4H), 2.10 (s, 3H), 2.24-2.52 (m, 9H), 2.90 (s, 2H), 2.95 (d, J=7 Hz, 1H), 3.02 (d, J=7 Hz, 1H), 3.12 (dd, J=5, 14 Hz, 1H), 3.77 (s, 3H), 4.01 (dd, J=10, 14 Hz, 1H), 4.49 (ABq, J=17 Hz, 43 Hz, 2H), 4.56 (m, 1H), 6.79-6.87 (m, 3H), 7.05-7.24 (m, 4H), 7.34-7.41 (m, 2H), 7.67 (d, J=8 Hz, 1H), 8.22 (s, 1H).

| Analysis for $C_{33}H_{45}N_5O_3$: | | | |
|---|---|---|---|
| Theory: | C, 70.81; | H, 8.10; | N, 12.51. |
| Found: | C, 70.71; | H, 8.21; | N, 12.42. |

Preparation of 2-(4-(piperidin-1-yl)piperidin-1-yl)acetic acid, potassium salt

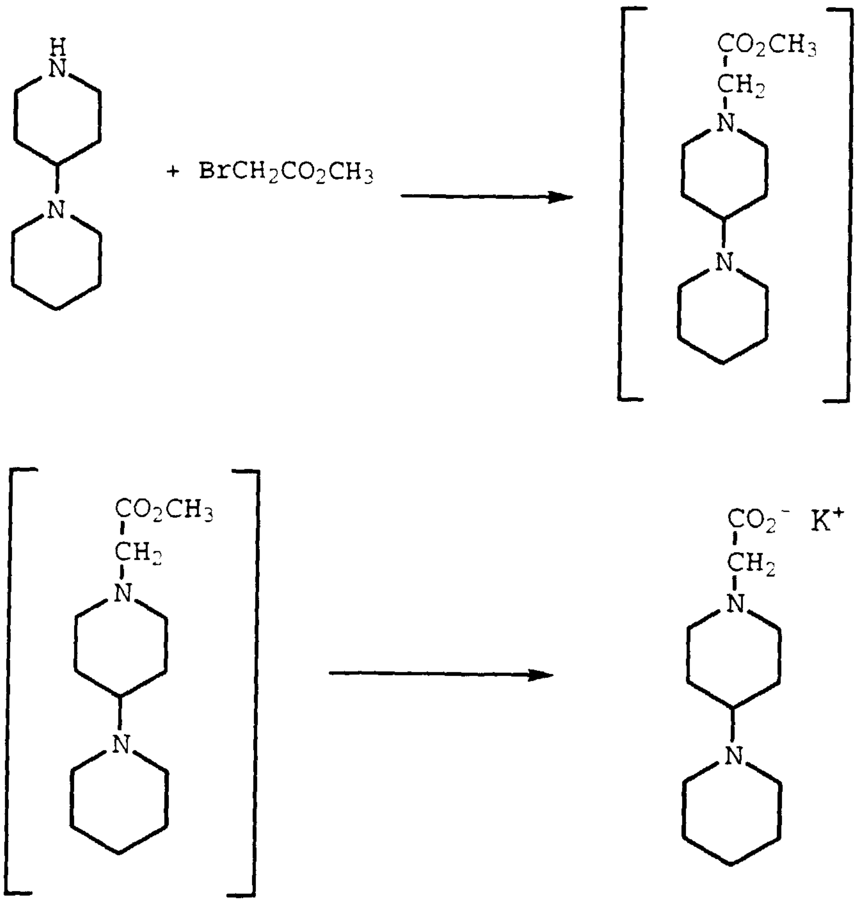


4-(Piperidin-1-yl)piperidine (1.20 kg, 7.13 mol) was added to methylene chloride (12.0 L) under a nitrogen atmosphere. Tetrabutylammonium bromide (0.150 kg, 0.47 mol) and sodium hydroxide (1.7 L of a 5 N solution, 8.5 mol) were then added. The reaction mixture was cooled to 10-15°C and methyl bromoacetate (1.17 kg, 7.65 mol) was added and the resulting mixture was stirred for a minimum of 16 hours.

Deionized water (1.2 L) was then added to the mixture and the layers separated. The aqueous layer was back-extracted with methylene chloride (2.4 L). The organic fractions were combined and washed with deionized water (3 x 1.2 L), a saturated sodium bicarbonate solution (1.1 L) and a saturated sodium chloride solution (1.1 L). The organic fraction was then dried over anhydrous magnesium sulfate and concentrated to an oil on a rotary evaporator to yield 1.613 kg (93.5%) of methyl 2-(4-(piperidin-1-yl)piperidin-1-yl)acetate.

A solution of methyl 2-[4-(piperidin-1-yl)piperidin-1-yl]acetate (2.395 kg, 9.96 mol) in methanol (2.4 L) was added to a solution of potassium hydroxide (0.662 kg, 10.0 mol @ 85% purity) in methanol (10.5 L) under a nitrogen atmosphere. The reaction mixture was heated to 45-50°C for a minimum of 16 hours.

A solvent exchange from methanol to acetone (15.0 L) was performed on the solution on a rotary evaporator. This solution was slowly cooled to room temperature over 16 hours. The resulting solids were filtered, rinsed with acetone (5.0 L) and then dried to yield 2.471 kg (93.8%) of 2-(4-(piperidin-1-yl)piperidin-1-yl)acetic acid, potassium salt. MS 265 ($M^{+1}$)

Preparation of (R)-3-(1H-indol-3-yl)-1-[N-(2-methoxybenzyl)acetylamino]-2-[N-(2-(4-(piperidin-1-yl)piperidin-1-yl) acetyl)amino]propane dihydrochloride trihydrate

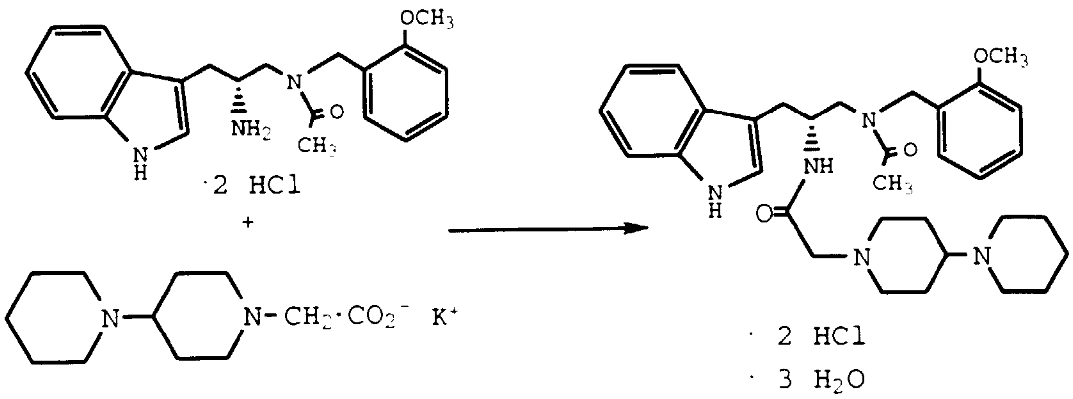

Under a nitrogen atmosphere 2-(4-(piperidin-1-yl)piperidin-1-yl)acetic acid, potassium salt (0.75 kg, 2.84 mol) was added to methylene chloride (7.5 L). The resulting mixture was cooled to -15 to -8°C and isobutyl chloroformate (0.29 kg, 2.12 mol) was added at such a rate so as to maintain the temperature of the reaction mixture below -8°C. After the addition the resulting reaction mixture was stirred for 90 minutes between -15 and -8°C.

The reaction mixture was then cooled to -35°C and solid (R)-2-amino-3-(1H-indol-3-yl)-1-[N-(2-methoxybenzyl) amino]propane dihydrochloride (0.60 kg, 1.14 mol) was added at such a rate that the reaction temperature was maintained at less than -20°C. After the addition, the reaction mixture was stirred for about one hour with the temperature being maintained between -37°C and -20°C. The reaction was quenched by the addition of deionized water (7.5 L). The reaction mixture was basified to pH 12.8-13.2 by the addition of 5 N sodium hydroxide. The aqueous fraction was removed and retained. Additional deionized water (3.75 L) was added to the organic fraction as was sufficient 5 N sodium hydroxide to re-adjust the pH to 12.8-13.2.

The two aqueous fractions were combined, back-extracted with methylene chloride (1.5 L) and then discarded. The organic fractions were combined and washed with deionized water (4 x 3.5 L). These extracts were combined, back-extracted with methylene chloride (1.5 L), and then discarded. The two organic layers were combined and washed with a saturated sodium chloride solution (3.7 L).

The organic fraction was dried over anhydrous magnesium sulfate, filtered, and solvent exchanged from methylene chloride to acetone (3.75 L) on a rotary evaporator. An aqueous solution of hydrochloric acid (0.48 L of 6 N solution, 2.88 mol) and seed crystals (2 g) were added and mixture was stirred for 30-90 minutes. Acetone (13.2 L) was then added and the slurry stirred for one hour. The resulting solid was then filtered, washed with acetone (2 x 1.4 L), and dried to yield 633 g (90%) of (R)-3-(1H-indol-3-yl)-1-[N-(2-methoxybenzyl)acetylamino]-2-[N-(2-(4-(piperidin-1-yl)piperidin-1-yl)acetyl)amino]propane dihydrochloride trihydrate.

Preparation of (R)-3-(1H-indol-3-yl)-1-[N-(2-methoxybenzyl)acetylamino]-2-[N-(2-(4-(piperidin-1-yl)piperidin-1-yl) acetyl)amino]propane dioxalate

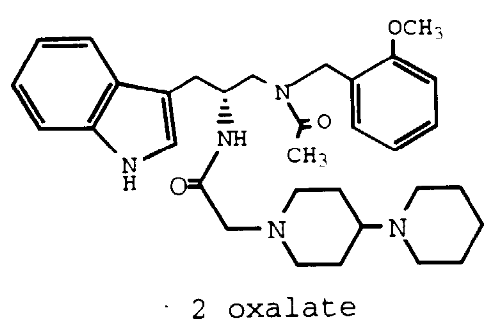

Into a 500 ml jacketed round bottom flask was placed 2-(4-(piperidin-1-yl)piperidin-1-yl)acetic acid, potassium salt (25.0 g, 94.5 mmol) and 375 ml of N,N-dimethylformamide. The resulting slurry was cooled to -19°C and isobutylchloroformate (12.9 g, 94.5 mmol) was added over five minutes. The resulting mixture was stirred for twenty minutes and then (R)-2-amino-3-(1H-indol-3-yl)-1-[N-(2-methoxybenzyl)acetylamino]propane dihydrochloride (25.0 g, 58.1 mmol), dissolved in 75 ml of anhydrous N,N-dimethylformamide, was added over ten minutes.

The resulting mixture is then cooled to 0°C, stirred for about ten minutes, and then permitted to warm to room temperature. The progress of the reaction was monitored by chromatography. High performance liquid chromatography showed 99% conversion of the reactants after ninety minutes.

The reaction mixture was partitioned between ethyl acetate (375 ml) and a saturated sodium bicarbonate solution (375 ml). The aqueous layer was back extracted with 375 ml of ethyl acetate. The organic fractions were combined, washed with water (3 x 375 ml), and then dried over magnesium sulfate. Potassium hydroxide is then added to the aqueous fraction from above and this resulting basified solution is extracted with ethyl acetate. This organic fraction is then dried over magnesium sulfate.

The combined dried organic fractions are then treated with a concentrated oxalic acid solution. The resulting solids are filtered and dried at 50°C in a vacuum oven to yield 23.5 grams of the desired intermediate.

As would be appreciated by a skilled practitioner the mixed anhydride process will work in a number of organic solvents, in addition to the anhydrous N,N-dimethylformamide depicted above. Representative examples of solvents which may be employed include acetonitrile, tetrahydrofuran, dichloromethane. The mixed anhydride process can be performed at temperatures below 0°C.

The oxalate can be isolated from ethyl acetate as well as from other solvents, probably including acetone, acetonitrile, and *t*-butyl methyl ether. The use of oxalic acid is, however, very important for the precipitation as a large number of acids do not give a precipitate. Among those acids attempted, but found not satisfactory for the processes of the present invention, are citric, anhydrous hydrochloric, tartaric, mandelic, trifluoroacetic, p-nitrobenzoic, phenoxyacetic, maleic, fumaric, glutaric, adipic, methanesulfonic, p-toluenesulfonic, pamoic, trans-1,2-cyclohexane dicarboxylic, succinic, phthalic, trans-1,2-diaminocyclohexane-N,N,N',N'-naphthalenedisulfonic, and 5-sulfosalicylic acids. Only oxalic acid and 1,5-naphthalene disulfonic acid reproducibly produced a solid.

Preparation of (R)-3-(1H-indol-3-yl)-1-[N-(2-methoxybenzyl)acetylamino]-2-[N-(2-(4-(piperidin-1-yl)piperidin-1-yl) acetyl)amino]propane dihydrochloride trihydrate

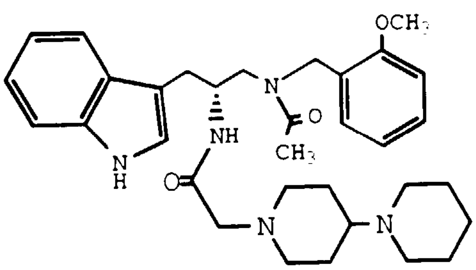

$\cdot 2\ HCl$

$\cdot 3\ H_2O$

Into a flask were added (R)-3-(1H-indol-3-yl)-1-[N-(2-methoxybenzyl)acetylamino]-2-[N-(2-(4-(piperidin-1-yl)piperidin-1-yl)acetyl)amino]propane oxalate (3.31 g, 4.22 mmol), methylene chloride (30 ml, 39.75 g), and water (30 ml). The resulting mixture was stirred and the pH of the reaction mixture was adjusted to 10-12 using 50% caustic.

The phases were separated and the aqueous phase was extracted with methylene chloride (20 ml) and separated. The combined organic fractions were back extracted with water (30 ml) and dried over magnesium sulfate. The methylene was removed on an evaporator, leaving a residue. This residue was transferred to a jacketed flask and dissolved into acetone (24 g, 10.25 volumes).

Enough water was added to bring the water concentration to eleven percent (by weight) and the resulting mixture was heated to 55°C. Enough concentrated hydrochloric acid was added to lower the pH to 2.0 and the reaction mixture was then permitted to cool to 37°C over 45 minutes.

The product solution was seeded and permitted to stir for 10-30 minutes. The product solution was cooled to 19°C over two hours and acetone (11.8 equivalent volumes) was added over three hours, after which time the reaction mixture was stirred for one to three hours, maintaining the temperature at 19°C. The product solution was filtered and the residue was washed with 10.2 equivalents (by volume) of acetone. The residue was then dried in a vacuum oven at 42°C to give the desired title product. Yield 2.407 grams (80.7%).

The following table illustrates many of the compounds produced using essentially the steps described in Schemes

I through IV. A person of ordinary skill in the art would readily understand that a certain order of steps must be employed in many instances to avoid reactions other than the one sought. For example, as in the above methods, it is frequently necessary to employ a protecting group in order to block a reaction at a particular moiety.

The abbreviations used in the following table are commonly used in the field and would be readily understood by a practitioner in the field. For exmple, the abbreviation "Ph" refers to a phenyl group, "i-Pr" refers to an isopropyl group, "Me" describes a methyl group, "Et" refers to an ethyl group, "t-Bu" describes a *tert*-butyl group, and the like.

In the following table, the first column gives the example number of the compound. The next columns (may be one, two, or three columns) describe the substitution patterns of the particular example. The column entitled "Mp °C" gives the melting point of the compound if it is a solid or notes the form of the substance at ambient temperature. The next column, entitled "MS", defines the mass of the compound as determined by mass spectroscopy. The following column gives the nuclear magnetic resonance profile of the example compound as synthesized. The final columns give the molecular formula of the example compound as well as its elemental analysis.

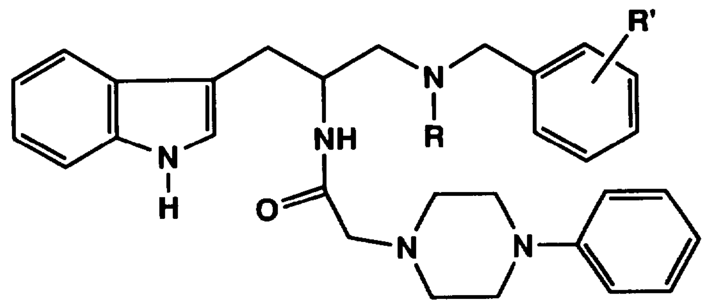

| Example No. | R | R' | Mp °C | MS | $^1$H NMR | Formula | Analysis % Theory/Found C | H | N |
|---|---|---|---|---|---|---|---|---|---|
| 1 | H | H | foam | 481 ($M^+$) | $CDCl_3$ 2.28 (m, 1H), 2.32-2.45 (m, 2H), 2.45-2.61 (m, 2H), 2.73 (m, 1H), 2.79-3.15 (m, 8H), 3.21 (m, 1H), 3.96 (ABq, J=8 Hz, Δv=20 Hz, 2H), 4.50 (m, 1H), 6.78-6.99 (m, 3H), 7.04 (m, 1H), 7.10-7.59 (m, 11H), 7.66 (d, J=8 Hz, 1H), 8.10 (br s, 1H) | $C_{30}H_{35}N_5O$ | 74.81<br>74.83 | 7.32<br>7.38 | 14.54<br>14.67 |
| 2 | H | 2-Cl | foam | 515, 517 ($M^+$'s for Cl isotopes) | DMSO-$d_6$ 2.33-2.50 (m, 4H), 2.56-2.75 (m, 2H), 2.75-3.09 (m, 8H), 3.20 (m, 1H), 4.78 (s, 2H), 5.21 (m, 1H), 6.78 (t, J=8 Hz, 1H), 6.88 (d, J=8 Hz, 2H), 6.98 (t, J=8 Hz, 1H), 7.06 (t, J=8 Hz, 1H), 7.13 (m, 1H), 7.13-7.31 (m, 4H), 7.34 (d, J=7 Hz, 1H), 7.39 (dd, J=2, 6 Hz, 1H), 7.50 (dd, J=2, 7 Hz, 1H), 7.55 (d, J=8 Hz, 1H), 7.61 (d, J=7 Hz, 1H), 10.81 (br s, 1H) | | | | |

| Example No. | R | R' | Mp °C | MS | $^1$H NMR | Formula | Analysis % Theory/Found C | H | N |
|---|---|---|---|---|---|---|---|---|---|
| 3 | H | 2-$CF_3$ | foam | 549 ($M^+$) Exact Mass FAB theory: 550.2794 found: 550.2801 | $CDCl_3$ 2.12 (m, 1H), 2.36-2.44 (m, 2H), 2.44-2.60 (m, 2H), 2.77-3.09 (m, 10H), 4.02 (s, 2H), 4.50 (m, 1H), 6.73-7.00 (m, 3H), 7.00-7.56 (m, 9H, 7.56-7.85 (m, 3H), 8.16 (br s, 1H) | $C_{31}H_{34}F_3N_5O$ | | | |
| 4 | H | 2-OMe (RS) | foam | 512 ($M+1^+$) | $CDCl_3$ 2.30-2.43 (m, 2H), 2.43-2.54 (m, 2H), 2.70-3.10 (m, 11H), 3.82 (s, 3H), 3.84 (m, 2H), 4.44 (m, 1H), 6.74-6.94 (m, 6H), 7.04 (m, 1H), 7.07-7.36 (m, 7H), 7.64 (d, J=8 Hz, 1H), 8.09 (br s, 1H) | $C_{31}H_{37}N_5O_2$ | 72.77<br>72.49 | 7.29<br>7.33 | 13.69<br>13.90 |
| 5 | H | 2-OMe (R) | foam | 512 ($M+1^+$) | $CDCl_3$ 2.30-2.43 (m, 2H), 2.43-2.56 (m, 2H), 2.64-3.12 (m, 11H), 3.59-3.93 (m, 2H), 3.82 (s, 3H), 4.43 (m, 1H), 6.68-6.96 (m, 6H), 7.03 (m, 1H), 7.07-7.45 (m, 7H), 7.66 (d, J=8 Hz, 1H), 8.04 (br s, 1H) | $C_{31}H_{37}N_5O_2$ | 72.77<br>72.58 | 7.29<br>7.39 | 13.69<br>13.65 |
| 6 | H | 2-OMe (S) | foam | 512 ($M+1^+$) | $CDCl_3$ 2.22-2.38 (m, 2H), 2.38-2.50 (m, 2H), 2.50-3.27 (m, 11H), 3.84 (s, 3H), 3.96 (ABq, J=13 Hz, Δv=21 Hz, 2H), 4.27 (m, 1H), 6.75-6.97 (m, 6H), 6.99-7.39 (m, 8H), 7.63 (d, J=8 Hz, 1H), 8.12 (br s, 1H) | $C_{31}H_{37}N_5O_2$ | 72.77<br>73.01 | 7.29<br>7.50 | 13.69<br>13.69 |

| Example No. | R | R' | Mp °C | MS | $^1$H NMR | Formula | Analysis % Theory/Found C | H | N |
|---|---|---|---|---|---|---|---|---|---|
| 7 | H | 3-OMe | foam | 511 ($M^+$) | $CDCl_3$ 7:3 mixture of amide rotamers 2.20-3.74 (m, 14H), 3.74 (m, 1H), 3.76 (s, 3/10•3H), 3.80 (s, 7/10•3H), 4.13 (ABq, J=14 Hz, Δv=50 Hz, 7/10•2H), 4.67 (m, 1H), 4.70 (ABq, J=14 Hz, Δv=160 Hz, 3/10•2H), 6.82-7.00 (m, 6H), 7.00-7.45 (m, 8H), 7.59 (d, J=8 Hz, 1H), 8.10 (br s, 3/10•1H), 8.41 (br s, 7/10•1H) | $C_{31}H_{37}N_5O_2$ | 72.77<br>73.00 | 7.29<br>7.19 | 13.69<br>13.91 |
| 8 | H | 4-OMe | foam | 511 ($M^+$) | $CDCl_3$ 2.21-2.63 (m, 4H), 2.63-2.90 (m, 4H), 2.90-3.40 (m, 6H), 3.75 (m, 1H), 3.77 (s, 3H), 4.04 (ABq, J=12 Hz, Δv=54 Hz, 2H), 4.64 (m, 1H), 6.83-6.95 (m, 5H), 6.95-7.48 (m, 8H), 7.50-7.75 (m, 2H), 8,23 (br s, 1H) | $C_{31}H_{37}N_5O_2$ | 72.77<br>72.58 | 7.29<br>7.35 | 13.69<br>13.70 |
| 9 | Et | 2-OMe | foam | 540 ($M+1^+$) | $CDCl_3$ 1.04 (t, J=8 Hz, 3H), 2.32-2.43 (m, 2H), 2.43-2.66 (m, 6H), 2.83-2.91 (m, 4H), 2.94 (d, J=5 Hz, 2H), 3.08 (t, J=6 Hz, 2H), 3.65 (ABq, J=14 Hz, Δv=22 Hz, 2H), 3.77 (s, 3H), 4.41 (q, J=6 Hz, 1H), 6.78-6.96 (m, 6H), 7.06-7.29 (m, 6H), 7.33 (d, J=8 Hz, 1H), 7.40 (d, J=7 Hz, 1H), 7.64 (d, J=8 Hz, 1H), 7.99 (br s, 1H) | $C_{33}H_{41}N_5O_2$ | 73.44<br>73.21 | 7.66<br>7.63 | 12.98<br>13.14 |

| Example No. | R | R' | Mp °C | MS | $^1$H NMR | Formula | Analysis % Theory/Found C | H | N |
|---|---|---|---|---|---|---|---|---|---|
| 10 | $MeO(OC)CH_2$ | 2-OMe | foam | 584 ($M+1^+$) | $CDCl_3$ 2.37-2.47 (m, 2H), 2.50-2.58 (m, 2H), 2.78-2.98 (m, 6H), 3.00 (s, 2H), 3.12 (t, J=6 Hz, 2H), 3.37 (ABq, J=18 Hz, Δv=26 Hz, 2H), 3.65 (s, 3H), 3.77 (s, 3H), 3.83 (s, 2H), 4.45 (m, 1H), 6.80-6.92 (m, 5H), 7.00 (s, 1H), 7.10-7.40 (m, 8H), 7.70 (d, J=9 Hz, 1H), 8.08 (s, 1H) | $C_{34}H_{41}N_5O_4$ | 69.96<br>69.69 | 7.08<br>6.98 | 11.99<br>11.87 |
| 11 | $HO(OC)CH_2$ | 2-OMe | 95-100 | 570 ($M+1^+$) | DMSO- $d_6$ 2.31-2.49 (m, 4H), 2.75 (d, J=8 Hz, 2H), 2.81-3.05 (m, 7H), 3.13-3.49 (m, 3H), 3.65-3.80 (m, 2H), 3.71 (s, 3H), 4.20 (m, 1H), 6.78 (t, J=8 Hz, 1H), 6.83-6.98 (m, 5H), 7.00-7.10 (m, 2H), 7.21 (t, J=8 Hz, 3H), 7.30 (t, J=9 Hz, 2H), 7.56 (br d, J=8 Hz, 2H), 10.81 (br s, 1H) | $C_{33}H_{39}N_5O_4$ | 69.57<br>69.80 | 6.90<br>6.79 | 12.29<br>11.99 |
| 12 | MeCO | H | foam | 523 ($M^+$) | DMSO-d6 1:1 mixture of amide rotamers 1.99 (s, 1/2•3H), 2.07 (s, 1/2•3H), 2.20-2.50 (m, 4H), 2.69-2.95 (m, 4H), 2.95-3.12 (m, 4H), 3.12-3.52 (m, 1/2•1H+1H), 3.63 (m, 1/2•1H), 4.40 (m, 1H), 4.51 (ABq, J=16 Hz, Δv=140 Hz, 1/2•2H), 4.54 (ABq, J=16 Hz, Δv=30 Hz, 1/2•2H), 6.78 (t, J=8 Hz, 1H), 6.86-6.94 (m, 2H), 6.98 (m, 1H), 7.03-7.15 (m, 4H), 7.15-7.38 (m, 6H), 7.50-7.60 (m, 1.5H), 7.74 (d, J=8 Hz, 1/2•1H), 10.93 (br s, 1H) | $C_{32}H_{37}N_5O_2$ | 73.39<br>73.67 | 7.12<br>7.23 | 13.37<br>13.60 |

| Example No. | R | R' | Mp °C | MS | $^1$H NMR | Formula | Analysis % Theory/Found C | H | N |
|---|---|---|---|---|---|---|---|---|---|
| 13 | MeCO | 2-Cl | foam | 557 ($M^+$) | DMSO-$d_6$ 3:2 mixture of amide rotamers 1.93 (s, 2/5•3H), 2.09 (s, 3/5•3H), 2.25-2.50 (m, 4H), 2.70-2.96 (m, 4H), 2.96-3.19 (m, 4H), 3.20-3.64 (m, 2H), 4.50 (m, 1H), 4.59 (ABq, J=16 Hz, Δν=70 Hz, 3/5•2H), 4.64 (s, 2/5•2H), 6.78 (t, J=7 Hz, 1H), 6.91 (d, J=8 Hz, 2H), 6.98 (t, J=7 Hz, 1H), 7.02-7.10 (m, 2H), 7.12 (m, 1H), 7.16-7.37 (m, 5H), 7.44 (m, 1H), 7.50-7.62 (m, 2/5•1H +1H), 7.75 (d, J=8 Hz, 3/5•1H), 10.83 (br s, 1H) | $C_{32}H_{36}ClN_5O_2$ | 68.86<br>69.06 | 6.50<br>6.48 | 12.55<br>12.56 |
| 14 | MeCO | 2-Me | | 538 ($M+1^+$) | $CDCl_3$ 2.06 (s, 3H), 2.21 (s, 3H), 2.1-2.6 (m, 2H), 2.9-3.3 (m, 12H), 3.58 (m, 1H), 4.4-4.6 (m, 2H), 6.8-7.0 (m, 5H), 7.0-7.4 (m, 9H), 7.62 (d, J=7 Hz, 1H), 8.15 (br s, 1H) | $C_{33}H_{39}N_5O_2$ | 73.71<br>74.00 | 7.31<br>7.37 | 13.02<br>13.21 |
| 15 | MeCO | 2-$CF_3$ | foam | 592 ($M+1^+$) | $CDCl_3$ 2.03 (s, 3H), 2.15-2.80 (m, 5H), 2.80-3.73 (m. 8H), 3.88 (m, 1H), 4.47-4.93 (m, 3H), 6.72-7.03 (m, 4H), 7.03-7.45 (m, 7H), 7.45-7.76 (m, 4H), 8.22 (br s, 1H) | $C_{33}H_{36}F_3N_5O_2$ | 66.99<br>66.83 | 6.13<br>6.20 | 11.84<br>12.10 |
| 16 | MeCO | 2-$NO_2$ | foam | 569 ($M+1^+$) | $CDCl_3$ 2.05 (s, 3H), 2.28 (m, 1H), 2.3-2.7 (m, 4H), 2.8-3.2 (m, 8H), 3.2-3.9 (m, 2H), 4.58 (m, 1H), 4.97 (m, 1H), 6.8-7.0 (m, 2H), 7.0-7.5 (m, 10H), 7.5-7.7 (m, 2H), 8.12 (d, J=7 Hz, 1H), 8.15 (br s, 1H) | $C_{32}H_{36}N_6O_4$ | 67.59<br>67.32 | 6.38<br>6.35 | 14.78<br>14.56 |

| Example No. | R | R' | Mp °C | MS | $^1$H NMR | Formula | Analysis % Theory/Found C | H | N |
|---|---|---|---|---|---|---|---|---|---|
| 17 | MeCO | 2-OMe (RS) | foam | 553 ($M^+$) | DMSO-$d_6$ 3:2 mixture of amide rotamers 1.97 (s, 1.8H), 2.07 (s, 1.2H), 2.26-2.50 (m, 4H), 2.70-2.96 (m, 4H), 2.96-3.16 (m, 4H), 3.16-3.65 (m, 2H), 3.72 (s, 2/5•3H), 3.74 (s, 3/5•3H), 4.40 (m, 1H), 4.42 (ABq, J=18 Hz, Δv=30 Hz, 3/5•2H), 4.46 (ABq, J=16 Hz, Δv=62 Hz, 2/5•2H), 6.70-7.03 (m, 7H), 7.03-7.13 (m, 2H), 7.13-7.29 (m, 3H), 7.34 (d, J=8 Hz, 1H), 7.49-7.62 (m, 3/5H+1H), 7.72 (d, J=6 Hz, 2/5H), 10.93 (br s, 1H) | $C_{33}H_{39}N_5O_3$ | 71.58<br>71.50 | 7.10<br>7.18 | 12.65<br>12.73 |
| 18 | MeCO | 2-OMe (R) | foam | 553 ($M^+$) Exact Mass FAB (M+1): calc.: 554.3131 found:. 554.3144 | $CDCl_3$ 2.11 (s, 3H), 2.41-2.43 (m, 2H), 2.50-2.55 (m, 2H), 2.87-3.18 (m, 9H), 3.78 (s, 3H), 4.02 (dd, J=10, 14 Hz, 1H), 4.51 (ABq, J=17 Hz, Δv=42 Hz, 2H), 4.59 (m, 1H), 6.80-6.98 (m, 6H), 7.07-7.45 (m, 8H), 7.68 (d, J=8 Hz, 1H), 8.14 (s, 1H) | $C_{33}H_{39}N_5O_3$ | 71.58<br>72.19 | 7.10<br>7.25 | 12.65<br>12.93 |

| Example No. | R | R' | Mp °C | MS | $^1$H NMR | Formula | Analysis % Theory/Found C | H | N |
|---|---|---|---|---|---|---|---|---|---|
| 19 | MeCO | 2-OMe (S) | foam | 553 ($M^+$) | DMSO-$d_6$ 3:2 mixture of amide rotamers 1.97 (s, 3/5•3), 2.07 (s, 2/5•3H), 2.23-2.60 (m, 4H), 2.71-2.95 (m, 4H), 2.95-3.17 (m, 4H), 3.17-3.80 (m, 2H), 3.71 (s, 3/5•2H), 3.74 (s, 3/5•3H), 4.26 (m, 1H), 4.44 (ABq, J=16 Hz, Δv=26 Hz, 3/5•2H), 4.45 (ABq, J=16 Hz, Δv=60 Hz, 2/5•2H), 6.70-7.02 (m, 7H), 7.02-7.12 (m, 2H), 7.12-7.30 (m, 3H), 7.34 (d, J=8 Hz, 1H), 7.56 (d, J=10 Hz, 3/5H+1H), 7.70 (d, J=10 Hz, 2/5•1H), 10.82 (br s, 1H) | $C_{33}H_{39}N_5O_3$ | 71.58<br>71.62 | 7.10<br>7.28 | 12.65<br>12.38 |
| 20 | MeCO | 3-F | 86-88 | 541 ($M^+$) | $CDCl_3$ 2.09 (s, 3H), 2.23 (m, 1H), 2.3-2.7 (m, 2H), 2.7-3.2 (m, 8H), 3.30 (m, 1H), 3.60 (m, 1H), 4.02 (m, 1H), 4.2-4.7 (m, 3H), 6.7-7.0 (m, 6H), 7.0-7.5 (m, 8H), 7.66 (d, J=7 Hz, 1H), 8.16 (br s,1H) | | | | |
| 21 | MeCO | 3-OMe | foam | 553 ($M^+$) | $CDCl_3$ 2.08 (s, 3H), 2.15-2.63 (m, 4H), 2.72-3.27 (m, 8H), 3.75 (m, 1H), 3.78 (s, 3H), 4.04 (m, 1H), 4.51 (ABq, J=16 Hz, Δv=46 Hz, 2H), 4.56 (m, 1H), 6.60-6.70 (m, 2H), 6.72-6.94 (m, 5H), 7.04-7.46 (m, 7H), 7.65 (d, J=8 Hz, 1H), 8.04 (br s, 1H) | $C_{33}H_{39}N_5O_3$ | 71.58<br>71.32 | 7.10<br>7.01 | 12.65<br>12.65 |

| Example No. | R | R' | Mp °C | MS | $^1$H NMR | Formula | Analysis % Theory/Found C | H | N |
|---|---|---|---|---|---|---|---|---|---|
| 22 | MeCO | 4-OMe | foam | 553 ($M^+$) | DMSO-$d_6$ 1:1 mixture of amide rotamers 2.01 (s, 1/2•3H), 2.05 (s, 1/2•3H), 2.23-2.60 (m, 4H), 2.74-3.30 (m, 8H), 3.69 (m, 1H), 3.72 (s, 1/2•3H), 3.74 (s, 1/2•3H), 4.23 (ABq, J=16 Hz, Δν=42 Hz, 1/2•2H), 4.52 (m, 1H), 4.36 (ABq, J=14 Hz, Δν=164 Hz, 1/2•2H), 6.70-7.16 (m, 10H), 7.24 (m, 2H), 7.35 (m, 1H), 7.55 (m, 1/2•1H+1H), 7.73 (m, 1/2•1H), 10.84 (br s, 1H) | $C_{33}H_{39}N_5O_3$ | 71.58<br>71.85 | 7.10<br>7.24 | 12.65<br>12.65 |
| 23 | MeCO | 4-SMe | dec 138 | 569 ($M^+$) | $CDCl_3$ 2.09 (s, 3H), 2.1-2.6 (m, 3H), 2.46 (s, 3H), 2.8-3.1 (m, 8H), 3.30 (m, 1H), 3.55 (m, 1H), 3.98 (m, 1H), 4.47 (ABq, J=12 Hz, Δν=52 Hz, 2H), 4.58 (m, 1H), 6.8-6.9 (m, 3H), 6.95 (d, J=8 Hz, 2H), 7.0-7.4 (m, 9H), 7.66 (d, J=8 Hz, 1H), 8.08 (br s, 1H) | $C_{33}H_{39}N_5O_2S$ | 69.57<br>69.86 | 6.90<br>6.93 | 12.29<br>12.33 |
| 24 | HCO | 2-OMe | foam | 540 (M+1) | $CDCl_3$ 2.33-2.47 (m, 2H), 2.50-2.65 (m, 2H), 2.87-3.10 (m, 9H), 3.75 (s, 3H), 3.77 (m, 1H), 4.40 (ABq, J=15 Hz, Δν=35 Hz, 2H), 4.65 (m, 1H), 6.75-6.95 (m, 6H), 7.03-7.42 (m, 8H), 7.67 (d, J=9 Hz, 1H), 8.20 (br s, 1H), 8.33 (s, 1H) | $C_{32}H_{37}N_5O_3$ | 71.21<br>70.99 | 6.91<br>6.96 | 12.98<br>13.25 |

| Example No. | R | R' | Mp °C | MS | $^1$H NMR | Formula | Analysis % Theory/Found C | H | N |
|---|---|---|---|---|---|---|---|---|---|
| 25 | $BrCH_2CO$ | 2-OMe | foam | 631, 633 ($M^+$'s for Br isotopes) Exact Mass FAB (M+1): calc.: 632.2236 found:. 632.2213 | $CDCl_3$ 2.37-2.47 (m, 2H), 2.53-2.63 (m, 2H), 2.90-3.17 (m, 8H), 3.80 (s, 3H), 3.95-4.13 (m, 2H), 3.98 (ABq, J=11 Hz, Δν=61 Hz, 2H), 4.57 (ABq, J=18 Hz, Δν=80 Hz, 2H), 4.67 (m, 1H), 6.78 (d, J=5 Hz, 1H), 6.80-6.90 (m, 4H), 7.07 (d, J=3 Hz, 1H), 7.10-7.30 (m, 6H), 7.37 (d, J=8 Hz, 1H), 7.50 (d, J=10 Hz, 1H), 7.70 (d, J=9 Hz, 1H), 8.07 (s, 1H) | $C_{33}H_{38}BrN_5O_3$ | | | |
| 26 | EtCO | 2-OMe | oil | 568 ($M+1^+$) | $CDCl_3$ 1.12 (t, J=9 Hz, 3H), 2.38 (q, J=9 Hz, 2H), 2.33-2.60 (m, 4H), 2.83-3.13 (m, 8H), 3.22 (br d, J=13 Hz, 1H), 3.80 (s, 3H), 4.03 (br t, J=13 Hz, 1H), 4.55 (ABq, J=20 Hz, Δν=40 Hz, 2H), 4.60 (m, 1H), 6.83-6.97 (m, 6H), 7.10-7.57 (m, 8H), 7.68 (d, J=8 Hz, 1H), 8.24 (br s, 1H) | $C_{34}H_{41}N_5O_3$ | 71.93<br>72.17 | 7.28<br>7.42 | 12.34<br>12.10 |
| 27 | PhCO | 2-OMe | foam | 615 ($M^+$) | $CDCl_3$ 2.28-2.57 (m, 4H), 2.77-3.17 (m, 9H), 3.65 (s, 3H), 4.22 (t, J=13 Hz, 1H), 4.60 (ABq, J=15 Hz, Δν=30 Hz, 2H), 4.82 (m, 1H), 6.70-6.92 (m, 5H), 7.02-7.55 (m, 14H), 7.68 (d, J=7 Hz, 1H), 8.22 (br s, 1H) | $C_{38}H_{41}N_5O_3$ | 74.12<br>74.38 | 6.71<br>6.87 | 11.37<br>11.32 |

| Example No. | R | R' | Mp °C | MS | $^1$H NMR | Formula | Analysis % Theory/Found C | H | N |
|---|---|---|---|---|---|---|---|---|---|
| 28 | EtOCO | 2-OMe | foam | 584 (M+1) | DMSO- $d_6$ 1.05 (t, J=8 Hz, 3H), 2.31- 2.45 (m, 4H), 2.73-2.90 (m 4H), 2.93-3.10 (m 4H), 3.22-3.48 (m, 2H), 3.66 (s, 3H), 3.87-4.03 (m, 2H), 4.26-4.55 (m, 3H), 6.77 (t, J=7 Hz, 1H), 6.80-7.00 (m, 6H), 7.05 (t, J=8 Hz, 1H), 7.11 (br s, 1H), 7.20 (t, J=9 Hz, 3H), 7.32 (d, J=10 Hz, 1H), 7.52 (br d, J=6 Hz, 2H) | $C_{34}H_{41}N_5O_4$ | 69.96<br>69.85 | 7.08<br>7.19 | 12.00<br>11.98 |
| 29 | MeNHCO | 2-OMe | oil | 568 ($M^+$) | DMSO-d6 2.32-2.46 (m, 4H), 2.55 (d, J=5 Hz, 3H), 2.78-2.90 (m, 4H), 2.96-3.10 (m, 4H), 3.18 (dd, J=5, 14 Hz, 1H), 3.44 (dd, J=8, 13 Hz, 1H), 3.70 (s, 3H), 4.30 (m, 1H), 4.37 (ABq, J=18 Hz, Δv=42 Hz, 2H), 6.32 (br d, J=5 Hz, 1H), 6.77 (t, J=7 Hz, 1H), 6.82-7.00 (m, 6H), 7.05 (t, J=8 Hz, 1H), 7.11 (d, J=3 Hz, 1H), 7.16-7.25 (m, 3H), 7.32 (d, J=9 Hz, 1H), 7.53 (d, J=8 Hz, 1H), 7.61 (d, J=9 Hz, 1H), 10.82 (br s, 1H) | $C_{33}H_{40}N_6O_3$ | 69.69<br>69.94 | 7.09<br>7.13 | 14.78<br>14.83 |

| Example No. | R | R' | Mp °C | MS | $^1$H NMR | Formula | Analysis % Theory/Found C | H | N |
|---|---|---|---|---|---|---|---|---|---|
| 30 | $MeO(OC)CH_2CO$ | 2-OMe | foam | 611 ($M^+$) | $CDCl_3$ 2.37-2.47 (m, 2H), 2.50-2.60 (m, 2H), 2.82-3.18 (m, 9H), 3.57 (s, 2H), 3.72 (s, 3H), 3.78 (s, 3H), 4.02 (dd, J=10, 14 Hz, 1H), 4.47 (ABq, J=20 Hz, Δv=40 Hz, 2H), 4.60 (m, 1H), 6.77-6.92 (m, 6H), 7.03-7.30 (m, 6H), 7.37 (d, J=7 Hz, 1H), 7.45 (d, J=10 Hz, 1H), 7.68 (d, J=9 Hz, 1H), 8.12 (s, 1H) | $C_{35}H_{41}N_5O_5$ | 68.72<br>68.44 | 6.76<br>6.76 | 11.45<br>11.44 |
| 31 | $HO(OC)CH_2CO$ | 2-OMe | 103-107 | 598 ($M+1^+$) Exact Mass FAB (M+1): calc.: 598.3029 found:. 598.3046 | $CDCl_3$ 2.68-2.90 (m, 4H), 2.90-3.37 (m, 9H), 3.57 (br s, 2H), 3.78 (s, 3H), 3.93 (t, J=12 Hz, 1H), 4.53 (ABq, J=17 Hz, Δv=47 Hz, 2H), 4.70 (m, 1H), 6.77-6.97 (m, 6H), 7.07-7.33 (m, 7H), 7.37 (d, J=8 Hz, 1H), 7.63 (d, J=8 Hz, 1H), 7.85 (br s, 1H), 8.33 (br s, 1H) | $C_{34}H_{39}N_5O_5$ | | | |
| 32 | $Me(CO)OCH_2CO$ | 2-OMe | foam | 612 ($M+1^+$) | $CDCl_3$ 2.10 (s, 3H), 2.35-2.43 (m, 2H), 2.47-2.57 (m, 2H), 2.90-3.13 (m, 9H), 3.80 (s, 3H), 4.03 (dd, J=10, 15 Hz, 1H), 4.40 (ABq, J=19 Hz, Δv=30 Hz, 2H), 4.57 (m, 1H), 4.85 (ABq, J=15 Hz, Δv=19 Hz, 2H), 6.75-6.90 (m, 6H), 7.03 (d, J=2 Hz, 1H), 7.10-7.30 (m, 5H), 7.35-7.43 (m, 2H), 7.66 (d, J=9 Hz, 1H), 8.32 (br s, 1H) | $C_{35}H_{41}N_5O_5$ | 68.72<br>68.50 | 6.76<br>6.86 | 11.45<br>11.20 |

| Example No. | R | R' | Mp °C | MS | $^1$H NMR | Formula | Analysis % Theory/Found C | H | N |
|---|---|---|---|---|---|---|---|---|---|
| 33 | $HOCH_2CO$ | 2-OMe | foam | 569 ($M^+$) | $CDCl_3$ 2.35-2.57 (m, 4H), 2.80-3.17 (m, 9H), 3.52 (t, J=5 Hz, 1H), 3.75 (s, 3H), 4.08 (m, 1H), 4.27 (dd, J=5, 10 Hz, 2H), 4.33 (d, J=5 Hz, 2H), 4.63 (m, 1H), 6.73-6.92 (m, 6H), 7.03 (d, J=3 Hz, 1H), 7.12-7.32 (m, 5H), 7.33-7.40 (m, 2H), 7.67 (d, J=10 Hz, 1H), 8.07 (br s, 1H) | $C_{33}H_{39}N_5O_4 \cdot 0.5\ H_2O$ | 68.49<br>68.51 | 6.97<br>6.86 | 12.10<br>11.91 |
| 34 | $H_2NCH_2CO$ | 2-OMe | foam | 568 ($M^+$) | $CDCl_3$ 2.20 (m, 2H), 2.35-2.45 (m, 2H), 2.45-2.53 (m, 2H), 2.80-3.07 (m, 8H), 3.30 (dd, J=5, 15 Hz, 1H), 3.47-3.57 (m, 2H), 3.77 (s, 3H), 3.93 (dd, J=10, 15 Hz, 1H), 4.42 (ABq, J=20 Hz, Δv=30 Hz, 2H), 4.62 (m, 1H), 6.77-6.90 (m, 5H), 7.03-7.40 (m, 9H), 7.65 (d, J=8 Hz, 1H), 8.12 (br s, 1H) | $C_{33}H_{40}N_6O_3$ | 69.69<br>69.82 | 7.09<br>7.14 | 14.78<br>14.49 |
| 35 | $Me_2NCH_2CO$ | 2-OMe | foam | 596 ($M^+$) | $CDCl_3$ 2.30 (s, 6H), 2.32-2.50 (m, 4H), 2.87-3.05 (m, 8H), 3.20 (s, 2H), 3.33 (dd, J=6, 9 Hz, 1H), 3.78 (s, 3H), 3.85 (m, 1H), 4.58 (m, 1H) 4.65 (ABq, J=18 Hz, Δv=42 Hz, 2H), 6.81-6.93 (m, 6H), 7.10-7.40 (m, 8H), 7.65 (d, J=11 Hz, 1H), 8.17 (br s, 1H) | $C_{35}H_{44}N_6O_3$ | 70.44<br>70.15 | 7.43<br>7.39 | 14.08<br>14.02 |

| Example No. | R | R' | Mp °C | MS | $^1$H NMR | Formula | Analysis % Theory/Found C | H | N |
|---|---|---|---|---|---|---|---|---|---|
| 36 | t-Bu-O(CO)NH-$CH_2$CO | 2-OMe | foam | 668 ($M^+$) | $CDCl_3$ 1.43 (s, 9H), 2.33-2.57 (m, 4H), 2.82-3.12 (m, 8H), 3.17 (dd, J=5, 15 Hz, 1H), 3.77 (s, 3H), 3.93-4.10 (m, 3H), 4.42 (ABq, J=18 Hz, Δv=41 Hz, 2H), 4.60 (m, 1H), 5.50 (br s, 1H), 6.73-6.92 (m, 6H), 7.05 (s, 1H), 7.08-7.32 (m, 5H), 7.35 (d, J=10 Hz, 2H), 7.65 (d, J=10 Hz, 1H), 8.10 (br s, 1H) | $C_{38}H_{48}N_6O_5$ | 68.24<br>68.44 | 7.23<br>7.50 | 12.57<br>12.61 |
| 37 | $MeSO_2$ | 2-OMe | foam | 589 ($M^+$) | DMSO-$d_6$ 2.28-2.46 (m, 4H), 2.83 (d, J=7 Hz, 4H), 2.90 (s, 3H), 2.98-3.04 (m, 4H), 3.26-3.34 (m, 2H), 3.67 (s, 3H), 4.30 (m, 1H), 4.36 (d, J=5 Hz, 2H), 6.77 (t, J=8 Hz, 1H), 6.84-6.92 (m, 3H), 6.92-7.00 (m, 2H), 7.03-7.09 (m, 2H), 7.18-7.30 (m, 4H), 7.33 (d, J=8 Hz, 1H), 7.46 (d, J=8 Hz, 1H), 7.54 (d, J=9 Hz, 1H), 10.82 (br s, 1H) | $C_{32}H_{39}N_5O_4S$ | 65.17<br>64.88 | 6.67<br>6.72 | 11.88<br>11.60 |

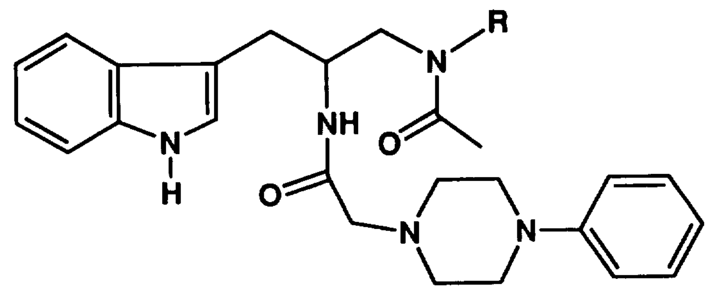

| Example No. | R | Mp °C | MS | ¹H NMR | Formula | Analysis Theory/Found C | H | N |
|---|---|---|---|---|---|---|---|---|
| 38 | Me | 128-129 | 447 ($M^+$) | $CDCl_3$ 2.07 (s, 3H), 2.38-2.78 (m, 3H), 2.8-3.3 (m, 11H), 3.42 (m, 1H), 3.67 (m, 1H), 3.95 (m, 1H), 4.58 (m, 1H), 6.8-7.0 (m, 3H), 7.1-7.4 (m, 7H), 7.68 (d, J=7 Hz, 1H), 8.21 (br s, 1H) | $C_{26}H_{33}N_5O_2$ | 69.77<br>69.59 | 7.43<br>7.52 | 15.65<br>15.65 |
| 39 | n-Bu | foam | 489 ($M^+$) | ¹H $CDCl_3$ 0.88 (t, J=6 Hz, 3H), 1.1-1.40 (m, 2H), 1.4-1.6 (m, 2H), 2.08 (s, 3H), 2.2-2.4 (m, 4H), 2.8-3.1 (m, 8H), 3.1-3.4 (m, 3H), 3.9 (m, 1H), 4.5 (br s, 1H), 6.8-7.0 (m, 3H), 7.0-7.5 (m, 7H), 7.68 (d, J=6 Hz, 1H), 8.31 (br s, 1H). | $C_{29}H_{39}N_5O_2$ | 71.13<br>71.40 | 8.03<br>8.05 | 14.30<br>14.41 |
| 40 | n-Hex | foam | 517 ($M^+$) | ¹H $CDCl_3$ 0.82-0.92 (m, 3H), 1.12-1.36 (m, 6H), 1.40-1.70 (m, 3H), 2.05 (s, 3H), 2.31-2.61 (m, 3H), 2.80-3.11 (m, 8H), 3.11-3.42 (m, 3H), 3.9 (m, 1H), 4.5 (m, 1H), 6.75-6.98 (m, 3H), 7.08-7.48 (m, 7H), 7.7 (m, 1H), 8.1 (brs, 1H). | $C_{31}H_{43}N_5O_2$ | 71.92<br>71.85 | 8.37<br>8.35 | 13.53<br>13.59 |
| 41 | (c-hexyl)$CH_2$ | foam | 530 ($M+1^+$) | $CDCl_3$ 0.65-1.02 (m, 2H), 1.02-1.36 (m, 3H), 1.36-1.87 (m, 9H), 2.07 (s, 3H), 2.15-3.70 m, 12H), 3.95 (m, 1H), 4.57 (m, 1H), 6.70-7.03 (m, 4H), 7.03-7.23 (m, 4H), 7.31-7.44 (m, 2H), 7.69 (d, J=10 Hz, 1H), 8.16 (br s, 1H) | $C_{32}H_{43}N_5O_2$ | 72.56<br>72.46 | 8.18<br>8.12 | 13.22<br>13.07 |

| Example No. | R | Mp °C | MS | $^1$H NMR | Formula | Analysis Theory/Found C | H | N |
|---|---|---|---|---|---|---|---|---|
| 42 | Ph | 183-184 | 509 ($M^+$) | $^1$H DMSO 1.71 (s, 3H), 2.23-2.43 (m, 4H), 2.71-2.94 (m, 4H), 2.94-3.10 (m, 4H), 3.61 (m, 1H), 4.03 (m, 1H), 4.24 (m, 1H), 6.77 (t, J=8 Hz, 1H), 6.92-6.99 (m, 3H), 6.99-7.12 (m, 2H), 7.21 (t, J=8 Hz, 2H), 7.24-7.35 (m, 3H), 7.4 (m, 1H), 7.40-7.54 (m, 4H), 10.92 (brs, 1H). | $C_{31}H_{35}N_5O_2$ | 73.04<br>73.30 | 6.92<br>7.11 | 13.74<br>13.73 |
| 43 | $PhCH_2CH_2$ | | 537 ($M^+$) | $^1$H DMSO (3:2 mixture of amide rotamers) 1.69 (s, 3/5•3H), 2.00 (s, 2/5•3H), 2.50-2.60 (m, 5H), 2.70-3.05 (m, 5H), 3.05-3.19 (m, 4H), 3.19-3.36 (m, 2H), 3.36-3.64 (m, 2H), 4.32 (m, 1H), 6.76 (t, J=8 Hz, 1H), 6.90 (d, J=8 Hz, 2H), 6.95-7.39 (m, 11H), 7.56 (m, 1H), 7.76 (m, 2/5•1H), 7.92 (m, 3/5•1H), 10.81 (br s, 2/5•1H), 10.85 (br s, 3/5•1H). | $C_{33}H_{39}N_5O_2$ | 73.71<br>73.95 | 7.31<br>7.45 | 13.02<br>13.07 |

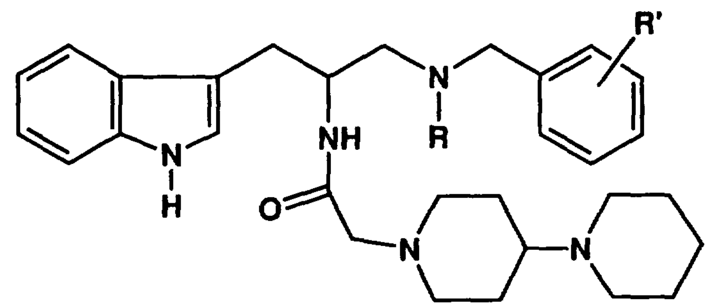

| Example No. | R | R' | Mp °C | MS | $^1H$ NMR | Formula | Analysis % Theory/Found C | H | N |
|---|---|---|---|---|---|---|---|---|---|
| 44 | H | 2-OMe (R) | foam | 517 ($M^+$) | $CDCl_3$ 1.10-2.18 (m, 12H), 2.18-3.18 (m, 14H), 3.61-3.95 (m, 2H), 3.93 (s, 3H), 4.36 (m, 1H), 6.76-6.96 (m, 3H), 7.04-7.44 (m, 5H), 7.42 (d, J=8 Hz, 1H), 7.65 (d, J=8 Hz, 1H), 9.13 (br s, 1H) | $C_{31}H_{43}N_5O_2$ | 71.92<br>71.69 | 8.37<br>8.25 | 13.53<br>13.26 |
| 45 | H | 2-OMe (S) | foam | 517 ($M^+$) | $CDCl_3$ 1.13-2.18 (m, 12H), 2.18-3.33 (m, 14H), 3.61-3.96 (m, 2H), 3.85 (s, 3H), 4.36 (m, 1H), 6.80-6.97 (m, 3H), 6.97-7.36 (m, 6H), 7.44 (d, J=8 Hz, 1H), 9.60 (br s, 1H) | $C_{31}H_{43}N_5O_2$ | 71.92<br>71.91 | 8.37<br>8.25 | 13.53<br>13.42 |

| Example No. | R | R' | Mp °C | MS | $^1$H NMR | Formula | Analysis % Theory/Found C | H | N |
|---|---|---|---|---|---|---|---|---|---|
| 46 | MeCO | H | foam | 530 ($M+1^+$) | $CDCl_3$ 3:1 mixture of amide rotamers 1.21-1.69 (m, 10H), 1.90-2.19 (m, 3H), 2.07 (s, 3/4•3H), 2.10 (s, 1/4•3H), 2.37-2.55 (m, 5H), 2.65-3.18 (m, 6H), 4.02 (dd, J=13 Hz, J=10 Hz, 1H), 4.50 (ABq, J=17 Hz, Δv=52 Hz, 3/4•2H), 4.67 (ABq, J=17 Hz, Δv=228 Hz, 1/4•2H), 4.55 (m, 1H), 6.94-7.44 (m, 10H), 7.65 (d, J=8 Hz, 3/4•1H), 7.53 (d, J=8 Hz, 1/4•1H), 8.08 (br s, 3/4•1H), 8.22 (br s, 1/4•1H). | $C_{32}H_{43}N_5O_2$ | 72.56<br>72.36 | 8.18<br>8.17 | 13.22<br>13.12 |
| 47 | MeCO | 2-Cl (RS) | foam | 563 ($M^+$) Exact Mass FAB theory: 564.3105 found: 564.3130 ($M^{+1}$) | $CDCl_3$ 1.17-1.80 (m, 10H), 1.90-2.27 (m, 3H), 2.03 (s, 3H), 2.35-2.59 (m, 5H), 2.67-3.23 (m, 6H), 3.97 (dd, J=10, 15 Hz, 1H), 4.53 (m, 1H), 4.58 (ABq, J=17 Hz, Δv=21 Hz, 2H), 6.95-7.29 (m, 6H), 7.34 (d, J=8 Hz, 2H), 7.42 (d, J=9 Hz, 1H), 7.63 (d, J=8 Hz, 1H), 8.19 (br s, 1H) | $C_{32}H_{42}ClN_5O_2$ | | | |
| 48 | MeCO | 2-Cl (R) | foam | 563 ($M^+$) | $^1$H $CDCl_3$ 1.1-1.8 (m, 10H), 1.8-2.3 (m, 4H), 2.04 (s, 3H), 2.4-2.6 (m, 3H), 2.6-2.8 (m, 2H), 2.8-2.9 (m, 2H), 2.9-3.1 (m, 2H), 3.2 (m, 1H), 3.9 (m, 1H), 4.5-4.7 (m, 3H), 7.0-7.6 (m, 9H), 7.62 (d, J=6 Hz, 1H), 8.32 (br s, 1H). | $C_{32}H_{42}ClN_5O_2$ | 68.13<br>68.20 | 7.50<br>7.60 | 12.41<br>12.17 |
| 49 | MeCO | 2-Cl (S) | foam | 563 ($M^+$) | $^1$H $CDCl_3$ 1.3-1.8 (m, 6H), 2.04 (s, 3H), 1.8-2.1 (m, 3H), 2.1-2.3 (m, 3H), 2.4-2.6 (m, 5H), 2.7-2.8 (m, 2H), 2.86 (d, J=2 Hz, 2H), 2.9-3.1 (m, 2H), 3.2 (m, 1H), 3.9 (m, 1H), 4.5-4.7 (m, 3H), 7.0-7.5 (m, 9H), 7.63 (d, J=7 Hz, 1H), 8.38 (br s, 1H) | $C_{32}H_{42}ClN_5O_2$ | 68.13<br>68.40 | 7.50<br>7.61 | 12.41<br>12.60 |

| Example No. | R | R' | Mp °C | MS | $^1$H NMR | Formula | Analysis % Theory/Found C | H | N |
|---|---|---|---|---|---|---|---|---|---|
| 50 | MeCO | 2-OMe (RS) | foam | 559 ($M^+$) | $CDCl_3$ 1.30-1.86 (m, 10H), 1.93-2.32 (m, 3H), 2.10 (s, 3H), 2.45-2.67 (m, 4H), 2.71-3.18 (m, 5H), 2.87 (s, 2H), 3.76 (s, 3H), 3.99 (dd, J=14 Hz, J=10 Hz, 1H), 4.49, (ABq, J=17 Hz, $\Delta\nu$=41 Hz, 2H), 4.55 (m, 1H), 6.79-6.93 (m, 3H), 7.06-7.27 (m, 4H), 7.36 (d, J=8 Hz, 1H), 7.45 (d, J=9 Hz, 1H), 7.66 (d, J=8 Hz, 1H), 8.28 (br s, 1H) | $C_{33}H_{45}N_5O_3$ | 70.81<br>70.95 | 8.10<br>8.05 | 12.51<br>12.45 |
| 51 | MeCO | 2-OMe (R) | | 559 ($M+1^+$) | DMSO-$d_6$ 3:2 mixture of amide rotamers, 1.25-1.70 (m, 10H), 1.77-2.00 (m, 2H), 1.95 (s, 3/5•3HH), 2.04 (s, 2/5•3HH), 2.10-2.97 (m, 9H), 3.10-3.65 (m, 3H), 3.72 (s, 2/5•3HH), 3.74 (s, 3/5•3HH), 4.26-4.58 (m, 3H), 6.76-7.12 (m, 6H), 7.13-7.35 (m, 2H), 7.42-7.66 (m, 2H), 10.80 (br s, 1H) | $C_{33}H_{45}N_5O_3$ | 70.81<br>70.57 | 8.10<br>8.05 | 12.51<br>12.39 |
| 52 | MeCO | 2-OMe (S) | | 559 ($M+1^+$) | DMSO-$d_6$ 3:2 mixt. of amide rotamers, 1.15-1.68 (m, 10H), 1.68-2.20 (m, 3H), 1.95 (s, 3/5•3HH), 2.04 (s, 2/5•3HH), 2.20-3.00 (m, 9H), 3.00-3.65 (m, 3H), 3.74 (s, 2/5•3HH), 3.76 (s, 3/5•3HH), 4.20-4.60 (m, 3H), 6.75-7.15 (m, 6H), 7.15-7.40 (m, 2H), 7.40-7.68 (m, 2H), 10.78 (br s, 1H) | $C_{33}H_{45}N_5O_3$ | 70.81<br>71.01 | 8.10<br>8.39 | 12.51<br>12.63 |

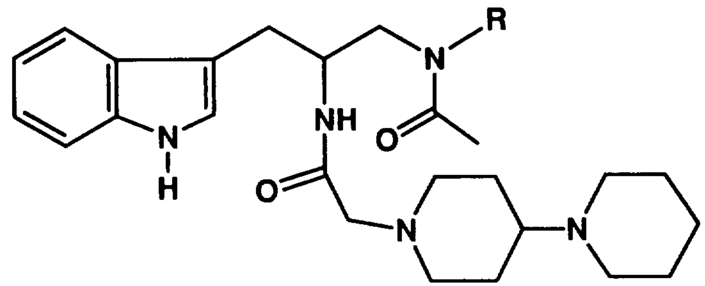

| Example No. | R | Mp °C | MS | $^1$H NMR | Formula | Analysis, % Theory/Found C | H | N |
|---|---|---|---|---|---|---|---|---|
| 53 | Ph | 140-141 | 515 ($M^+$) | $^1$H DMSO 1.21-1.58 (m, 10H), 1.70 (s, 3H), 1.87 (ABq, J=8 Hz, Δv=20 Hz, 2H), 2.04 (m, 1H), 2.29-2.49 (m, 4H), 2.45-2.64 (m, 2H), 2.63-2.79 (m, 2H), 2.79-2.95 (m, 2H), 3.58 (m, 1H), 4.02 (t, J=12 Hz, 1H), 4.20 (m, 1H), 6.93 (t, J=8 Hz, 1H), 6.98-7.11 (m, 2H), 7.17-7.53 (m, 8H), 10.91 (br s, 1H). | $C_{31}H_{41}N_5O_2$ | 72.20<br>71.98 | 8.01<br>8.07 | 13.58<br>13.53 |
| 54 | $PhCH_2CH_2$ | foam | 543 ($M^+$) | $^1$H DMSO (3:2 mixture of amide rotamers) 1.23-1.57 (m, 10H), 1.75-1.97 (m, 2H), 1.84 (s, 3/5•3H), 1.93 (s, 2/5•3H), 2.05 (m, 1H), 2.23-2.47 (m, 4H), 2.50-2.77 (m, 6H), 2.77-2.95 (m, 2H), 3.20-3.35 (m, 1H), 3.36-3.52 (m, 2H), 3.62 (m, 1H), 4.39 (m, 1H), 6.97 (m, 1H), 7.02-7.31 (m, 7H), 7.34 (d, J=8 Hz, 1H), 7.45 (d, J=8 Hz, 3/5H), 7.53-7.67 (m, 2/5•1H+1H), 10.84 (br s, 1H). | $C_{33}H_{45}N_5O_2$ | 72.89<br>72.60 | 8.34<br>8.29 | 12.88<br>12.64 |

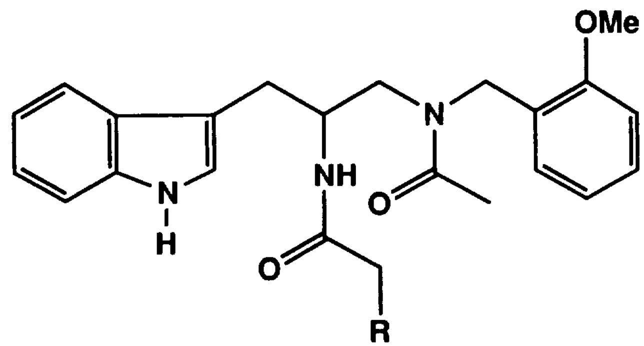

| Example No. | R | Mp, °C | MS | $^1$H NMR | Formula | Analysis, % Theory/Found C | H | N |
|---|---|---|---|---|---|---|---|---|
| 55 | Br (R) | foam | 473 (M+) | $CDCl_3$ 2.15 (s, 3H), 2.81 - 2.96 (m, 2H), 3.15 ($AB_q$, J=4.3 Hz, Δv=14.6 Hz, 1H), 3.72 (s, 3H), 3.79 (s, 2H), 4.06 - 4.15 (m, 1H), 4.30 (m, 1H), 4.38 ($AB_q$, J=16.7 Hz, Δv=49.0 Hz, 2H), 6.72 - 6.81 (m, 3H), 7.01 (s, 1H), 7.13 - 7.30 (m, 3H), 7.35 - 7.41 (m, 2H), 7.71 (d, J=7.8 Hz, 1H), 8.04.1H). | $C_{23}H_{26}N_3O_{32}Br$ | 58.48<br>58.69 | 5.55<br>5.66 | 8.90<br>8.94 |
| 56 | PhO | foam | 485 ($M^+$) | $CDCl_3$ 2.00 (s, 3H), 2.86 (dd, J=8, 14 Hz, 1H), 3.01 (dd, J=5, 14 Hz, 1H), 3.20 (dd, J=5, 15 Hz, 1H), 3.70 (s, 3H), 4.04 (dd, J=10, 14 Hz, 1H), 4.34 (ABq, J=18 Hz, Δv=44 Hz, 2H), 4.44 (ABq, J=15 Hz, Δv=25 Hz, 2H), 4.42 (m, 1H), 6.70-6.85 (m, 3H), 6.85-7.06 (m, 4H), 7.06-7.45 (m, 6H), 7.54 (m, 1H), 7.71 (d, J=8 Hz, 1H), 7.97 (br s, 1H) | $C_{29}H_{31}N_3O_4$ | 71.73<br>71.48 | 6.43<br>6.59 | 8.65<br>8.46 |

| Example No. | R | Mp, °C | MS | $^1H$ NMR | Formula | Analysis, % Theory/Found C | H | N |
|---|---|---|---|---|---|---|---|---|
| 57 | PhS | foam | 501 ($M^+$) | $CDCl_3$ 1.92 (s, 3H), 2.76 (dd, J=8, 14 Hz, 1H), 2.92 (dd, J=4, 14 Hz, 1H), 3.06 (dd, J=4, 14 Hz, 1H), 3.57 (s, 2H), 3.69 (s, 3H), 3.99 (dd, J=8, 14 Hz, 1H), 4.29 (ABq, J=16 Hz, Δv=44 Hz, 2H), 4.36 (m, 1H), 6.65 (m, 3H), 6.85 (d, J=3 Hz, 1H), 7.05-7.37 (m, 9H), 7.42 (m, 1H), 7.67 (d, J=8 Hz, 1H), 7.85 (br s, 1H) | $C_{29}H_{31}N_3O_3S$ | 69.44<br>69.55 | 6.23<br>6.49 | 8.38<br>8.10 |
| 58 | $PhNHCH_2CH_2NH$ | foam | 528 ($M+1^+$) | $CDCl_3$ 2.11 (s, 3H), 2.72-2.95 (m, 4H), 3.00-3.34 (m, 6H), 3.72 (s, 3H), 4.14 (dd, J=11, 13 Hz, 1H), 4.40 (ABq, J=17 Hz, Δv=63 Hz, 2H), 4.42 (m, 1H), 4.78 (br s, 1H), 6.65-6.84 (m, 6H), 6.95 (d, J=3 Hz, 1H), 7.07-7.35 (m, 6H), 7.67 (d, J=8 Hz, 1H), 7.80-7.91 (m, 2H). | $C_{31}H_{37}N_5O_3$ | 70.56<br>70.35 | 7.07<br>7.03 | 13.27<br>13.06 |

| Example No. | R | Mp, °C | MS | $^1$H NMR | Formula | Analysis, % Theory/Found C | H | N |
|---|---|---|---|---|---|---|---|---|
| 59 | 1-pyrrolidinyl | foam | 463 (M+1$^+$) | $CDCl_3$ 1.66-1.74 (m, 4H), 2.11 (s, 3H), 2.47 (m, J=19 Hz, 4H), 2.86-3.17 (m, 5H), 3.74 (s, 3H), 4.00 (dd, J=11, 14 Hz, 1H), 4.46 (ABq, J=17 Hz, Δν=46 Hz, 2H), 4.52 (br s, 1H), 6.76-6.83 (m, 2H), 7.08-7.28 (m, 3H), 7.18 (s, 1H), 7.35 (d, J=8 Hz, 1H), 7.52 (d, J=8 Hz, 1H), 7.69 (d, J=8 Hz, 1H), 8.38 (br s, 1H) | $C_{27}H_{34}N_4O_3$ | 70.10<br>70.42 | 7.41<br>7.29 | 12.11<br>11.75 |
| 60 | 1-piperidinyl | foam | 476 (M$^+$) | $CDCl_3$ 1.37-1.56 (m, 6H), 2.09 (s, 3H), 2.30 (br s, 4H), 280-3.19 (m, 5H), 3.75 (s, 3H), 3.95 (dd, J=11, 13 Hz, 1H), 4.46 (ABq, J=17 Hz, Δν=44 Hz, 2H), 4.53 (m, 1H), 6.75-6.88 (m, 3H), 7.04-7.24 (m, 5H), 7.34 (d, J=8 Hz, 1H), 7.68 (d, J=7 Hz, 1H), 8.04 (br s, 1H) | $C_{28}H_{36}N_4O_3$ | 70.56<br>70.68 | 7.61<br>7.70 | 11.58<br>11.58 |

| Example No. | R | Mp, °C | MS | $^1$H NMR | Formula | Analysis, % Theory/Found C | H | N |
|---|---|---|---|---|---|---|---|---|
| 61 | 1-hexamethyleneiminyl | foam | 490 (M$^+$) | $CDCl_3$ 1.52 (br s, 8H), 2.09 (s, 3H), 2.54 (br s, 4H), 2.87-3.10 (m, 4H), 3.21 (dd, J=5, 13 Hz, 1H), 3.76 (s, 3H), 3.92 (dd, J=10, 13 Hz, 1H), 4.48 (ABq, J=17 Hz, $\Delta\nu$=41 Hz, 2H), 4.53 (m, 1H), 6.73-6.89 (m, 3H), 7.04-7.25 (m, 4H), 7.34 (d, J=6 Hz, 1H), 7.58 (m, 1H), 7.66 (d, J=7 Hz, 1H), 8.04 (br s, 1H) | $C_{29}H_{38}N_4O_3$ | 70.99<br>71.27 | 7.81<br>7.98 | 11.42<br>11.39 |
| 62 | 4-morpholinyl | foam | 478 (M$^+$) | $CDCl_3$ 2.07 (s, 3H), 2.20-2.29 (m, 2H), 2.31-2.41 (m, 2H), 2.85-2.97 (m, 3H), 3.01-3.13 (m, 2H), 3.46-3.67 (m, 4H), 3.77 (s, 3H), 4.15 (dd, J=10, 13 Hz, 1H), 4.47 (ABq, J=17 Hz, $\Delta\nu$=48 Hz, 2H), 4.52 (m, 1H), 6.77-6.89 (m, 3H), 7.02-7.28 (m, 4H), 7.36 (d, J=6 Hz, 1H), 7.46 (d, J=8 Hz, 1H), 7.68 (d, J=7 Hz, 1H), 8.02 (br s, 1H) | $C_{27}H_{34}N_4O_4$ | 67.76<br>67.54 | 7.16<br>7.18 | 11.71<br>11.58 |

| Example No. | R | Mp, °C | MS | $^1$H NMR | Formula | Analysis, % Theory/Found C | H | N |
|---|---|---|---|---|---|---|---|---|
| 63 | 1-indolinyl | foam | 510 ($M^+$) | $CDCl_3$ 1.85 (s, 3H), 2.85-3.41 (m, 7H), 3.60 (ABq, J=17 Hz, Δv=42 Hz, 2H), 3.73 (s, 3H), 4.00 (dd, J=12, 13 Hz, 1H), 4.38 (ABq, J=17 Hz, Δv=48 Hz, 2H), 4.43-4.48 (m, 1H), 6.32 (d, J=8 Hz, 1H), 6.76 (m, 3H), 6.97-7.24 (m, 7H), 7.35 (d, J=8 Hz, 1H), 7.54 (d, J=8 Hz, 1H), 7.70 (d, J=8 Hz, 1H), 7.99 (br s, 1H) | $C_{31}H_{34}N_4O_3$ | 72.92<br>73.21 | 6.71<br>6.54 | 10.97<br>11.03 |
| 64 | 1,2,3,4-tetrahydroisoquinolin-4-yl | foam | 524 ($M^+$), 525 ($M+1^+$) | $CDCl_3$ 2.06 (s, 3H), 2.61-3.28 (m, 9H), 3.48-3.94 (m, 3H), 3.77 (s, 3H), 4.50 (ABq, J=17 Hz, Δv=36 Hz, 2H), 4.57 (m, 1H), 6.78-6.92 (m, 4H), 6.98-7.26 (m, 8H), 7.34 (d, J=9 Hz, 1H), 7.62 (d, J=8 Hz, 1H), 7.98 (br s, 1H) | $C_{32}H_{36}N_4O_3$ | 73.26<br>73.31 | 6.92<br>6.95 | 10.68<br>10.43 |

| Example No. | R | Mp, °C | MS | $^1$H NMR | Formula | Analysis, % Theory/Found C | H | N |
|---|---|---|---|---|---|---|---|---|
| 65 | 1-(4-Ph-piperidinyl) | foam | 552 (M$^+$) | $CDCl_3$ 1.50-1.91 (m, 4H), 2.08 (s, 3H), 2.06-2.22 (m, 2H), 2.40 (m, 1H), 2.64 (br d, J=11 Hz, 1H), 2.80 (br d, J=12 Hz, 1H), 2.86-2.98 (m, 3H), 3.04-3.18 (m, 2H), 3.73 (s, 3H), 4.01 (dd, J=10, 14 Hz, 1H), 4.46 (ABq, J=17 Hz, , Δv=45 Hz, 2H), 4.54 (m, 1H), 6.76-6.85 (m, 3H), 7.02-7.36 (m, 10H), 7.54 (d, J=8 Hz, 1H), 7.70 (d, J=8 Hz, 1H), 8.01 (br s, 1H) | $C_{34}H_{40}N_4O_3$ | 73.89<br>73.69 | 7.30<br>7.25 | 10.14<br>10.31 |
| 66 | 1-(4-$Me_2N$-piperidinyl) | foam | 519 (M$^+$) | $CDCl_3$ 1.26 (m, 1H), 1.48-1.76 (m, 3H), 1.90-2.11 (m, 3H), 2.09 (s, 3H), 2.25 (s, 6H), 2.51 (br d, J=13 Hz, 1H), 2.73 (br d, J=12 Hz, 1H), 2.85 (s, 2H), 2.85-3.23 (m, 3H), 3.75 (s, 3H), 3.94 (dd, J=10, 14 Hz, 1H), 4.47 (ABq, J=17 Hz, Δv=43 Hz, 2H), 4.51 (m, 1H), 6.77-6.88 (m, 3H), 7.01-7.28 (m, 4H), 7.35 (d, J=8 Hz, 1H), 7.41 (d, J=9 Hz, 1H), 7.66 (d, J=7 Hz, 1H), 8.09 (br s, 1H) | $C_{30}H_{41}N_5O_3$ | 69.34<br>69.58 | 7.95<br>8.01 | 13.48<br>13.52 |
| 67 | 1-(4-Ph-$\Delta^3$-piperidinyl) | foam | 550 (M$^+$) | $CDCl_3$ 2.12 (s, 3H), 2.21-2.70 (m, 4H), 2.90-3.25 (m, 7H), 3.77 (s, 3H), 3.95 (dd, J=10, 14 Hz, 1H), 4.52 (ABq, J=17 Hz, Δv=38 Hz, 2H), 4.61 (m, 1H), 5.95 (br s, 1H), 6.85 (m, 3H), 7.00-7.54 (m, 11H), 7.67 (d, J=8 Hz, 1H), 8.08 (br s, 1H) | $C_{34}H_{38}N_4O_3$ | 73.06<br>73.03 | 6.87<br>6.95 | 9.99<br>10.03 |
| 68 | 1-(4-AcNH-4-Ph-piperidinyl) | foam | 609 (M$^+$) | $^1$H $CDCl_3$ 1.87-2.50 (m, 7H), 2.00 (s, 3H), 2.07 (s, 3H), 2.60 (m, 1H), 2.87-3.19 (m, 5H), 3.73 (s, 3H), 4.06 (dd, J=10, 14 Hz, 1H), 4.46 (ABq, J=17 Hz, Δυ =47 Hz, 2H), 4.52 (m, 1H), 5.43 (br s, 1H), 6.75-6.90 (m, 3H), 7.04-7.48 (m, 10 H), 7.56 (d, J=8 Hz, 1H), 7.69 (d, J=8 Hz, 1H), 8.10 (br s, 1H). | $C_{36}H_{43}N_5O_4$ | 70.91<br>70.68 | 7.11<br>7.13 | 11.48<br>11.49 |

| Example No. | R | Mp, °C | MS | $^1$H NMR | Formula | Analysis, % Theory/Found C | H | N |
|---|---|---|---|---|---|---|---|---|
| 69 | 1-(4-(4-Cl-Ph)-piperazinyl) | foam | 587 ($M^+$) | $CDCl_3$ 2.11 (s, 3H), 2.20-2.42 (m, 2H), 2.42-2.58 (m, 2H), 2.82-3.20 (m, 9H), 3.76 (s, 3H), 4.01 (m, 1H), 4.50 (ABq, J=16 Hz, Δv=42 Hz, 2H), 4.54 (m, 1H), 6.68-6.90 (m, 5H), 7.04-7.32 (m, 6H), 7.35 (d, J=8 Hz, 1H), 7.40 (m, 1H), 7.66 (d, J=9 Hz, 1H), 8.03 (br s, 1H) | $C_{33}H_{38}N_5O_3Cl$ | 67.39<br>67.10 | 6.51<br>6.77 | 11.91<br>12.11 |
| 70 | 1-(4-(3-$CF_3$-Ph)-piperazinyl) | foam | 621 ($M^+$) | $CDCl_3$ 2.10 (s, 3H), 2.28-2.42 (m, 2H), 2.42-2.56 (m, 2H), 2.84-3.20 (m, 9H), 3.77 (s, 3H), 4.01 (m, 1H), 4.49 (ABq, J=18 Hz, Δv=42 Hz, 2H), 4.56 (m 1H), 6.76-6.90 (m, 3H), 6.90-7.27 (m, 7H), 7.28-7.46 (m, 3H), 7.66 (d, J=7 Hz, 1H), 8.06 (br s, 1H) | $C_{34}H_{38}N_5O_3F_3$ | 65.69<br>65.47 | 6.16<br>6.28 | 11.27<br>11.34 |

| Example No. | R | Mp, °C | MS | $^1H$ NMR | Formula | Analysis, % Theory/Found C | H | N |
|---|---|---|---|---|---|---|---|---|
| 71 | 1-(4-Me-piperazinyl) | foam | 492 (M+1+) | $CDCl_3$ 2.09 (s, 3H), 2.11-2.52 (m, 11H), 2.82-2.97 (m, 3H), 2.99-3.15 (m, 2H), 3.75 (s, 3H), 4.01 (dd, J=11, 14 Hz, 1H), 4.45 (ABq, J=16 Hz, Δν=46 Hz, 2H), 4.51 (m, 1H), 6.76-6.88 (m, 3H), 7.02-7.24 (m, 4H), 7.34 (d, J=8 Hz, 1H), 7.41 (d, J=8 Hz, 1H), 7.68 (d, J=8 Hz, 1H), 8.01 (br s, 1H) | $C_{28}H_{37}N_5O_3$ Exact Mass Data (M+1) Calc'd: 492.2975 Meas: 492.2977 | | | |

| Example No. | R | Mp, °C | MS | $^1$H NMR | Formula | Analysis, % Theory/Found C | H | N |
|---|---|---|---|---|---|---|---|---|
| 73 | 1-(4-i-Pr-piperazinyl) | foam | 519 ($M^+$) | $CDCl_3$ 1.07 (br d, J=6 Hz, 6H), 2.08 (s, 3H), 2.20-2.80 (m, 9H), 2.83-3.16 (m, 5H), 3.77 (s, 3H), 4.00 (dd, J=10, 14 Hz, 1H), 4.47 (ABq, J=8 Hz, Δv=42 Hz, 2H), 4.53 (m, 1H), 6.73-6.94 (m, 3H), 6.94-7.30 (m, 4H), 7.30-7.42 (m, 2H), 7.65 (d, J=10 Hz, 1H), 8.06 (br s, 1H) | $C_{30}H_{41}N_5O_3$ | 69.34<br>69.60 | 7.95<br>8.09 | 13.48<br>13.49 |
| 74 | 1-(4-cyclohexyl-piperazinyl) (RS) | foam | 559 ($M^+$) | $CDCl_3$ 1.05-1.34 (m, 6H), 1.55-1.95 (m, 4H), 2.09 (s, 3H), 2.20-2.60 (m, 9H), 2.90 (s, 2H), 2.85-3.16 (m, 3H), 3.77 (s, 3H), 4.02 (dd, J=11, 13 Hz, 1H), 4.47 (ABq, J=16 Hz, Δv=44 Hz, 2H), 4.54 (m, 1H), 6.77-6.88 (m, 3H), 7.05-7.25 (m, 4H), 7.31-7.42 (m, 2H), 7.66 (d, J=7 Hz, 1H), 8.08 (br s, 1H) | $C_{33}H_{45}N_5O_3$ | 70.81<br>71.10 | 8.10<br>8.28 | 12.51<br>12.53 |
| 75 | 1-(4-cyclohexyl-piperazinyl) (R) | foam | 560 ($M+1^+$) | $CDCl_3$ 1.09-1.28 (m, 5H), 1.64 (d, J=10 Hz, 1H), 1.80-1.89 (m, 4H), 2.10 (s, 3H), 2.24-2.52 (m, 9H), 2.90 (s, 2H), 2.95 (d, J=7 Hz, 1H), 3.02 (d, J=7 Hz, 1H), 3.12 (dd, J=5, 14 Hz, 1H), 3.77 (s, 3H), 4.01 (dd, J=10, 14 Hz, 1H), 4.49 (ABq, J=17 Hz, Δv=43 Hz, 2H), 4.56 (m, 1H), 6.79-6.87 (m, 3H), 7.05-7.24 (m, 4H), 7.34-7.41 (m, 2H), 7.67 (d, J=8 Hz, 1H), 8.22 (s, 1H) | $C_{33}H_{45}N_5O_3$ | 70.81<br>70.71 | 8.10<br>8.21 | 12.51<br>12.42 |
| 76 | 1-(4-cyclohexyl-piperazinyl) (S) | foam | 559 ($M^+$) | $^1$H $CDCl_3$ 1.05-1.31 (m, 5H), 1.64 (m, 1H), 1.75-1.90 (m, 4H), 2.10 (s, 3H), 2.24-2.52 (m, 9H), 2.87 (s, 2H), 2.95 (d, J=7 Hz, 1H), 3.01 (d, J=7 Hz, 1H), 3.12 (dd, J=5, 14 Hz, 1H), 3.77 (s, 3H), 3.99 (dd, J=10, 14 Hz, 1H), 4.46 (ABq, J=17 Hz, Δv=43 Hz, 2H), 4.56 (m, 1H), 6.75-6.90 (m, 3H), 7.05-7.24 (m, 4H), 7.34-7.41 (m, 2H), 7.67 (d, J=8 Hz, 1H), 8.14 (s, 1H) | $C_{33}H_{45}N_5O_3$ | 70.81<br>70.99 | 8.10<br>8.27 | 12.51<br>12.76 |

| Example No. | R | Mp, °C | MS | $^{1}$H NMR | Formula | Analysis, % Theory/Found C | H | N |
|---|---|---|---|---|---|---|---|---|
| 77 | 1-(4-PhCH$_2$-piperazinyl) | foam | 568 (M+1$^+$) | CDCl$_3$ 2.08 (s, 3H), 2.16-2.62 m, 8H), 2.82-2.97 (m, 3H), 2.99-3.18 (m, 2H), 3.41-3.62 (m, 2H), 3.76 (s, 3H), 4.02 (dd, J=10, 13 Hz, 1H), 4.49 (ABq, J=18 Hz, Δv=48 Hz, 2H), 4.53 (m, 1H), 6.76-6.88 (m, 3H), 7.06 (d, J=3 Hz, 1H), 7.06-7.45 (m, 10H), 7.68 (d, J=8 Hz, 1H), 8.06 (br s, 1H) | $C_{34}H_{41}N_5O_3$ | 71.93<br>72.15 | 7.28<br>7.37 | 12.34<br>12.56 |
| 78 | 1-(4-(2-pyrimidinyl)-piperazinyl) | foam | 555 (M$^+$) | CDCl$_3$ 2.11 (s, 3H), 2.28-2.55 (m, 4H), 2.88-3.12 (m, 5H), 3.56-3.86 (m, 4H), 3.77 (s, 3H), 4.02 (m, 1H), 4.47 (ABq, J=17 Hz, Δv=41 Hz, 2H), 4.52 (m, 1H), 6.50 (br s, 1H), 6.76-6.86 (m, 3H), 7.04-7.28 (m, 4H), 7.36 (d, J=7 Hz, 1H), 7.61 (br s, 1H), 7.67 (d, J=7 Hz, 1H), 8.10 (br s, 1H), 8.30 (d, J=5 Hz, 2H) | $C_{31}H_{37}N_7O_3$ | 67.01<br>66.90 | 6.71<br>6.85 | 17.64<br>17.43 |
| 79 | 1-(4-MeCO-piperazinyl) | foam | 519 (M$^+$), 520 (M+1$^+$) | CDCl$_3$ 2.04 (s, 3H), 2.09 (s, 3H), 2.16-2.48 (m, 4H), 2.86-3.11 (m, 4H), 3.21-3.65 (m, 5H), 3.78 (s, 3H), 4.04 (m, 1H), 4.46 (ABq, J=17 Hz, Δv=26 Hz, 2H), 4.50 (m, 1H), 6.76-6.86 (m, 3H), 7.02-7.28 (m, 4H), 7.36 (d, J=7 Hz, 1H), 7.50 (br s, 1H), 7.66 (d, J=7 Hz, 1H), 8.11 (br s, 1H) | $C_{29}H_{37}N_5O_4$ | 67.03<br>66.81 | 7.18<br>7.20 | 13.48<br>13.30 |

| Example No. | R | Mp, °C | MS | $^1$H NMR | Formula | Analysis, % Theory/Found C | H | N |
|---|---|---|---|---|---|---|---|---|
| 80 | 1-(4-EtO(CO)-piperazinyl) | foam | 549 ($M^+$) | $CDCl_3$ 1.23 (t, J=7 Hz, 3H), 2.08 (s, 3H), 2.12-2.40 (m, 4H), 2.85-2.97 (m, 3H), 2.98-3.12 (m, 2H), 3.22-3.49 (m, 4H), 3.75 (s, 3H), 4.03 (m, 1H), 4.11 (q, J=7 Hz, 2H), 4.44 (ABq, J=17 Hz, Δv=45 Hz, 2H), 4.48 (m, 1H), 6.76-6.86 (m, 3H), 7.04-7.25 (m, 4H), 7.34 (d, J=8 Hz, 1H), 7.46 (br s, 1H), 7.66 (d, J=8 Hz, 1H), 8.04 (br s, 1H) | $C_{30}H_{39}N_5O_5$ | 65.55<br>65.29 | 7.15<br>7.19 | 12.74<br>12.59 |
| 81 | (2-pyridyl)$CH_2NH$ | foam | 499 ($M^+$) | $CDCl_3$ 2.10 (s, 3H), 2.91 (m, 1H), 3.00-3.16 (m, 2H), 3.30 (s, 2H), 3.65-3.88 (m, 2H), 3.77 (s, 3H), 4.01 (dd, J=10, 16 Hz, 1H), 4.46 (ABq, J=17 Hz, Δv=53 Hz, 2H), 4.54 (m, 1H), 6.74-6.86 (m, 2H), 7.02-7.28 (m, 7H), 7.34 (d, J=8 Hz, 1H), 7.56-7.72 (m, 3H), 8.06 (br s, 1H), 8.55 (d, J=6 Hz, 1H) | $C_{29}H_{33}N_5O_3$ | 69.72<br>69.75 | 6.66<br>6.84 | 14.02<br>13.88 |
| 82 | (3-pyridyl)$CH_2NH$ | foam | 499 ($M^+$) | $CDCl_3$ 2.08 (s, 3H), 2.90 (dd, J=8, 15 Hz, 1H), 2.97-3.10 (m, 2H), 3.24 (s, 2H), 3.69 (ABq, J=14 Hz, Δv=25 Hz, 2H), 3.74 (s, 3H), 4.04 (dd, J=13, 16 Hz, 1H), 4.45 (ABq, J=18 Hz, Δv=53 Hz, 2H), 4.50 (m, 1H), 6.74-6.87 (m, 3H), 7.04 (d, J=4 Hz, 1H), 7.08-7.30 (m, 4H), 7.35 (d, J=8 Hz, 1H), 7.49 (d, J=8 Hz, 1H), 7.60-7.70 (m, 2H), 8.12 (br s, 1H), 8.48-8.52 (m, 2H) | $C_{29}H_{33}N_5O_3$ | 69.72<br>69.51 | 6.66<br>6.79 | 14.02<br>13.90 |

| Example No. | R | Mp, °C | MS | $^1$H NMR | Formula | Analysis, % Theory/Found | | |
|---|---|---|---|---|---|---|---|---|
| | | | | | | C | H | N |
| 83 | (4-pyridyl)$CH_2NH$ | foam | 499 ($M^+$) | $CDCl_3$ 2.09 (s, 3H), 2.84-3.10 (m, 3H), 3.20 (s, 2H), 3.65 (ABq, J=14 Hz, Δν=25 Hz, 2H), 3.72 (s, 3H), 4.08 (dd, J=12, 15 Hz, 1H), 4.40 (ABq, J=16 Hz, Δν=51 Hz, 2H), 4.48 (m, 1H), 6.73-6.84 (m, 3H), 7.00 (d, J=3 Hz, 1H), 7.08-7.25 (m, 5H), 7.32 (d, J=8 Hz, 1H), 7.45 (d, J=8 Hz, 1H), 7.67 (d, J=8 Hz, 1H), 8.01 (br s, 1H), 8.51 (d, J=7 Hz, 2H) | $C_{29}H_{33}N_5O_3$ | 69.72<br>69.99 | 6.66<br>6.77 | 14.02<br>13.79 |
| 84 | $PhNHCOCH_2NH$ | foam | 541 (M+) | $^1$H DMSO (3:2 mixture of amide rotamers) 1.95 (s, 3/5•3H), 2.20 (s, 2/5•3H), 2.75-2.93 (m, 2H), 3.07-3.17 (m, 2H), 3.17-3.30 (m, 3H), 3.39 (m, 1H), 3.53 (m, 1H), 3.67 (s, 2/5•3H), 3.72 (s, 3/5•3H), 4.25-4.61 (m, 3H), 6.77-6.87 (m, 2H), 6.87-7.09 (m, 4H), 7.12 (m, 1H), 7.14-7.36 (m, 4H), 7.55 (d, J=8 Hz, 1H), 7.63 (t, J=8 Hz, 2H), 7.91 (d, J=9 Hz, 3/5•1H), 8.05 (d, J=9 Hz, 2/5•1H), 9.92 (br s, 0.4H), 9.94 (br s, 0.6 H), 10.78 (br s, 0.6H), 10.80 (br s, 0.4H). | $C_{31}H_{35}N_5O_4$ | 68.74<br>68.51 | 6.51<br>6.56 | 12.93<br>12.78 |

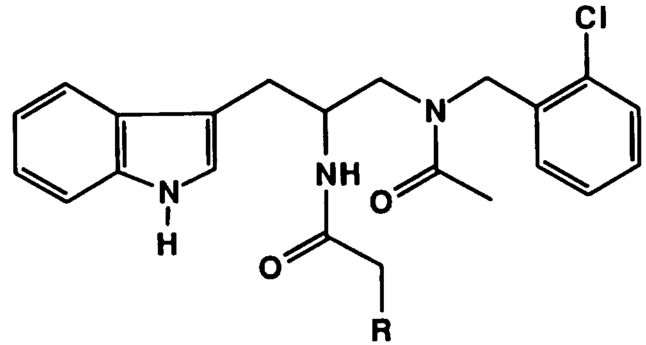

| Example No. | R | Mp, °C | MS | $^1$H NMR | Formula | Analysis, % Theory/Found C | H | N |
|---|---|---|---|---|---|---|---|---|
| 86 | 1-(4-i-Pr-piperazinyl) (R) | foam | 523 (M$^+$) | $^1$H $CDCl_3$ 0.9-1.1 (m, 6H), 2.05 (s, 3H), 2.1-2.5 (m, 11H), 2.8-3.1 (m, 3H), 3.2 (m, 1H), 4.0 (m, 1H), 4.5-4.7 (m, 2H), 6.9-7.4 (m, 9H), 7.63 (d, J=6 Hz, 1H), 8.23 (br s, 1H). | $C_{29}H_{38}ClN_5O_2$ | 66.46<br>66.72 | 7.31<br>7.33 | 13.36<br>13.30 |
| 87 | 1-(4-cyclohexyl-piperazinyl) (R) | foam | 563 (M$^+$) | $^1$H $CDCl_3$ 1.0-1.4 (m, 6H), 1.6 (m, 1H), 1.7-1.9 (m, 4H), 2.08 (s, 3H), 2.1-2.6 (m, 9H), 2.8-3.1 (m, 4H), 4.0 (m, 1H), 4.5-4.7 (m, 3H), 7.0-7.4 (m, 9H), 7.63 (d, J=6 Hz, 1H), 8.18 (br s, 1H). | $C_{32}H_{42}ClN_5O_2$ | 68.13<br>67.93 | 7.50<br>7.53 | 12.41<br>12.43 |

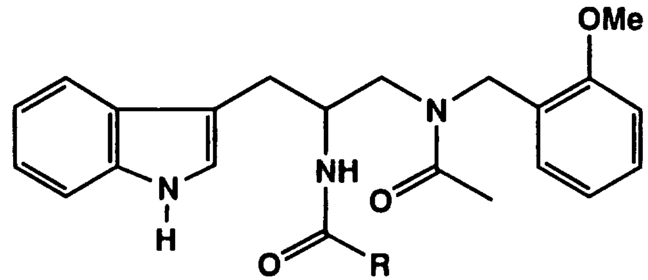

| Example No. | R | Mp, °C | MS | $^1$H NMR | Formula | Analysis, % Theory/Found | | |
|---|---|---|---|---|---|---|---|---|
| | | | | | | C | H | N |
| 88 | Ph | foam | 455 ($M^+$) | $CDCl_3$ 2.10 (s, 3H), 2.81-2.94 (m, 2H), 3.32 (dd, J=5, 15 Hz, 1H), 3.66 (s, 3H), 4.21 (dd, J=13, 15 Hz, 1H), 4.36 (ABq, J=15 Hz, Δν=43 Hz, 2H), 4.46 (m, 1H), 6.61-6.80 (m, 3H), 7.00 (d, J=5 Hz, 1H), 7.10-7.50 (m, 7H), 7.70 (d, J=8 Hz, 1H), 7.80 (d, J=6 Hz, 1H), 7.87 (d, J=6 Hz, 2H), 7.96 (br s, 1H) | $C_{28}H_{29}N_3O_3$ | 73.82<br>73.86 | 6.42<br>6.44 | 9.22<br>9.36 |
| 89 | $Ph(CH_2)_2$ (RS) | foam | 483 ($M^+$) | $CDCl_3$ 2.05 (s, 3H), 2.45 (t, J=9 Hz, 2H), 2.72-3.12 (m, 5H), 3.71 (s, 3H), 4.01 (dd, J=12, 14 Hz, 1H), 4.33 (ABq, J=16 Hz, Δν=60 Hz, 2H), 4.38 (m, 1H), 6.58 (d, J=9 Hz, 1H), 6.66-6.81 (m, 3H), 6.88 (d, J=3 Hz, 1H), 7.09-7.38 (m, 9H), 7.68 (d, J=7 Hz, 1H), 7.98 (br s, 1H) | $C_{30}H_{33}N_3O_3$ | 74.51<br>74.81 | 6.88<br>7.06 | 8.69<br>8.39 |

| Example No. | R | Mp, °C | MS | $^1H$ NMR | Formula | Analysis, % Theory/Found C | H | N |
|---|---|---|---|---|---|---|---|---|
| 90 | $Ph(CH_2)_2$ (R) | foam | 283 ($M^+$) | $^1H$ $CDCl_3$ 2.05 (s, 3H), 2.46 (t, J=8 Hz, 2H), 2.70-2.90 (m, 2H), 2.96 (t, J=8 Hz, 2H), 3.10 (m, 1H), 3.71 (s, 3H), 4.03 (m, 1H), 4.24 (d, J=17 Hz, 1H), 4.33-4.50 (m, 2H), 6.60-6.86 (m, 4H), 6.89 (s, 1H), 7.05-7.40 (m, 9H), 7.69 (d, J=8 Hz, 1H), 8.03 (s, 1H) | $C_{30}H_{33}N_3O_3$ | 74.51<br>74.30 | 6.88<br>6.66 | 8.69<br>8.46 |
| 91 | $Ph(CH_2)_2$ (S) | foam | 483 ($M^+$) | $^1H$ $CDCl_3$ 2.04 (s, 3H), 2.45 (t, J=8 Hz, 2H), 2.73-2.89 (m, 2H), 2.96 (t, J=8 Hz, 2H), 3.06 (dd, J=4, 10Hz, 1H), 3.71 (s, 3H), 4.03 (m, 1H), 4.20-4.50 (m, 3H), 6.58-6.88 (m, 4H), 6.89 (s, 1H), 7.07-7.40 (m, 9H), 7.69 (d, J=8 Hz, 1H), 8.03 (s, 1H) | $C_{30}H_{33}N_3O_3$ | 74.51<br>74.60 | 6.88<br>6.96 | 8.69<br>8.70 |
| 92 | $PhCH_2O$ (R) | foam | 485 ($M^+$) | $^1H$ $CDCl_3$ 2.09 (s, 3H), 2.83 (dd, J=7, 15 Hz, 1H), 2.95 (dd, J=3, 14 Hz, 1H), 3.10 (dd, J=3, 14 Hz, 1H), 3.70 (s, 3H), 3.96 (m, 1H), 4.22 (m, 1H), 4.26 (m, 1H), 4.72 (s, 1H), 5.12 (s, 2H), 5.68 (m, 1H), 6.68-6.83 (m, 2H), 6.97 (m, 1H), 7.07-7.46 (m, 10H), 7.66 (d, J=8 Hz, 1H), 8.02 (s, 1H) | $C_{29}H_{31}N_3O_4$ | 71.73<br>71.61 | 6.43<br>6.21 | 8.65<br>8.67 |

| Example No. | R | Mp, °C | MS | $^1$H NMR | Formula | Analysis, % Theory/Found C | H | N |
|---|---|---|---|---|---|---|---|---|
| 93 | $PhCH_2O$ (S) | oil | 485 (M+) | $^1$H $CDCl_3$ 1.70-2.10 (m, 3H), 2.75-3.00 (m, 2H), 3.10 (m, 1H), 3.70 (s, 3H), 3.95 (m, 1H), 4.10 (m, 1H), 4.45 (m, 1H), 4.61 (s, 1H), 5.13 (s, 2H), 5.73 (m, 1H), 6.66-6.85 (m, 2H), 6.95 (m, 1H), 7.03-7.50 (m, 10H), 7.66 (d, J=8 Hz, 1H), 8.02 (br s, 1H). | $C_{29}H_{31}N_3O_4$ | 71.73<br>71.90 | 6.43<br>6.60 | 8.65<br>8.51 |
| 94 | $Ph(CH_2)_3$ | foam | 497 ($M^+$) | $CDCl_3$ 1.88-2.00 (m, 2H), 2.09 (s, 3H), 2.13-2.23 (m, 2H), 2.61 (t, J=8 Hz, 2H), 2.78-2.92 (m, 2H), 3.12 (dd, J=4, 9 Hz, 1H), 3.69 (s, 3H), 4.10 (dd, J=7, 9 Hz, 1H), 4.40 (ABq, J=17 Hz, , Δν=56 Hz, 2H), 4.40 (m, 1H), 6.61 (br s, 1H), 6.67-6.81 (m, 3H), 6.99 (s, 1H), 7.04-7.36 (m, 9H), 7.70 (d, J=8 Hz, 1H), 7.98 (br s, 1H) | $C_{31}H_{35}N_3O_3$ | 74.82<br>74.58 | 7.09<br>7.13 | 8.44<br>8.32 |
| 95 | $PhCO(CH_2)_2$ (RS) | foam | 511 ($M^+$) | $CDCl_3$ 2.17 (s, 3H), 2.57 (t, J=7 Hz, 2H), 2.79-2.89 (m, 2H), 3.11 (dd, J=6, 14 Hz, 1H), 3.21-3.45 (m, 2H), 3.68 (s, 3H), 4.09 (dd, J=12, 14 Hz, 1H), 4.38 (ABq, J=16 Hz, Δν=75 Hz, 2H), 4.40 (m, 1H), 6.71-6.79 (m, 4H), 7.01 (d, J=3 Hz, 1H), 7.09-7.22 (m, 3H), 7.34 (d, J=7 Hz, 1H), 7.46 (t, J=8 Hz, 2H), 7.56 (m, 1H), 7.70 (d, J=8 Hz, 1H), 8.00 (d, J=8 Hz, 3H) | $C_{31}H_{33}N_3O_4$ | 72.78<br>72.71 | 6.50<br>6.38 | 8.21<br>7.95 |

| Example No. | R | Mp, °C | MS | $^1$H NMR | Formula | Analysis, % Theory/Found C | H | N |
|---|---|---|---|---|---|---|---|---|
| 96 | $PhCO(CH_2)_2$ (R) | oil | 511 ($M^+$) | $^1$H $CDCl_3$ 2.19 (s, 3H), 2.58 (t, J=4 Hz, 1H), 2.80-2.93 (m, 2H), 3.05 (m, 1H), 3.20-3.46 (m, 3H), 3.70 (s, 3H), 4.05 (m, 1H), 4.26 (m, 1H), 4.33-4.60 (m, 2H), 6.66-6.86 (m, 4H), 7.00 (s, 1H), 7.06-7.23 (m, 3H), 7.30 (d, J=8 Hz, 1H), 7.43-7.53 (m, 2H), 7.58 (d, J=8 Hz, 1H), 7.70 (d, J=8 Hz, 1H), 7.97 (d, J=8 Hz, 2H), 8.12 (s, 1H). | $C_{31}H_{33}N_3O_4$ | 72.78<br>72.84 | 6.50<br>6.61 | 8.21<br>8.22 |
| 97 | $PhCO(CH_2)_2$ (S) | oil | 511 ($M^+$) | $^1$H DMSO (4:3 mixture of amide rotamers) 1.70 (s, 4/7 · 1H), 1.77 (s, 3/7 · 1H), 1.92 (s, 4/7 · 3H), 2.00 (s, 3/7 · 3H), 2.40 (m, 1H), 2.60-2.80 (m, 2H), 3.10-3.25 (m, 3H), 3.50 (m, 1H), 3.65 (s, 3/7 · 3H), 3.72 (s, 4/7 · 3H), 4.25-4.60 (m, 3H), 6.75-7.35 (m, 8H), 7.45-7.70 (m, 4H), 7.74 (d, J=8 Hz, 1H), 7.80-8.00 (m, 2H), 10.77 (m, 1H). | $C_{31}H_{33}N_3O_4$ | 72.78<br>72.86 | 6.50<br>6.50 | 8.21<br>8.17 |
| 98 | $PhCO(CH_2)_3$ | foam | 525 ($M^+$) | $CDCl_3$ 2.00-2.11 (m, 2H), 2.11 (s, 3H), 2.25 (t, J=7 Hz, 2H), 2.76-2.91 (m, 2H), 2.98-3.16 (m, 3H), 3.71 (s, 3H), 4.04 (dd, J=11, 13 Hz, 1H), 4.38 (ABq, J=17 Hz, Δν=54 Hz, 2H), 4.39 (m, 1H), 6.60-6.81 (m, 4H), 6.98 (s, 1H), 7.08-7.24 (m, 3H), 7.34 (d, J=9 Hz, 1H), 7.45 (t, J=9 Hz, 2H), 7.55 (m, 1H), 7.70 (d, J=9 Hz, 1H), 7.96 (d, J=8 Hz, 2H), 8.01 (br s, 1H) | $C_{32}H_{35}N_3O_4$ | 73.12<br>72.86 | 6.71<br>6.66 | 7.99<br>7.73 |

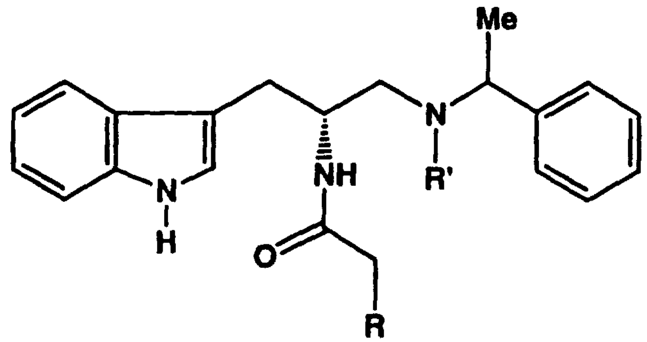

| Example No. | R | R' | Mp °C | MS | $^1$H NMR | Formula | Analysis, % Theory/Found C | H | N |
|---|---|---|---|---|---|---|---|---|---|
| 99 | H (RS) | MeCO | foam | 377 ($M^+$) | $CDCl_3$ 1.42 (d, J=8 Hz, 3H), 1.92 (s, 3H), 2.23 (s, 3H), 2.53 (dd, J=8, 14 Hz, 1H), 2.85-3.05 (m, 2H), 3.28 (m, 1H), 3.81 (dd, J=10, 14 Hz, 1H), 4.94 (q, J=8 Hz, 1H), 6.82 (m, 1H), 6.82-7.27 (m, 7H), 7.27-7.45 (m, 2H), 7.54 (d, J=8 Hz, 1H), 8.01 (br s, 1H) | $C_{23}H_{27}N_3O_2$ | 73.18<br>73.35 | 7.21<br>7.46 | 11.13<br>10.90 |
| 100 | H (RR) | MeCO | foam | 377 ($M^+$) | $CDCl_3$ 1.38 (d, J=8 Hz, 3H), 1.93 (s, 3H), 2.17 (s, 3H), 2.68 (dd, J=8, 14 Hz, 1H), 2.74 (dd, J=4, 14 Hz, 1H), 3.20 (dd, J=4, 14 Hz, 1H), 3.91 (dd, J=10, 14 Hz, 1H), 4.37 (m, 1H), 4.92 (m, 1H), 6.78-7.27 (m Hz, 9H), 7.37 (d, J=8 Hz, 1H), 7.75 (d, J=8 Hz, 1H), 7.98 (br s, 1H) | $C_{23}H_{27}N_3O_2$ | 73.18<br>73.39 | 7.21<br>7.33 | 11.13<br>10.96 |

| Example No. | R | R' | Mp °C | MS | $^1$H NMR | Formula | Analysis, % Theory/Found C | H | N |
|---|---|---|---|---|---|---|---|---|---|
| 101 | 1-(4-(1-piperidinyl)-piperidinyl) (RS) | H | foam | 501 ($M^+$) | $CDCl_3$ 1.32 (d, J=7 Hz, 3H), 1.15-1.91 (m, 11H), 1.91-2.23 (m, 3H), 2.30-2.60 (m, 6H), 2.65 (dd, J=6, 14 Hz, 1H), 2.72-2.94 (m, 4H), 3.01 (dd, J=6, 14 Hz, 1H), 3.72 (q, J=7 Hz, 1H), 4.35 (m, 1H), 6.95 (d, J=2 Hz, 1H), 7.03-7.42 (m, 9H), 7.64 (d, J=8 Hz, 1H), 8.08 (br s, 1H) | $C_{31}H_{43}N_5O$ | 74.21<br>74.50 | 8.64<br>8.49 | 13.96<br>13.94 |
| 102 | 1-(4-(1-piperidinyl)-piperidinyl) (RR) | H | foam | 501 ($M^+$) | $DMSOd_6$ 1.23 (d, J=6 Hz, 3H), 1.12-1.70 (m, 11H), 1.89-2.01 (m, 2H), 2.01-2.17 (m, 2H), 2.23-2.43 (m, 5H), 2.52 (m, 1H), 2.72 (m, 1H), 2.75 (ABq, J=15 Hz, Δv=30 Hz, 2H), 2.83 (dd, J=8, 14 Hz, 1H), 2.95 (dd, J=6, 14 Hz, 1H), 3.66 (q, J=6 Hz, 1H), 4.06 (m, 1H), 6.95 (t, J=8 Hz, 1H), 6.99-7.10 (m, 2H), 7.10-7.41 (m, 6H), 7.49 (d, J=9 Hz, 1H), 7.56 (d, J=8 Hz, 1H), 10.78 (br s, 1H) | $C_{31}H_{43}N_5O$ | 74.21<br>73.93 | 8.64<br>8.65 | 13.96<br>13.89 |
| 103 | 1-(4-(1-piperidinyl)-piperidinyl) (RS) | MeCO | foam | 543 ($M^+$) | $CDCl_3$ 1.29-1.88 (m, 12H), 1.88-2.08 (m, 2H), 2.15 (s, 3H), 2.21 (m, 1H), 2.36-2.62 (m, 6H), 2.62-2.88 (m, 4H), 2.96 (dd, J=6, 14 Hz, 1H), 3.28 (dd, J=6, 14 Hz, 1H), 3.65 (dd, J=10, 14 Hz, 1H), 3.82 (m, 1H), 4.98 (m, 1H), 6.85-7.45 (m, 9H), 7.48-7.59 (m, 2H), 8.10 (br s, 1H) | $C_{33}H_{45}N_5O_2$ | 72.89<br>73.13 | 8.34<br>8.27 | 12.88<br>12.91 |

| Example No. | R | R' | Mp °C | MS | $^1$H NMR | Formula | Analysis, % Theory/Found C | H | N |
|---|---|---|---|---|---|---|---|---|---|
| 104 | 1-(4-(1-piperidinyl)-piperidinyl) (RR) | MeCO | foam | 543 ($M^+$) | DMSO-$d_6$ 2:1 mixture of amide rotamers 1.19-1.84 (m, 12H), 1.84-2.16 (m, 3H), 2.06 (s, 3H), 2.32-2.52 (m, 5H), 2.57-3.00 (m, 6H), 3.20 (m, 1H), 3.79 (dd, J=11, 14 Hz, 1H), 4.28 (m, 1H), 5.04 (m, 2/3•1H), 5.49 (m, 1/3•1H), 6.89-7.15 (m, 5H), 7.15-7.28 (m, 3H), 7.32 (d, J=8 Hz, 1H), 7.47 (m, 1H), 8.41 (m, 1H), 10.77 (br s, 1H) | $C_{33}H_{45}N_5O_2$ | 72.89<br>72.65 | 8.34<br>8.14 | 12.88<br>12.71 |

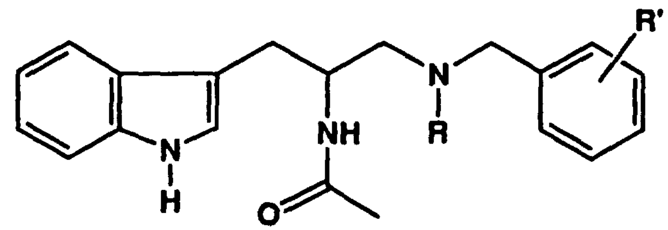

| Example No. | R | R' | Mp °C | MS | $^1$H NMR | Formula | Analysis, Theory/Found C | H | N |
|---|---|---|---|---|---|---|---|---|---|
| 105 | H | 2-OMe | foam | 351 ($M^+$) | $CDCl_3$ 1.97 (s, 3H), 2.38 (m, 1H), 2.73 (dd, J=6, 12 Hz, 1H), 2.82 (dd, J=6, 12 Hz, 1H), 2.97 (dd, J=8, 14 Hz, 1H), 3.10 (dd, J=6, 14 Hz, 1H), 3.75-3.94 (m, 2H), 3.82 (s, 3H), 4.42 (m, 1H), 6.34 (br d, J=8 Hz, 1H), 6.77-6.95 (m, 2H), 7.01 (d, J=2 Hz, 1H), 7.07-7.33 (m, 4H), 7.37 (d, J=8 Hz, 1H), 7.68 (d, J=8 Hz, 1H), 8.13 (br s, 1H) | $C_{21}H_{25}N_3O_2$ | 71.77<br>71.48 | 7.17<br>6.90 | 11.96<br>12.09 |

| Example No. | R | R' | Mp °C | MS | $^1$H NMR | Formula | Analysis, Theory/Found C | H | N |
|---|---|---|---|---|---|---|---|---|---|
| 106 | MeCO | 2-OMe | 147-148 | 393 ($M^+$) | $CDCl_3$/DMSO$d_6$ 1.95 (s, 3H), 2.13 (s, 3H), 2.81 (dd, J=8, 16 Hz, 1H), 2.89 (dd, J=4, 14 Hz, 1H), 3.72 (s, 3H), 3.99 (t, J=10 Hz, 1H), 4.35 (m, 1H), 4.37 (ABq, J=16 Hz, Δν=58 Hz, 2H), 7.65-7.82 (m, 4H), 6.99 (s, 1H), 7.01-7.22 (m, 3H), 7.37 (d, J=7 Hz, 1H), 7.66 (d, J=8 Hz, 1H), 9.19 (br s, 1H) | $C_{23}H_{27}N_3O_3$ | 70.21<br>69.93 | 6.92<br>7.06 | 10.68<br>10.58 |
| 107 | 1-(4-Ph-piperazinyl) $CH_2CO$ | 2-OMe | foam | 553 ($M^+$) | $CDCl_3$ 1.93 (s, 3H), 2.72-2.98 (m, 6H), 3.08 (dd, J=6, 15 Hz, 1H), 3.18-3.52 (m, 6H), 3.73 (s, 3H), 4.02 (t, J=13 Hz, 1H), 4.33 (d, J=16 Hz, 1H), 4.42 (m, 1H), 4.64 (d, J=16 Hz, 1H), 6.45 (d, J=8 Hz, 1H), 6.66-6.95 (m, 6H), 7.00 (d, J=3 Hz, 1H), 7.04-7.30 (m, 5H), 7.36 (d, J=9 Hz, 1H), 7.67 (d, J=8 Hz, 1H), 8.07 (br s, 1H) | $C_{33}H_{39}N_5O_3$ | 71.58<br>71.33 | 7.10<br>7.09 | 12.65<br>12.51 |

| Example No. | R | R' | Mp •C | MS | $^1$H NMR | Formula | Analysis, Theory/Found C | H | N |
|---|---|---|---|---|---|---|---|---|---|
| 108 | 1-(4-(1-piperidinyl)-piperidinyl) $CH_2CO$ | H | foam | 530 (M+1) | $CDCl_3$ 2:1 mixture of amide rotamers 1.24-1.89 (m, 10H), 1.90 (s, 2/3•3H), 1.96 (s, 1/3•3H), 1.92-2.10 (m, 2H), 2.23 (m, 1H), 2.34 (m, 1H), 2.42-2.53 (m, 2H), 2.62-2.94 (m, 5H), 3.01-3.23 (m, 3H), 3.57 (dd, J=12, 14 Hz, 1/3•1H), 4.06 (dd, J=12, 15 Hz, 2/3•1H), 4.43 (br s, 2/3•1H), 4.57 (ABq, J=16 Hz, Δv=169 Hz, 2/3•2H), 4.58 (ABq, J=16 Hz, Δv=273 Hz, 1/3•2H), 4.63 (br s, 1/3•1H), 6.38 (d, J=8 Hz, 2/3•1H), 6.73 (d, J=8 Hz, 1/3•1H), 6.84-6.98 (m, 2H), 7.05-7.30 (m, 6H), 7.34 (d, J=7 Hz, 1H), 7.53 (d, J=8 Hz, 1/3•1H), 7.66 (d, J=8 Hz, 2/3•1H), 7.99 (br s, 2/3•1H), 8.13 (br s, 1/3•1H) | $C_{32}H_{43}N_5O_2$ | 72.56<br>72.29 | 8.18<br>8.04 | 13.22<br>13.21 |
| 109 | 1-(4-(1-piperidinyl)-piperidinyl) $CH_2CO$ | 2-Cl | foam | 563 ($M^+$) | $CDCl_3$ 3:1 mixture of amide rotamers 1.38-1.86 (m, 11H), 1.93 (s, 3/4•3H), 1.98 (s, 1/4•3H), 1.86-2.12 (m, 2H), 2.18-2.73 (m, 5H), 2.77-2.98 (m, 3H), 2.99-3.19 (m, 3H), 3.57 (dd, J=12, 14 Hz, 1/4•1H), 4.10 (dd, J=12, 14 Hz, 3/4•1H), 4.41 (m, 3/4•1H), 4.65 (m, 1/4•1H), 4.66 (ABq, J=18 Hz, Δv=107 Hz, 3/4•2H), 4.72 (ABq, J=15 Hz, Δv=157 Hz, 1/4•2H), 6.40 (br d, J=7 Hz, 1H), 6.90 (d, J=7 Hz, 1H), 7.02 (br s, 1H), 7.06-7.40 (m, 6H), 7.55 (d, J=8 Hz, 1/4•1H), 7.64 (d, J=8 Hz, 3/4•1H), 8.04 (br s, 1H) | $C_{32}H_{42}ClN_5O_2$ | 68.13<br>66.92 | 7.50<br>7.48 | 12.41<br>12.32 |

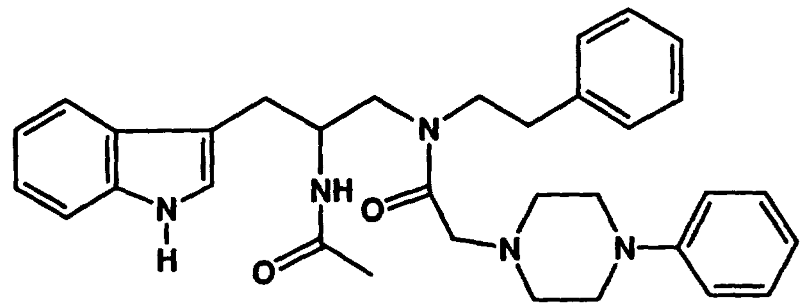

| Example No. | Mp °C | MS | $^1$H NMR | Formula | Analysis, Theory/Found C | H | N |
|---|---|---|---|---|---|---|---|
| 110 | foam | 537 (M+) | $^1$H DMSO (3:2 mixture of amide rotomers) 1.79 (s, 3/5•3H), 1.81 (s, 2/5•3H), 2.25-2.46 (m, 4H), 2.59-3.21 (m, 10H), 3.23-3.67 (m, 4H), 4.46 (m, 1H), 6.76 (t, J=8 Hz, 1H), 6.91 (d, J=8 Hz, 2H), 6.94-7.40 (m, 11H), 7.60 (m, 1H), 7.81-8.05 (m, 1H), 10.81 (br s, 2/5•1H), 10.84 (br s, 3/5•1H). | $C_{33}H_{39}N_5O_2$ | 73.71<br>73.64 | 7.31<br>7.33 | 13.02<br>13.08 |

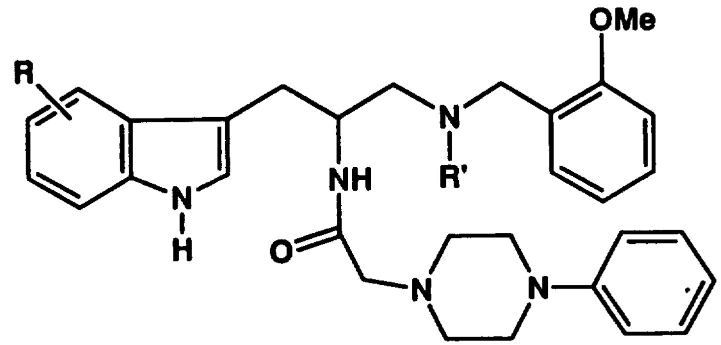

| Example No. | R | R' | Mp °C | MS | $^1$H NMR | Formula | Analysis, Theory/Found C | H | N |
|---|---|---|---|---|---|---|---|---|---|
| 111 | 5-Br | H | oil | 590, 592 (M+1) for Br isotopes) | $CDCl_3$ 2.33-2.45 (m, 2H), 2.45-2.53 (m, 2H), 2.80-3.10 (m, 11H), 3.75 (s, 1H), 3.88 (s, 3H), 3.94 (d, J=4 Hz, 2H), 6.80-6.96 (m, 6H), 7.10 (s, 1H), 7.20-7.36 (m, 5H), 7.40 (m, 1H), 7.75 (s, 1H), 8.20 (s, 1H) | $C_{31}H_{36}N_5O_2Br$ | 63.05<br>63.21 | 6.14<br>6.21 | 11.86<br>11.59 |
| 112 | 5-$OCH_2Ph$ | H | oil | 617 ($M^+$) | DMSO-d6 2.30-2.65 (m, 8H), 2.80-3.15 (m, 8H), 3.31 (s, 1H), 3.64 (s, 2H), 3.72 (s, 3H), 4.15 (m, 1H), 6.65-6.95 (m, 6H), 7.05 (s, 1H), 7.10-7.25 (m, 5H), 7.25-7.40 (m, 4H), 7.43 (d, J=9 Hz, 2H), 7.50 (d, J=9 Hz, 1H), 10.70 (s, 1H) | $C_{38}H_{43}N_5O_3$ | 73.88<br>74.09 | 7.02<br>7.03 | 11.34<br>11.31 |
| 113 | 1-Me | MeCO | oil | 567 ($M^+$) | $CDCl_3$ 2.11 (s, 3H), 2.36-2.60 (m, 3H), 2.85-3.20 (m, 10H), 3.71 (s, 3H), 3.77 (s, 3H), 3.97 (br s, 1H), 4.36-4.60 (m, 3H), 6.78-7.00 (m, 7H), 7.10 (s, 1H), 7.20-7.35 (m, 6H), 7.66 (d, J=8 Hz, 1H) | $C_{34}H_{41}N_5O_3$ | 71.93<br>71.69 | 7.28<br>7.36 | 12.34<br>12.28 |

| Example No. | R | R' | Mp °C | MS | $^1$H NMR | Formula | Analysis, Theory/Found C | H | N |
|---|---|---|---|---|---|---|---|---|---|
| 114 | 6-Me | MeCO | oil | FD-MS 567 ($M^+$) | $^1$H $CDCl_3$ 2.10(s, 3H), 2.10 (m, 1H), 2.40-2.70 (m, 7H), 2.90-3.10 (m, 7H), 3.16 (dd, J=4, 13 Hz, 1H), 3.78 (s, 3H), 3.97 (m, 1H), 4.40-4.70 (m, 3H), 6.80-7.10 (m, 8H), 7.16 (s, 1H), 7.20-7.40 (m, 3H), 7.45 (m,1H), 7.54 (d, J=8 Hz, 1H), 7.94 (m, 1H). | $C_{34}H_{41}N_5O_3$ | 71.93<br>71.72 | 7.28<br>6.99 | 12.34<br>12.10 |
| 115 | 7-Me | MeCO | foam | 567 ($M^+$) | $^1$H $CDCl_3$ 2.08 (s, 3H), 2.35-2.53 (m, 7H), 2.88-3.15 (m, 10H), 3.76 (s, 3H), 4.48 (ABq, J=17.1 Hz, Δv=41.2 Hz, 2H), 4.55 (m, 1H), 6.78-6.90 (m, 6H), 6.96-7.08 (m, 3H), 7.22 (m, 3H), 7.40 (m, 1H), 7.50 (d, J=8.0 Hz, 1H), 7.95 (s, 1H). | $C_{34}H_{41}N_5O_3$ | 71.93<br>71.82 | 7.28<br>7.31 | 12.34<br>12.32 |
| 116 | 5-Br | MeCO | 124-126 | 631, 633 ($M^+$'s for Br isotopes) | $CDCl_3$ 2.12 (s, 3H), 2.40-2.66 (m, 4H), 2.83-3.20 (m, 9H), 3.80 (s, 3H), 3.96 (m, 1H), 4.43-4.60 (m, 3H), 6.83-6.96 (m, 6H), 7.10 (s, 1H), 7.20-7.33 (m, 5H), 7.46 (br s, 1H), 7.75 (s, 1H), 8.44 (s, 1H) | $C_{33}H_{38}N_5O_3Br$ | 62.66<br>62.92 | 6.05<br>6.04 | 11.07<br>11.25 |
| 117 | 5-OMe | MeCO | oil | 583 ($M^+$) Exact Mass FAB (M+1) theory: 584.3237 found: 584.3214 | DMSO-d6 1:1 mixture of amide rotamers 1.86 (s, 1/2•3H), 1.94 (s, 1/2•3H), 2.23-2.43 (m, 4H), 2.73-2.93 (m, 4H), 2.93-3.10 (m, 4H), 3.16 (m, 1H), 3.56 (m, 1H), 3.66 (s, 1/2•3H), 3.69 (s, 1/2•3H), 3.71 (s, 1/2•3H), 3.72 (s, 1/2•3H), 4.23-4.60 (m, 3H), 6.66-7.00 (m, 7H), 7.08 (s, 2H), 7.15-7.26 (m, 4H), 7.59 (d, J=8 Hz, 1/2•1H), 7.77 (d, J = 8 Hz, 1/2•1H), 10.65 (s, 1H) | $C_{34}H_{41}N_5O_4$ | | | |
| 118 | 5-$OCH_2Ph$ | MeCO | oil | 660 ($M+1^+$) | DMSO-d6 3:2 mixture of amide rotamers 1.94 (s, 3/5•3H), 2.04 (s, 2/5•3H), 2.23-2.56 (m, 5H), 2.66-2.93 (m, 4H), 2.93-3.13 (m, 3H), 3.30-3.50 (m, 3H), 3.58 (m, 1H), 3.68 (s, 2/5•3H), 3.70 (s, 3/5•3H), 4.24-4.60 (m, 3H), 6.70-7.00 (m, 7H), 7.06 (s, 1H), 7.13-7.50 (m, 10H), 7.55 (d, J=8 Hz, 3/5•1H), 7.66 (d, J=8 Hz, 2/5•1H), 10.70 (s, 1H) | $C_{40}H_{45}N_5O_4$ | 72.81<br>72.58 | 6.87<br>6.85 | 10.61<br>10.37 |

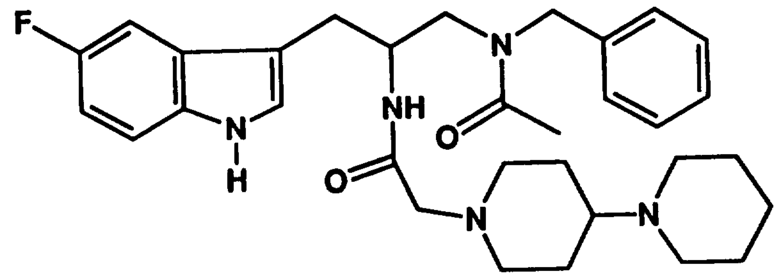

| Example No. | Mp °C | MS | $^1$H NMR | Formula | Analysis, % Theory/Found C | H | N |
|---|---|---|---|---|---|---|---|
| 119 | foam | 548 (M+) | $^1$H $CDCl_3$ 1.30-1.72 (m, 10H), 1.96-2.24 (m, 6H), 2.41-2.56 (m, 5H), 2.70-2.77 (m, 1H), 2.85 (s, 2H), 2.87-3.00 (m, 2H), 3.16 (dd, J=4.7, 13.8 Hz, 1H), 4.00 (dd, J=10.1, 13.8 Hz, 1H), 4.48-4.57 (m, 1H), 4.55 (ABq, J=17.0 Hz, Δν=47.7 Hz, 2H), 6.93 (m, 1H), 7.08-7.16 (m, 3H), 7.21-7.41 (m, 6H), 8.27 (s, 1H). | $C_{32}H_{42}FN_5O_2$ | 70.17<br>69.94 | 7.73<br>7.80 | 12.79<br>12.74 |

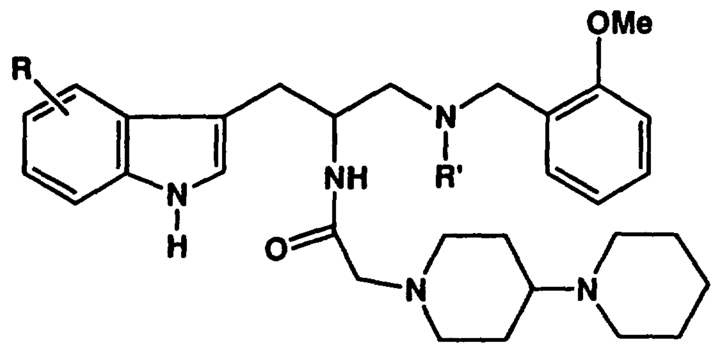

| Example No. | R | R' | Mp °C | MS | ¹H NMR | Formula | Analysis, Theory/Found C | H | N |
|---|---|---|---|---|---|---|---|---|---|
| 120 | 5-Br | H | oil | 596, 598 (M+1) for Br isotopes) | DMSO-d6 1.20-1.56 (m, 12H), 1.75-2.00 (m, 2H), 2.20-2.40 (m, 7H), 2.60-2.80 (m, 3H), 2.85 (d, J=6 Hz, 2H), 3.63 (br s, 2H), 3.74 (s, 3H), 4.10 (m, 1H), 6.83-6.93 (m, 2H), 7.10-7.23 (m, 3H), 7.23-7.30 (m, 2H), 7.45 (d, J=8 Hz, 1H), 7.55 (s, 1H), 11.10 (s, 1H) | $C_{31}H_{42}BrN_5O_2$ | 62.41<br>62.63 | 7.10<br>6.96 | 11.74<br>12.01 |

| Example No. | R | R' | Mp °C | MS | $^1$H NMR | Formula | Analysis, Theory/Found C | H | N |
|---|---|---|---|---|---|---|---|---|---|
| 121 | 5-OMe | H | oil | 547 (M+) | DMSO-d6 1.20-1.70 (m, 11H), 1.66-2.20 (m, 4H), 2.20-2.43 (m, 4H), 2.43-2.65 (m, 3H), 2.65-2.90 (m, 4H), 3.61 (s, 2H), 3.77 (s, 3H), 3.80 (s, 3H), 4.13 (m, 1H), 6.70 (m, 1H), 6.80-7.00 (m, 2H), 7.02 (s, 1H), 7.08 (s, 1H), 7.10-7.40 (m, 3H), 7.45 (d, J=8 Hz, 1H), 10.65 (s, 1H) | $C_{32}H_{45}N_5O_3$ | 70.17<br>70.29 | 8.28<br>8.09 | 12.79<br>12.56 |
| 122 | 5-$OCH_2Ph$ | H | oil | 624 (M+1+) | DMSO-d6 1.20-1.33 (m, 11H), 1.80-2.10 (m, 4H), 2.25-2.40 (m, 5H), 2.50-2.60 (m, 3H), 2.65-2.90 (m, 5H), 3.63 (s, 2H), 3.74 (s, 3H), 4.08 (m, 1H), 6.77 (d, J=2 Hz, 1H), 6.80-7.00 (m, 2H), 7.03 (s, 1H), 7.13-7.25 (m, 3H), 7.25-7.50 (m, 7H), 10.70 (s, 1H) | $C_{38}H_{49}N_5O_3$ | 73.16<br>73.45 | 7.92<br>7.92 | 11.23<br>11.14 |
| 123 | 6-F | H | foam | 536 (M+1) | $^1$H $CDCl_3$ 1.22-1.78 (m, 12H), 1.95-2.15 (m, 3H), 2.43-2.57 (m, 4H), 2.69-3.08 (m, 7H), 3.74-3.88 (m, 5H), 4.39 (m, 1H), 6.85-7.13 (m, 5H), 7.21-7.27 (m, 2H), 7.33 (d, J=4.9 Hz, 1H), 7.58 (m, 1H), 8.25 (s, 1H). | $C_{31}H_{42}FN_5O_2$ | 71.17<br>70.89 | 8.26<br>8.26 | 12.21<br>11.91 |
| 124 | 1-Me | MeCO | oil | 573 (M+) | DMSO-$d_6$ 3:2 mixture of amide rotamers 1.30-1.60 (m, 11H), 1.80-1.95 (m, 2H), 1.93 (s, 3/5•3H), 2.03 (s, 2/5•3H), 2.05 (m, 1H), 2.40 (br s, 3H), 2.50-2.86 (m, 6H), 3.14 (m, 1H), 3.67 (m, 1H), 3.68 (s, 3/5•6H), 3.71 (s, 2/5•6H), 4.23-4.56 (m, 3H), 6.79 (m, 1H), 6.86-7.28 (m, 5H), 7.34 (d, J=8Hz, 1H), 7.53 (m, 3/5•2H), 7.63 (m, 2/5•2H), 8.30 (s, 1H) | $C_{34}H_{47}N_5O_3$ | 71.17<br>71.30 | 8.25<br>7.97 | 12.21<br>12.09 |

| Example No. | R | R' | Mp °C | MS | $^1$H NMR | Formula | Analysis, Theory/Found C | H | N |
|---|---|---|---|---|---|---|---|---|---|
| 125 | 4-Me | MeCO | foam | 573 (M+) | $^1$H $CDCl_3$ 1.46 (m, 3H), 1.51-1.81 (m, 7H), 2.01-2.26 (m, 6H), 2.43-2.68 (m, 5H), 2.70-2.84 (m, 4H), 2.87 (s, 2H), 3.07-3.24 (m, 3H), 3.78 (s, 3H), 3.98 (dd, J=9.8, 13.6 Hz, 1H), 4.45-4.61 (m, 3H), 6.84 (m, 3H), 6.88-6.94 (m, 1H),7.03-7.10 (m, 2H), 7.15-7.39 (m, 3 H), 8.07 (s, 1H). | $C_{34}H_{47}N_5O_3$ | 71.17<br>70.84 | 8.26<br>8.26 | 12.21<br>11.91 |
| 126 | 5-Me | MeCO | foam | 573 (M+) | $^1$H $CDCl_3$ 1.25-1.72 (m, 11H), 1.99-2.17 (m, 6H), 2.46 (m, 7H), 2.75 (dd, J=1.4, 9.7 Hz, 1H), 2.86 (s, 2H), 2.91 (d, J=7.0 Hz, 1H), 2.99 (d, J=6.3 Hz, 1H), 3.14 (dd, J=4.7, 13.8 Hz, 1H), 3.77 (s, 3H), 3.96 (dd, J=10.1, 13.8 Hz, 1H), 4.49 (ABq, J=17.0 Hz, Δv=40.3 Hz, 2H), 4.54 (m, 1H), 6.82-6.89 (m, 3 H), 7.02 (m, 2H), 7.23 (d, H=8.1 Hz, 2H), 7.42 (m, 2H), 7.95 (s, 1H) | $C_{34}H_{47}N_5O_3$ | 71.17<br>71.45 | 8.26<br>8.33 | 12.21<br>11.96 |

| Example No. | R | R' | Mp °C | MS | $^1$H NMR | Formula | Analysis, Theory/Found C | H | N |
|---|---|---|---|---|---|---|---|---|---|
| 127 | 6-Me | MeCO | oil | 573 ($M^+$) | $^1$H $CDCl_3$ 1.25-1.40 (m, 2H), 1.40-1.52 (m, 3H), 1.52-1.80 (m, 6H), 2.02 (d, J=12 Hz, 2H), 2.09 (s, 3H), 2.46 (s, 3H), 2.46-2.60 (m, 5H), 2.75 (m, 1H), 2.86 (s, 2H), 2.90 (d, J=15 Hz, 1H), 2.95 (d, J=15 Hz, 1H), 3.15 (dd, J=9, 18 Hz, 1H), 3.70 (s, 3H), 3.95 (m, 1H), 4.44 (s, 1H), 4.50-4.60 (m, 2H), 6.80-6.93 (m, 3H), 6.93-7.00 (m, 2H), 7.14 (s, 1H), 7.25 (s, 1H), 7.42 (d, J=9 Hz, 1H), 7.53 (d, J=8 Hz, 1H), 8.03 (brs, 1H) | $C_{34}H_{47}N_5O_3$ | 71.17<br>70.99 | 8.26<br>8.05 | 12.21<br>12.41 |
| 128 | 7-Me | MeCO | foam | 573 ($M^+$) | $^1$H $CDCl_3$ 1.32-1.41 (m, 4 H), 1.45-1.66 (m, 6 H), 1.96-2.07 (m, 2 H), 2.09 (s, 3 H), 2.19 (m, 1 H), 2.48-2.58 (m, 8 H), 2.74 (m, 1 H), 2.81-3.07 (m, 4 H), 3.14 (dd, J=4.6,13.8 Hz, 1 H), 3.76 (s, 3 H), 3.97 (dd, J=10.2,13.8 Hz, 1 H), 4.47 (ABq, J=17.1 Hz, Δν=42.3 Hz, 2 H), 4.55 (m, 1 H), 6.78-6.87 (m, 3 H), 6.96-7.07 (m, 3 H), 7.23 (m, 1 H), 7.45 (d, J=8.6 Hz, 1 H), 7.51 (d, J=7.6 Hz, 1 H), 8.18 (s, 1 H). | $C_{34}H_{47}N_5O_3$ | 71.17<br>71.33 | 8.26<br>8.20 | 12.21<br>12.29 |
| 129 | 5-Br | MeCO | oil | 638, 640 (M+1$^+$'s for Br isotopes) Exact Mass FAB (M+1): theory 638.2706 found: 638.2729 | DMSO-d6 2:1 mixture of amide rotamers 1.20-1.60 (m, 3H), 1.60-1.90 (m, 6H), 1.95 (s, 2/3•3H), 2.07 (s, 1/3•3H), 1.90-2.07 (m, 3H), 2.55-2.90 (m, 5H), 2.90-3.20 (m, 4H), 3.20-3.50 (m, 3H), 3.62 (m, 1H), 3.73 (s, 3H), 4.20-4.42 (m, 3H), 6.85 (m, 1H), 6.90-7.00 (m, 2H), 7.10-7.30 (m, 4H), 7.50 (m, 1H), 7.70 (s, 2/3•1H), 7.75 (s, 1/3•1H), 11.10 (s, 1H) | $C_{33}H_{44}BrN_5O_3$ | | | |

| Example No. | R | R' | Mp °C | MS | $^1H$ NMR | Formula | Analysis, Theory/Found C | H | N |
|---|---|---|---|---|---|---|---|---|---|
| 130 | 5-OMe | MeCO | oil | 590 (M+1⁺) | DMSO-d6 3:2 mixture of amide rotamers 1.20-1.60 (m, 12H), 1.73-1.96 (m, 2H), 1.93 (s, 3/5•3H), 2.02 (s, 2/5•3H), 2.33-2.43 (m, 4H), 2.60-2.90 (m, 6H), 3.57 (m, 1H), 3.70 (s, 3H), 3.71 (s, 3H), 4.26-4.56 (m, 3H), 6.66 (d, J=6 Hz, 1H), 6.82 (m, 1H), 6.93 (m, 2H), 7.03 (s, 2H), 7.20 (m, 2H), 7.44 (d, J=6 Hz, 3/5•1H), 7.68 (d, J=6 Hz, 2/5•1H), 10.65 (s, 1H) | $C_{34}H_{47}N_5O_4$ | 69.24<br>69.52 | 8.03<br>8.14 | 11.87<br>11.92 |
| 131 | 5-$OCH_2Ph$ | MeCO | oil | 666 (M+1⁺) | DMSO-$d_6$ 1.16-1.80 (m, 12H), 1.90 (m, 6H), 2.20-2.43 (m, 3H), 2.53-2.90 (m, 6H), 3.16 (m, 1H), 3.43 (m, 1H), 3.60 (m, 1H), 3.70 (d, J=6 Hz, 3H), 4.20-4.60 (m, 3H), 6.73-6.88 (m, 3H), 6.88-7.00 (m, 2H), 7.04 (s, 1H), 7.15-7.26 (m, 3H), 7.26-7.40 (m, 3H), 7.40-7.53 (m, 2H), 10.70 (s, 1H) | $C_{40}H_{51}N_5O_4$ | 72.15<br>71.95 | 7.72<br>7.66 | 10.52<br>10.31 |
| 131a | 6-F | MeCO | foam | 577 ($M^+$) | $CDCl_3$ δ 1.32-1.46 (m, 4H), 1.58-1.66 (m, 6H), 1.97-2.08 (m, 2H), 2.11 (s, 3H), 2.19 (m, 1H), 2.49 (m, 5H), 2.72-3.04 (m, 5H), 3.13 (dd, J=4.5 Hz, Δυ=13.9 Hz, 1H), 3.76 (s, 3H), 3.97 (dd, J=10.3 Hz, Δυ=13.7 Hz, 1H), 4.47 (ABq, J=17.0 Hz, Δυ=42.7 Hz, 2H), 4.49 (m, 1H), 6.78-6.90 (m, 1H), 7.00 (s, 1H), 7.04 (d, 2.2 Hz, 1H), 7.23 (m, 1H), 7.47 (d, J=8.5Hz, 1H), 7.57 (dd, J=5.3Hz, Δυ=8.7Hz, 1H), 8.62 (s, 1H) | $C_{33}H_{44}FN_5O_3$ | 68.61<br>68.76 | 7.68<br>7.86 | 12.12<br>12.28 |

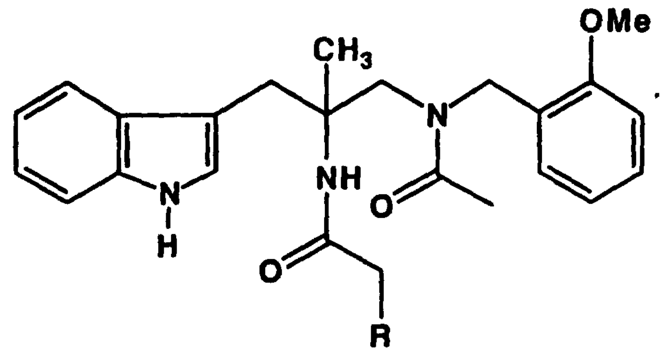

| Example No. | R | Mp °C | MS | $^1$H NMR | Formula | Analysis, %Theory/Found C | H | N |
|---|---|---|---|---|---|---|---|---|
| 132 | 1-(4-(1-piperidinyl)-piperidinyl) | foam | 574 (M+1$^+$) | $^1$H CDCl$_3$ 1.44 (s, 3H), 1.40-2.00 (m, 13H), 2.08 (s, 3H), 2.20-2.40 (m, 2H), 2.45-2.80 (m, 6H), 3.16-3.35 (m, 2H), 3.66 (d, J=14 Hz, 1H), 3.81 (s, 3H), 4.23 (d, J=14 Hz, 1H), 4.60 (ABq, J=14 Hz, Δυ =28 Hz, 2H), 6.86 (d, J=8 Hz, 1H), 6.96 (d, J=8 Hz, 1H), 7.03-7.20 (m, 4H), 7.27 (s, 2H), 7.40 (d, J=8 Hz, 1H), 7.60 (d, J=6 Hz, 2H) | $C_{34}H_{47}N_5O_3$ | 71.17<br>70.94 | 8.26<br>8.38 | 12.21<br>12.28 |
| 133 | 1-(4-phenyl)-piperazinyl | foam | 568 (M+1$^+$) | $^1$H CDCl$_3$ 1.56 (s, 3H), 2.09 (s, 3H), 2.43-2.85 (m, 3H), 2.85-3.20 (m, 7H), 3.20-3.50 (m, 3H), 3.81 (s, 3H), 4.20 (d, J=14 Hz, 1H), 4.60 (ABq, J=18 Hz, Δυ=56 Hz, 2H), 6.80-7.00 (m, 6H), 7.00-7.20 (m, 3H), 7.20-7.36 (m, 5H), 7.59 (d, J=7 Hz, 1H), 8.24 (s, 1H). | $C_{34}H_{41}N_5O_3$ | 71.93<br>71.68 | 7.28<br>7.49 | 12.34<br>12.29 |

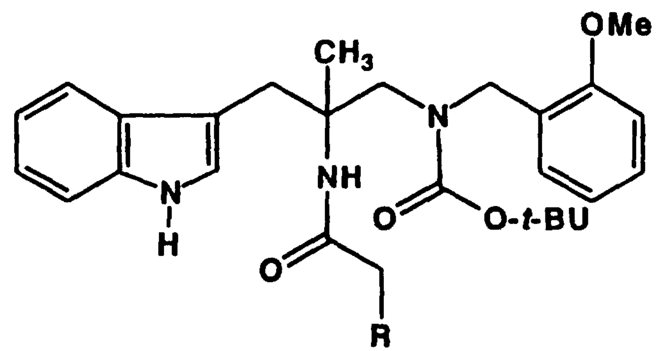

| Example No. | R | Mp °C | MS | $^1$H NMR | Formula | Analysis, %Theory/Found C | H | N |
|---|---|---|---|---|---|---|---|---|
| 134 | Br | foam | 543, 545 (M+'s for Br isotopes) | $^1$H $CDCl_3$ 1.31 (s, 12H), 3.07 (d, J=14 Hz, 1H), 3.25 (d, J=14 Hz, 1H), 3.40 (d, J=14 Hz, 1H), 3.66 (s, 3H), 3.68 (d, J=14 Hz, 1H), 3.80-3.95 (m, 2H), 4.23 (d, J=16 Hz, 1H), 4.64 (d, J=16 Hz, 1H), 6.82 (d, J=8 Hz, 1H), 6.90 (m, 1H), 7.00-7.15 (m, 2H), 7.15-7.30 (m, 3H), 7.30-7.40 (m, 2H), 7.55 (d, J=8 Hz, 1H), 8.07 (brs, 1H). | $C_{27}H_{34}BrN_3O_4$ | 59.56<br>58.80 | 6.29<br>6.21 | 7.72<br>7.47 |

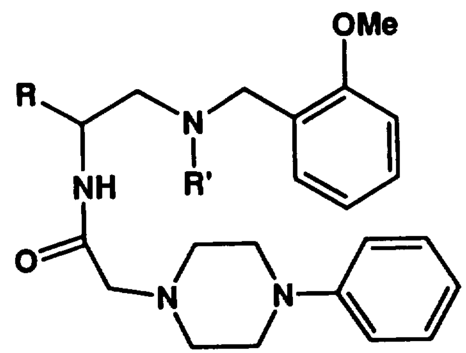

| Example No. | R | R' | Mp °C | MS | $^1$H NMR | Formula | Analysis, %Theory/Found C | H | N |
|---|---|---|---|---|---|---|---|---|---|
| 135 | 1-naphthyl-$CH_2$ | H | foam | 523 ($M+1^+$) | $CDCl_3$ 2.32-2.45 (m, 2H), 2.40 (m, 1H), 2.45-2.57 (m, 2H), 2.75-3.10 (m, 8H), 3.36 (m, 2H), 3.84 (s, 3H), 3.92 (ABq, J=12 Hz, Δv= 22 Hz, 2H), 4.48 (m, 1H), 6.75-7.00 (m, 5H), 7.15-7.42 (m, 6H), 7.42-7.64 (m, 3H), 7.74 (d, J=8 Hz, 1H), 7.83 (d, J=8 Hz, 1H), 8.28 (d, J=8 Hz, 1H) | $C_{33}H_{38}N_4O_2$ | 75.83<br>75.55 | 7.33<br>7.26 | 10.72<br>10.60 |
| 136 | 2-naphthyl-$CH_2$ | H | foam | 522 ($M^+$) | $CDCl_3$ 2.03 (m, 1H), 2.26-2.35 (m, 2H), 2.35-2.55 (m, 2H), 2.65-2.95 (m, 7H), 2.95-3.10 (m, 2H), 3.18 (dd, J=8, 14 Hz, 1H), 3.74-4.03 (m, 2H), 3.85 (s, 3H), 4.45 (m, 1H), 6.75 (d, J=9 Hz, 2H), 6.78-6.97 (m, 3H), 7.03-7.40 (m, 6H), 7.40-7.52 (m, 2H), 7.63 (s, 1H), 7.66-7.83 (m, 3H) | $C_{33}H_{38}N_4O_2$ | 75.83<br>76.07 | 7.33<br>7.25 | 10.72<br>10.66 |

| Example No. | R | R' | Mp °C | MS | $^1$H NMR | Formula | Analysis, %Theory/Found C | H | N |
|---|---|---|---|---|---|---|---|---|---|
| 137 | 3-indolinyl-$CH_2$ | H | foam | 514 (M+1$^+$) | DMSO-$d_6$ 1:1 mixture of diastereomers 1.54-1.70 (m, 1H), 1.86-1.98 (m, 1H), 2.52-2.64 (m, 6H), 2.84-3.18 (m, 8H) 3.32 (br s, 1H), 3.54 (m, 1H), 3.64-3.70 (m, 2H), 3.76 (s, 1/2•3H), 3.78 (s, 1/2•3H), 4.03 (m, 1H), 5.40 (br s, 1H), 6.44-6.56 (m, 2H), 6.77 (t, J=7 Hz, 1H), 6.82-6.98 (m, 6H), 7.10-7.24 (m, 3H), 7.30 (br d, J=8 Hz, 1H), 7.65 (t, J=9 Hz, 1H) | $C_{31}H_{39}N_5O_2$ | 72.48<br>72.57 | 7.65<br>7.50 | 13.63<br>13.70 |
| 138 | Ph | MeCO | oil | 500 ($M^+$) | $CDCl_3$ 2.14 (s, 3H), 2.60-2.80 (m, 4H), 3.00-3.20 (m, 2H), 3.20-3.43 (m, 5H), 3.82 (s, 3H), 4.30 (m, 1H), 4.40-4.63 (m, 2H), 5.18 (m, 1H), 6.80-7.06 (m, 6H), 7.03-7.40 (m, 8H), 8.24 (br s, 1H) | $C_{30}H_{36}N_4O_3$ | 71.97<br>71.67 | 7.25<br>7.29 | 11.19<br>11.18 |
| 139 | 3,4-diCl Ph | MeCO | oil | 568 ($M^+$) | $^1$H $CDCl_3$ 2.19 (s, 3H), 2.63-2.83 (m, 2H), 2.93-3.20 (m, 4H), 3.20-3.50 (m, 3H), 3.50-3.70 (m, 2H), 3.85 (s, 3H), 4.23 (m, 1H), 4.30-4.60 (m, 2H), 5.00 (m, 1H), 6.85-7.06 (m, 5H), 7.13 (m, 1H), 7.20-7.45 (m, 6H), 8.41 (br s, 1H). | $C_{30}H_{34}Cl_2N_4O_3$ | 63.27<br>63.12 | 6.02<br>5.82 | 9.84<br>9.55 |
| 140 | $PhCH_2$ | MeCO | oil | 514 ($M^+$) | DMSO-$d_6$ 3:2 mixture of amide rotamers 1.93 (s, 3/5•3H), 2.09 (s, 2/5•3H), 2.23-2.46 (m, 4H), 2.60-2.90 (m, 4H), 3.00-3.20 (m, 2H), 3.30-3.53 (m, 4H), 3.75 (s, 3H), 4.20-4.60 (m, 3H), 6.70-7.04 (m, 7H), 7.04-7.30 (m, 7H), 7.57 (d, J=9 Hz, 3/5•1H), 7.71 (d, J=9 Hz, 2/5•1H) | $C_{31}H_{38}N_4O_3$ | 72.35<br>72.57 | 7.44<br>7.47 | 10.89<br>10.69 |

| Example No. | R | R' | Mp °C | MS | $^1$H NMR | Formula | Analysis, %Theory/Found C | H | N |
|---|---|---|---|---|---|---|---|---|---|
| 141 | 1-naphthyl-$CH_2$ | MeCO | foam | 564 ($M^+$) | $CDCl_3$ 2.13 (s, 3H), 2.38-2.70 (m, 4H), 2.82-3.07 (m, 4H), 3.07-3.30 (m, 4H), 3.56 (dd, J=7, 14 Hz, 1H), 3.66 (s, 3H), 4.14 (m, 1H), 4.34 (ABq, J=16 Hz, Δv=58 Hz, 2H), 4.47 (m, 1H), 6.52-6.67 (m, 2H), 6.73 (d, J=8 Hz, 1H), 6.77-7.00 (m, 3H), 7.09-7.20 (m, 1H), 7.20-7.40 (m, 4H), 7.43-7.70 (m, 3H), 7.73 (d, J=8 Hz, 1H), 7.86 (d, J=8 Hz, 1H), 8.34 (d, J=8 Hz, 1H) | $C_{35}H_{40}N_4O_3$ | 74.44<br>74.50 | 7.14<br>7.25 | 9.92<br>9.94 |
| 142 | 2-naphthyl-$CH_2$ | MeCO | foam | 564 ($M^+$) | $CDCl_3$ 2.12 (s, 3H), 2.26-2.50 (m, 4H), 2.59-3.30 (m, 9H), 3.78 (s, 3H), 3.98 (m, 1H), 4.51 (ABq, J=17 Hz, Δv=30 Hz, 2H), 4.53 (m, 1H), 6.55-7.03 (m, 6H), 7.05-7.39 (m, 5H), 7.39-7.53 (m, 2H), 7.60 (m, 1H), 7.71-7.85 (m, 3H) | $C_{35}H_{40}N_4O_3$ | 74.44<br>74.46 | 7.14<br>7.31 | 9.92<br>9.94 |
| 143 | 3-benzo[b]thienyl-$CH_2$ | MeCO | foam | 571 ($M+1^+$) | $^1$H $CDCl_3$ 2.15 (s, 3H), 2.44-2.60 (m, 4H), 2.89-3.26 (m, 9H), 3.73 (s, 3H), 4.07 (dd, J=10.4,13.9 Hz, 1H), 4.43 (ABq, J=16.5 Hz, Δv=45.4 Hz, 2H), 4.50 (m, 1H), 6.74-6.92 (m, 6H), 7.15 (s, 1H), 7.18-7.30 (m, 3H), 7.39 (m, 2 H), 7.57 (d, J=8.1 Hz, 1H), 7.87 (d, J=7.4 Hz, 1H), 7.98 (d, J=7.6 Hz, 1H). | $C_{33}H_{38}N_4O_3S$ | 69.45<br>69.23 | 6.71<br>6.71 | 9.82<br>9.77 |

| Example No. | R | R' | Mp °C | MS | $^1$H NMR | Formula | Analysis, %Theory/Found C H N |
|---|---|---|---|---|---|---|---|
| 144 | 3-indolinyl-$CH_2$ | MeCO | 102-105 | 556 (M+1$^+$)Exact Mass FAB (M+1): calc.: 556.3287 found: 556.3280 | $CDCl_3$ 1:1 mixture of diastereomers 1.57-2.08 (m, 2H), 2.15 (s, 1/2•3H), 2.17 (s, 1/2•3H), 2.75-3.60 (m, 13H), 3.65-4.00 (m, 2H), 3.82 (s, 1/2•3H), 3.85 (s, 1/2•3H), 4.18-4.48 (m, 2H), 4.58 (s, 2H), 6.70-7.40 (m, 13H), 7.67 (m, 1H) | $C_{33}H_{41}N_5O_3$ | |
| 145 | N-Ac-3-indolinyl-$CH_2$ | MeCO | 80-84 | 597 (M$^+$) Exact Mass FAB (M+1): calc.: 598.3393 found: 598.3397 | $CDCl_3$ 1:1 mixture of diastereomers 1.70-2.00 (m, 2H), 2.13 (s, 1/2•3H), 2.17 (s, 1/2•3H), 2.23 (s, 1/2•3H), 2.27 (s, 1/2•3H), 2.57-3.53 (m, 12H), 3.63-4.03 (m, 2H), 3.82 (s, 1/2•3H), 3.85 (s, 1/2•3H), 4.03-4.33 (m, 2H), 4.52 (s, 1/2•1H), 4.54 (s, 1/2•1H), 6.80-7.40 (m, 12H), 7.57 (m, 1H), 8.19 (m, 1H) | $C_{35}H_{43}N_5O_4$ | |

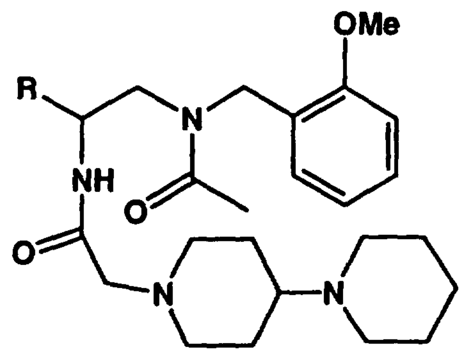

| Example No. | R | Mp °C | MS | $^1$H NMR | Formula | Analysis,% Theory/Found C | H | N |
|---|---|---|---|---|---|---|---|---|
| 146 | Ph | oil | 506 (M⁺) | DMSO-d6 2:1 mixture of amide rotamers 1.30-1.76 (m, 11H), 1.90-2.20 (m, 4H), 1.96 (s, 2/3•3H), 2.00 (s, 1/3•3H), 2.35-2.55 (m, 4H), 2.60-2.95 (m, 4H), 3.78 (s, 3H), 4.43 (s, 2/3•2H), 4.43 (ABq, J=15 Hz, Δv=49 Hz, 1/3•2H), 4.96 (m, 2/3•1H), 5.24 (m, 1/3•1H), 6.80-7.05 (m, 3H), 7.15-7.40 (m, 6H), 8.26 (d, J=9 Hz, 1H) | $C_{30}H_{42}N_4O_3$ | 71.11<br>71.38 | 8.35<br>8.25 | 11.06<br>11.07 |
| 147 | 3,4-diCl-Ph | oil | FD 574 ($M^+$) FAB Exact Mass Theory: 575.2555 Found: 575.2595 ($M+1^+$) | $^1$H CDCl3 1.40-1.60 (m, 2H), 1.60-1.80 (m, 4H), 1.80-2.05 (m, 5H), 2.17 (s, 3H), 2.18 (m, 1H), 2.40-2.80 (m, 5H), 2.80-3.05 (m, 5H), 3.85 (s, 3H), 4.23 (ABq, J=11 Hz, Δv=14 Hz, 1H), 4.48 (ABq, J=17 Hz, Δv=33 Hz, 2H), 4.93 (m, 1H), 6.85-7.10 (m, 4H), 7.20-7.40 (m, 3H), 8.35 (m, 1H) | $C_{30}H_{40}Cl_2N_4O_3$ | 62.60<br>63.05 | 7.01<br>6.91 | 9.73<br>9.78 |
| 148 | $PhCH_2$ | oil | 520 (M⁺) | DMSO 3:2 mixture of amide rotamers 1.30-1.63 (m10H), 1.73-2.00 (m, 3H), 1.88 (s, 3/5•3H), 2.07 (s, 2/5•3H), 2.40 (m, 3H), 2.55-2.80 (m, 4H), 3.15-3.50 (m, 5H), 3.76 (s, 3H), 4.20-4.60 (m, 3H), 6.80-7.00 (m, 3H), 7.05-7.30 (m, 6H), 7.49 (d, J=9 Hz, 3/5•1H), 7.62 (d, J=9 Hz, 2/5•1H) | $C_{31}H_{44}N_4O_3$ | 71.51<br>71.50 | 8.52<br>8.25 | 10.76<br>10.51 |

| Example No. | R | Mp °C | MS | $^1$H NMR | Formula | Analysis,% Theory/Found C | H | N |
|---|---|---|---|---|---|---|---|---|
| 149 | 3-benzo[b]thienyl-$CH_2$ | foam | 576 ($M^+$) | $^1$H $CDCl_3$ 1.41-1.73 (m, 9H), 2.00-2.21 (m, 7H), 2.41-2.48 (m, 4H), 2.59 (d, J=11.4 Hz, 1H), 2.74 (d, J=12.6 Hz, 1H), 2.88 (s, 3H), 3.04 (dd, J=4.3,13.9 Hz, 1H), 3.20 (dd, J=6.1,14.5 Hz, 1H), 3.70 (s, 3H), 4.04 (dd, J=10.5,13.9 Hz, 1H), 4.40 (ABq, J=16.5 Hz, Δv=46.1 Hz, 2H), 4.50 (m, 1H), 6.73 (m, 2H), 6.78 (d, J=8.2 Hz, 1H), 7.13 (s, 1H), 7.19 (m, 1H), 7.27 (m, 2H), 7.57 (d, J=8.1 Hz, 1H), 7.84 (d, J=7.5 Hz, 1H), 7.96 (d, J=7.6 Hz, 1H) | $C_{33}H_{44}N_4O_3S$ | 68.72<br>68.47 | 7.69<br>7.79 | 9.71<br>9.77 |

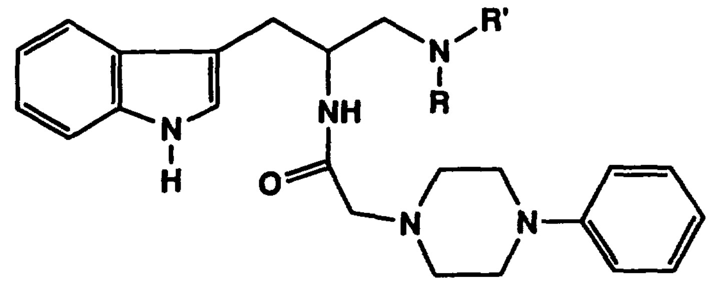

| Example No. | R | R' | Mp °C | MS | $^1$H NMR | Formula | Analysis % Theory/Found C | H | N |
|---|---|---|---|---|---|---|---|---|---|
| 150 | H | H | 144-145 | 391 (M+) | $CDCl_3$ 2.18-2.42 (m, 2H), 2.42-2.77 (m, 4H), 2.77-3.50 (m, 10H), 4.43 (m, 1H), 6.73-7.00 (m, 3H), 7.07-7.59 (m, 7H), 7.64 (d, J=8 Hz, 1H), 8.24 (br s, 1H) | $C_{23}H_{29}N_5O$ | 70.56<br>70.51 | 7.47<br>7.60 | 17.89<br>17.91 |
| 151 | t-Bu-O(CO) | H | 121-122 | 491 (M+) | $CDCl_3$ 1.63 (s, 9H), 2.22-2.67 (m, 4H), 2.75-3.23 (m, 8H), 3.30 (m, 1H), 3.40 (m, 1H), 4.41 (m, 1H), 5.03 (m, 1H), 6.75-7.00 (m, 4H), 7.07-7.70 (m, 6H), 7.65 (d, J=8 Hz, 1H), 8.18 (br s, 1H) | $C_{28}H_{37}N_5O_3$ | 68.40<br>68.16 | 7.59<br>7.56 | 14.25<br>14.05 |
| 152 | PhCO | H | 188-189 | 495 (M+) | $CDCl_3/DMSOd_6$ 1.90-2.74 (m, 6H), 2.74-3.40 (m, 4H), 3.11 (d, J=7 Hz, 2H), 3.58-3.82 (m, 2H), 4.55 (m, 1H), 6.63-6.96 (m, 3H), 7.00-7.53 (m, 10H), 7.68 (d, J=8 Hz, 1H), 7.60-8.00 (m, 3H), 9.28 (br s, 1H) | $C_{30}H_{33}N_5O_2$ | 72..70<br>72.46 | 6.71<br>6.71 | 14.13<br>13.84 |
| 153 | H | (c-hexyl)$CH_2$ | foam | 487 (M+) | $CDCl_3$ 0.73-1.41 (m, 6H), 1.41-2.08 (m, 8H), 2.10-3.38 (m, 14H), 4.56 (m, 1H), 6.81 (d, J=8 Hz, 1H), 6.81-6.97 (m, 4H), 7.02-7.40 (m, 4H), 7.57-7.73 (m, 2H), 8.10 (br s, 1H) | $C_{30}H_{41}N_5O$ | 73.88<br>73.60 | 8.47<br>8.36 | 14.36<br>14.24 |

| Example No. | R | R' | Purification | Yield % | Mp °C | MS | $^1$H NMR | Formula | Analysis, %Theory/Found C | H | N |
|---|---|---|---|---|---|---|---|---|---|---|---|
| 154 | t-Bu-O(CO)NH-$CH_2CO$ | (c-hexyl)$CH_2$ | chrom (EtOH/ EtOAc) | 84 mg 43% | foam | 644 ($M^+$) | $CDCl_3$ 0.75-1.00 (m, 2H), 1.00-1.94 (m, 10H), 1.44 (s, 9H), 2.40-2.65 (m, 3H), 2.65-3.66 (m, 11H), 3.76-4.20 (m, 3H), 4.60 (m, 1H), 5.54 (m, 1H), 6.75-7.05 (m, 3H), 7.05-7.46 (m, 7H), 7.67 (d, J=8 Hz, 1H), 8.13 (br s, 1H) | $C_{37}H_{52}N_6O_4$ | 68.92<br>68.93 | 8.13<br>8.28 | 13.03<br>13.11 |

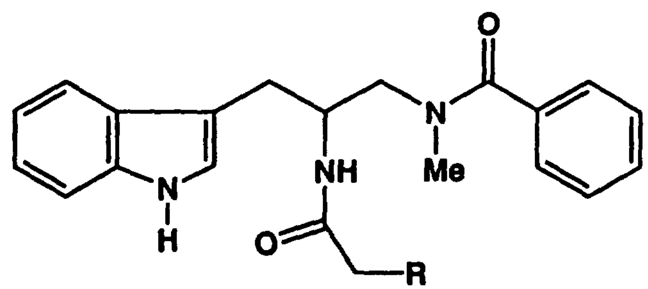

| Example No. | R | Mp °C | MS | $^1$H NMR | Formula | Analysis, %Theory/Found C | H | N |
|---|---|---|---|---|---|---|---|---|
| 155 | 1-(4-(1-piperidinyl)-piperidinyl | foam | 515 ($M^+$) | $CDCl_3$ 1.3-2.1 (m, 11H), 2.30 (m, 1H), 2.4-3.3 (m, 12H), 3.00 (s, 3H), 4.28 (m, 1H), 4.74 (m, 1H), 7.1- 7.5 (m, 10H), 7.68 (d, J=8 Hz, 1H), 8.83 (br s, 1H) | $C_{31}H_{41}N_5O_2$ | 72.20<br>72.12 | 8.01<br>8.22 | 13.58<br>13.82 |
| 156 | 1-(4-AcNH-4-Ph-piperidinyl) | 168-9 | 565 ($M^+$) | $CDCl_3$ 1.97 (s, 3H), 2.0-2.6 (m, 8H), 2.8-3.3 (m, 4H), 2.99 (s, 3H), 3.52 (m, 1H), 4.30 (m, 1H), 4.72 (m, 1H), 5.48 (m, 1H), 7.0-7.7 (m, 15H), 7.68 (m, 1H), 8.41 (br s, 1H) | $C_{34}H_{39}N_5O_3$ | 72.19<br>72.47 | 6.95<br>7.08 | 12.38<br>12.63 |

| Example No. | R | Mp °C | MS | $^1$H NMR | Formula | Analysis, %Theory/Found C | H | N |
|---|---|---|---|---|---|---|---|---|
| 157 | 1-(4-Ph-piperazinyl) | foam | 509 (M+) | $CDCl_3$ 2.3-2.7 (m, 3H), 2.7-3.7 (m, 10H), 3.02 (s, 3H), 4.30 (m, 1H), 4.78 (m, 1H), 6.7-6.9 (m, 3H), 7.1-7.5 (m, 12H), 7.70 (d, J=7 Hz, 1H), 8.22 (br s, 1H) | $C_{31}H_{35}N_5O_2$ | 73.06<br>72.91 | 6.92<br>6.96 | 13.74<br>13.70 |
| 158 | 1-(4-cyclohexyl-piperazinyl) | foam | 515 (M+) | $CDCl_3$ 1.0-1.3 (m, 6H), 1.6-2.0 (m, 4H), 2.2-2.6 (m, 9H), 2.9-3.2 (m, 5H), 2.99 (s, 3H), 4.38 (m, 1H), 4.75 (m, 1H), 7.1-7.5 (m, 10H), 7.69 (d, J=6 Hz, 1H), 8.23 (br s, 1H) | $C_{31}H_{41}N_5O_2$ | 72.40<br>72.20 | 8.00<br>8.01 | 13.66<br>13.58 |

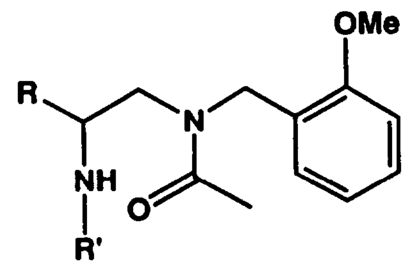

| Example No. | R | R' | Mp, °C | MS | $^1$H NMR | Formula | Analysis, % Theory/Found C | H | N |
|---|---|---|---|---|---|---|---|---|---|
| 159 | $PhCH_2$ | H | oil | 312 (M+) | $CDCl_3$ 3:1 mixture of amide rotamers 1.90-2.15 (m, 2H), 2.17 (s, 3/4•3H), 2.23 (s, 1/4•3H), 2.62 (dd, J=8, 13 Hz, 1H), 2.83 (dd, J=5, 13 Hz, 1H), 3.26-3.55 (m, 3H), 3.84 (s, 3H), 4.55 (d, J=14 Hz, 3/4•2H), 4.63 (d, J=11 Hz, 1/4•2H), 6.80-7.03 (m, 3H), 7.13-7.36 (m, 6H) | $C_{19}H_{24}N_2O_2$ | 73.05<br>72.82 | 7.74<br>7.68 | 8.97<br>8.80 |

| Example No. | R | R' | Mp, °C | MS | $^1$H NMR | Formula | Analysis, % Theory/Found C | H | N |
|---|---|---|---|---|---|---|---|---|---|
| 160 | 1-Me-3-indolyl-$CH_2$ | H | oil | 365 ($M^+$) | $CDCl_3$ 2.00-2.30 (m, 4H), 2.78 (dd, J=7, 15 Hz, 1H), 2.93 (m, 1H), 3.30-3.60 (m, 4H), 3.75 (s, 3H), 3.82 (s, 3H), 4.60 (ABq, J=16 Hz, Δv=30, 2H), 6.83-7.00 (m, 4H), 7.10 (m, 1H), 7.16-7.33 (m, 3H), 7.55 (m, 1H) | $C_{22}H_{27}N_3O_2$ | 72.30<br>72.02 | 7.45<br>7.43 | 11.50<br>11.24 |
| 161 | Ph | $BrCH_2CO$ | oil | 418, 420 ($M^+$'s for Br isotopes) | $CDCl_3$ 2.22 (s, 3H), 3.06 (dd, J=3, 14 Hz, 1H), 3.83 (s, 2H), 3.87 (s, 3H), 4.26 (dd, J=11, 15 Hz, 1H), 4.45 (ABq, J=17 Hz, Δv=62 Hz, 2H), 4.93 (m, 1H), 6.88-7.06 (m, 3H), 7.23-7.36 (m, 6H), 8.23 (d, J=6 Hz, 1H) | $C_{20}H_{23}BrN_2O_3$ | 57.29<br>57.24 | 5.53<br>5.48 | 6.68<br>6.49 |
| 161a | $PhCH_2$ | $BrCH_2CO$ | oil | 432, 434 ($M^+$'s for Br isotopes) | $CDCl_3$ 2.17 (s, 3H), 2.66 (dd, J=8, 14 Hz, 1H), 2.84 (dd, J=9, 14 Hz, 1H), 2.97 (dd, J=5, 14 Hz, 1H), 3.73-3.85 (m, 5H), 4.05 (m, 1H), 4.18 (m, 1H), 4.40 (ABq, J=16 Hz, Δv=39 Hz, 2H), 6.79-6.90 (m, 3H), 7.16-7.40 (m, 7H) | $C_{21}H_{25}BrN_2O_3$ | 58.21<br>58.28 | 5.81<br>5.80 | 6.46<br>6.32 |
| 162 | 1-Me-3-indolyl$CH_2$ | $BrCH_2CO$ | foam | 485, 487 ($M^+$'sfor Br isotopes), | $^1$H $CDCl_3$ 2.15 (s, 3H), 2.90 (dd, J=8, 14 Hz, 1H), 2.92 (dd, J=6, 14 Hz, 1H), 3.10 (dd, J=4, 14 Hz, 1H), 3.72 (s, 3H), 3.74 (s, 3H), 3.80 (s, 2H), 4.07 (m, 1H), 4.23-4.40 (m, 2H), 4.46 (m, 1H), 6.70-6.90 (m, 4H), 7.13 (d, J=8 Hz, 1H), 7.20-7.33 (m, 3H), 7.33 (d, J=12 Hz, 1H), 7.68 (d, J=8 Hz, 1H). | $C_{24}H_{28}BrN_3O_3$ | 59.26<br>59.50 | 5.80<br>5.76 | 8.64<br>8.52 |

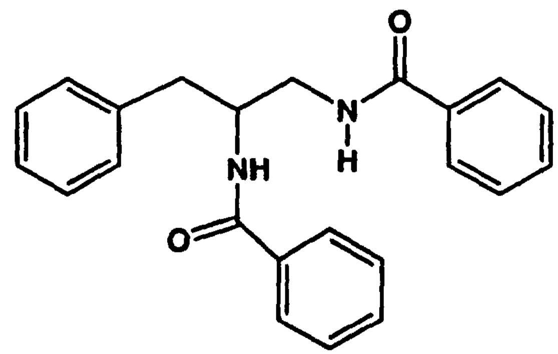

| Example No. | Mp, °C | MS | $^1$H NMR | Formula | Analysis, % Theory/Found C | H | N |
|---|---|---|---|---|---|---|---|
| 163 | 203-205 | 358 ($M^+$) | $CDCl_3$ 2.89 (dd, J=9, 14 Hz, 1H), 3.19 (dd, J=6, 14 Hz, 1H), 3.54 (dt, J=4, 14 Hz, 1H), 3.75 (m, 1H), 4.54 (m, 1H), 7.01 (m, 1H), 7.15 (m, 1H), 7.18-7.35 (m, 4H), 7.35-7.55 (m, 7H), 8.65-8.79 (m, 4H) | $C_{23}H_{22}N_2O_2$ | 77.07<br>76.83 | 6.19<br>6.21 | 7.81<br>7.88 |

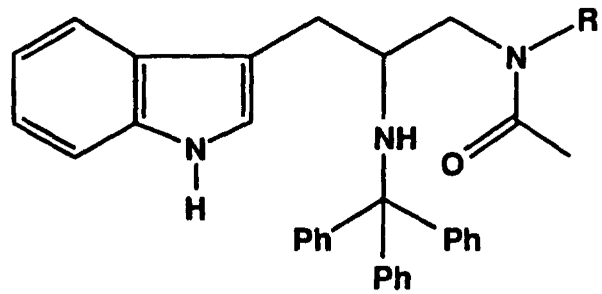

| Example No. | R | Mp, °C | MS | $^1$H NMR | Formula | Analysis, % Theory/Found C | H | N |
|---|---|---|---|---|---|---|---|---|
| 164 | Me | 183-184 | 488 (M+1⁺) | $CDCl_3$ 1.56 (s, 3H), 1.90 (m, 1H), 2.10 (m, 1H), 2.35 (m, 1H), 2.5-2.6 (br s, 3H), 2.75 (m, 1H), 2.95 (m, 1H), 3.20 (m, 1H), 6.9-7.1 (m, 2H), 7.1-7.6 (m, 17H), 7.85 (m, 1H), 7.96 (br s, 1H) | $C_{33}H_{33}N_3O$ | 81.28<br>81.26 | 6.82<br>6.91 | 8.62<br>8.71 |
| 165 | n-Bu | foam | 530 (M+1⁺) | $^1$H $CDCl_3$ 0.51-0.81 (m, 3H), 0.85-1.31 (m, 3H), 1.58 (s, 1H), 1.88 (s, 2H), 1.98 (s, 1H), 2.00-2.10 (m, 1H), 2.40-2.78 (m, 3H), 2.86-3.00 (m, 2H), 3.20-3.40 (m, 2H), 6.88 (s, 1H), 6.89-7.08 (m, 2H), 7.09-7.38 (m, 11H), 7.40-7.60 (m, 5H), 7.80-8.00 (m, 2H). | $C_{36}H_{39}N_3O$ | 81.63<br>81.90 | 7.42<br>7.44 | 7.93<br>8.03 |
| 166 | n-Hex | foam | 558 (M+1⁺) | $^1$H $CDCl_3$ 0.80-0.88 (m, 6H), 0.88-1.30 (m, 7H), 1.92 (s, 2H), 1.98 (s, 1H), 2.20-2.72 (m, 3H), 2.85-3.02 (m, 1H), 3.06-3.38 (m, 2H), 6.92 (s, 1H), 6.97-7.06 (m, 2H), 7.11-7.38 ( (m, 12H), 7.38-7.58 (m, 5H), 7.85-7.98 (m, 1H) | $C_{38}H_{43}N_3O$ | 81.83<br>82.10 | 7.77<br>7.74 | 7.53<br>7.24 |
| 167 | Ph | 182-183 | 550 (M+1⁺) | $^1$H DMSO 1.64 (s, 3H), 2.55 (m, 1H), 2.59-2.82 (m, 3H), 3.30 (m, 1H), 3.63 (dd, J=7, 14 Hz, 1H), 6.72 (d, J=2 Hz, 1H), 6.74-6.82 (m, 2H), 6.84 (t, J=8 Hz, 1H), 6.99 (t, J=8 Hz, 1H), 7.05-7.21 (m, 10H), 7.21-7.64 (m, 10H), 10.67 (br s, 1H). | $C_{38}H_{35}N_3O$ | 83.03<br>82.80 | 6.42<br>6.65 | 7.64<br>7.39. |

| Example No. | R | Mp, °C | MS | ¹H NMR | Formula | Analysis, % Theory/Found C | H | N |
|---|---|---|---|---|---|---|---|---|
| 168 | $PhCH_2CH_2$ | 174-175 | 577 ($M^+$) | ¹H DMSO (3:2 mixture of amide rotamers) 1.77 (s, 3/5•3H), 1.97 (s, 2/5•3H), 2.06-2.44 (m, 4H), 2.64-3.04 (m, 4H), 3.18 (m, 1H), 3.38-3.61 (m, 1H), 6.61-6.71 (m, 2H), 6.88 (m, 1H), 6.96-7.08 (m, 2H), 7.08-7.34 (m, 14H), 7.41-7.56 (m, 6H), 10.78 (br s, 1H). | $C_{40}H_{39}N_3O$ | 83.15<br>82.92 | 6.80<br>6.83 | 7.27<br>7.57 |

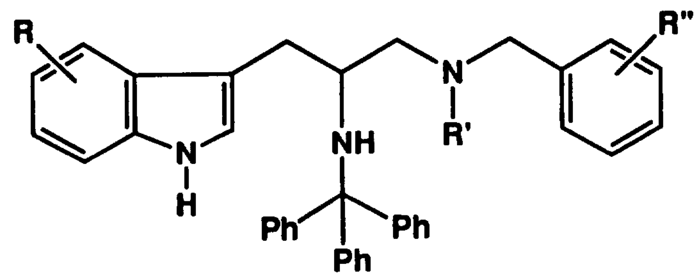

| Example No. | R | R' | R'' | Mp, °C | MS | ¹H NMR | Formula | Analysis, % Theory/Found C | H | N |
|---|---|---|---|---|---|---|---|---|---|---|
| 169 | 6-Me | H | 2-OMe | oil | 566 ($M+1^+$) | $CDCl_3$ 1.90 (m, 1H), 2.18-2.33 (m, 2H), 2.44 (s, 3H), 2.60 (m, 1H), 2.68-2.96 (m, 2H), 3.48-3.68 (m, 3H), 3.80 (s, 3H), 6.86 (d, J=8 Hz, 3H), 6.99-7.46 (m, 15H), 7.46-7.73 (m, 5H), 7.76 (s, 1H) | $C_{39}H_{39}N_3O$ | 82.80<br>82.81 | 6.95<br>7.02 | 7.43<br>7.32 |
| 170 | H | MeCO | 2-Cl | foam | 598 (M+1) | $CDCl_3$ 3:2 mixture of amide rotamers 1.80 (s, 3/5•3H), 2.05 (s, 2/5•3H), 2.30-2.53 (m, 2H), 2.65 (m, 1H), 3.00-3.33 (m, 3H), 3.91 (ABq, J=20 Hz, Δv=30 Hz, 3/5•2H), 4.61 (ABq, J=18 Hz, Δv=77 Hz, 2/5•2H), 6.58-6.67 (m, 3/5•1H), 6.80-6.89 (m, 2/5•1H), 6.94-7.33 (m, 18H), 7.42-7.56 (m, 5H), 7.86 (br s, 1H) | $C_{39}H_{36}ClN_3O$ | 78.37<br>78.10 | 6.07<br>6.25 | 7.02<br>6.78 |
| 171 | 6-Me | MeCO | 2-OMe | oil | 608 ($M+1^+$) | $CDCl_3$ 3:1 mixture of amide rotamers 1.92 (s, 3/4•3H), 1.97 (s, 1/4•3H), 2.44 (s, 3H), 2.56-2.76 (m, 2H), 3.04-3.36 (m, 4H), 3.62 (s, 1H), 3.72 (s, 3H), 4.03 (d, J=18 Hz, 1H), 6.43 (d, J=9 Hz, 1H), 6.58-7.00 (m, 4H), 7.00-7.28 (m, 11H), 7.40-7.60 (m, 7H), 7.74 (br s, 1H) | $C_{41}H_{41}N_3O_2$ | 81.02<br>80.90 | 6.80<br>6.66 | 6.91<br>7.16 |

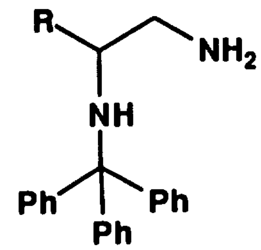

| Example No. | R | Mp, °C | MS | $^1$H NMR | Formula | Analysis, % Theory/Found C | H | N |
|---|---|---|---|---|---|---|---|---|
| 172 | 3,4-diCl-Ph | oil | 447 (M+1$^+$) | $^1$H CDCl$_3$ 1.50-1.95 (m, 2H), 2.04 (dd, J=6, 13 Hz, 1H), 2.52 (dd, J= 4, 12 Hz, 1H), 2.90 (m, 1H), 3.67 (m, 1H), 7.03 (m, 1H), 7.06-7.36 (m, 12H), 7.40-7.55 (m, 5H). | $C_{27}H_{24}Cl_2N_2$ | 72.48<br>72.45 | 5.41<br>5.38 | 6.26<br>6.02 |

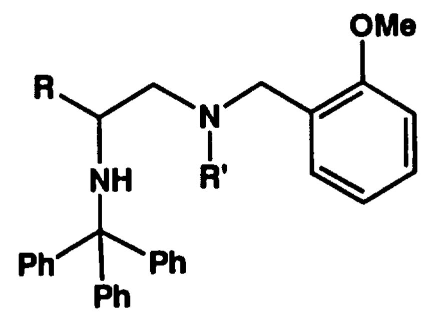

| Example No. | R | R' | Mp, °C | MS | $^1$H NMR | Formula | Analysis, % Theory/Found C | H | N |
|---|---|---|---|---|---|---|---|---|---|
| 173 | Ph | H | oil | 499 (M+1$^+$) | CDCl$_3$ 2.25-2.36 (m, 2H), 3.06 (m, 1H), 3.40-3.50 (m, 2H), 3.54 (s, 3H), 3.75-3.90 (m, 2H), 6.74 (d, J=8 Hz, 1H), 6.85 (m, 1H), 6.98 (m, 1H), 7.03-7.40 (m, 15H), 7.45-7.60 (m, 6H) | $C_{35}H_{34}N_2O$ | 84.30<br>84.47 | 6.87<br>6.87 | 5.62<br>5.74 |

| Example No. | R | R' | Mp, °C | MS | $^1$H NMR | Formula | Analysis, % Theory/Found C | H | N |
|---|---|---|---|---|---|---|---|---|---|
| 174 | $PhCH_2$ | H | oil | 513 ($M+1^+$) | $CDCl_3$ 1.93-2.10 (m, 2H), 2.20 (m, 1H), 2.23-2.40 (m, 2H), 2.60 (m, 1H), 2.75 (m, 1H), 3.55-3.65 (m, 2H), 3.82 (s, 3H), 6.83-6.98 (m, 4H), 7.03-7.40 (m, 14H), 7.53-7.66 (m, 6H) | $C_{36}H_{36}N_2O$ | 84.34<br>84.41 | 7.08<br>6.95 | 5.46<br>5.76 |
| 175 | Ph | MeCO | foam | 540 ($M^+$) | $CDCl_3$ 2:1 misture of amide rotamers 1.9 (s, 2/3•3H), 1.96 (s, 1/3•3H), 2.93 (m, 1H), 3.05 (m, 1H), 3.67 (s, 2/3•3H), 3.75 (s, 1/3•3H), 3.75 (m, 1H), 3.93 (d, J=18 Hz, 2H), 4.21 (ABq J=14 Hz, Δv=21 Hz, 1H), 6.66-6.90 (m, 3H), 6.90-7.35 (m, 15H), 7.35-7.55 (m, 6H) | $C_{37}H_{36}N_2O_2$ | 82.19<br>82.37 | 6.71<br>6.69 | 5.18<br>5.03 |
| 176 | 3,4-diCl-Ph | MeCO | 181-182.5 | 608 ($M^+$ for Cl isotope), Exact M.S. Theory: 609.2075, Found: 609.2053 | $^1$H $CDCl_3$ 1.99 (s, 3H), 2.96 (dd, J=6, 14 Hz, 1H), 3.12 (m, 1H), 3.60 (dd, J=8, 14 Hz, 1H), 3.81 (s, 3H), 3.90-4.16 (m, 3H), 6.73-6.96 (m, 4H), 6.96-7.30 (m, 12H), 7.30-7.49 (m, 6H) | $C_{37}H_{34}Cl_2N_2O_2$ | 72.90<br>73. 56 | 5.62<br>5.70 | 4.59<br>4.66 |
| 177 | $PhCH_2$ | MeCO | foam | 554 ($M^+$) | $CDCl_3$ 2:1 mixture of amide rotamers 1.90 (s, 2/3•3H), 1.95 (s, 1/3•3H), 2.36-2.53 (m, 2H), 2.63 (dd, J=4, 13 Hz, 1H), 3.00 (m, 1H), 3.06-3.23 (m, 2H), 3.66 (s, 1/3•3H), 3.76 (s, 2/3•3H), 3.85 (ABq, J=17 Hz, Δv=110 Hz, 2/3•2H), 4.59 (ABq, J=17 Hz, Δv=100 Hz, 1/3•2H), 6.42 (d, J=7 Hz, 1H), 6.68-6.85 (m, 3H), 6.92-7.05 (m, 2H), 7.05-7.43 (m, 12H), 7.50-7.63 (m, 6H) | $C_{38}H_{38}N_2O_2$ | 82.28<br>82.01 | 6.90<br>6.96 | 5.05<br>5.25 |

The biological activity of the compounds employed the present invention may be measured by a number of methods known to those skilled in the art, including those described in the patents and scientific literature described supra.

The growth hormone secretagogues of Formula I are useful in vitro as unique tools for understanding how growth

hormone secretion is regulated at the pituitary level. This includes use in the evaluation of many factors thought or known to influence growth hormone secretion, such as age, sex, nutritional factors, glucose, amino acids, fatty acids, as well as fasting and non-fasting states. In addition, the compounds employed in this invention can be used in the evaluation of how other hormones modify growth hormone releasing activity. For example, it has already been established that somatostatin inhibits growth hormone release.

Other hormones that are important and in need of study as to their effect on growth hormone release include the gonadal hormones, e.g., testosterone, estradiol, and progesterone; the adrenal hormones, e.g., cortisol and other corticoids, epinephrine and norepinephrine; the pancreatic and gastrointestinal hormones, e.g., insulin, glucagon, gastrin, secretin; the vasoactive peptides, e.g., bombesin, the neurokinins; and the thyroid hormones, e.g., thyroxine and triiodothyronine. The compounds of Formula I can also be employed to investigate the possible negative or positive feedback effects of the pituitary hormones, e.g., growth hormone and endorphin peptides, on the pituitary to modify growth hormone release. Of particular scientific importance is the use of these compounds to elucidate the subcellular mechanisms mediating the release of growth hormone.

The compounds of Formula I can be administered to animals, including man, to release growth hormone in vivo. For example, the compounds can be administered to commercially important animals such as swine, cattle, sheep, and the like to accelerate and increase their rate and extent of growth, to improve feed efficiency, and to increase milk production in such animals. In addition, these compounds can be administered to humans in vivo as a diagnostic tool to directly determine whether the pituitary is capable of releasing growth hormone. For example, the compounds of Formula I can be administered in vivo to children. Serum samples taken before and after such administration can be assayed for growth hormone. Comparison of the amounts of growth hormone in each of these samples would be a means for directly determining the ability of the patient's pituitary to release growth hormone.

The present invention generally employs at least one of the compounds of Formula I in association with a pharmaceutical carrier or diluent. Optionally, the active ingredient of the pharmaceutical compositions can comprise an anabolic agent in addition to at least one of the compounds of Formula I or another composition which exhibits a different activity, e.g., an antibiotic growth permittant or an agent to treat osteoporosis or in combination with a corticosteroid to minimize the catabolic side effects or with other pharmaceutically active materials wherein the combination enhances efficacy and minimizes side effects.

Growth promoting and anabolic agents include, but are not limited to, TRH, diethylstilbesterol, estrogens, β-agonists, theophylline, anabolic steroids, enkephalins, E series prostaglandins, compounds disclosed in United States Patent 3,239,345, e.g., zeranol, and compounds disclosed in United States Patent 4,036,979, e.g., sulbenox, or peptides disclosed in United States Patent 4,411,890.

A still further use of the growth hormone secretagogues of this invention is in combination with other growth hormone secretagogues such as the growth hormone releasing peptides GHRP-6, GHRP-1 as described in United States Patent 4,411,890 and Patent Cooperation Treaty Publications WO 89/07110 and WO 89/07111, as well as hexarelin and the newly discovered GHRP-2 as described in Patent Cooperation Treaty Publication WO 93/04081; or growth hormone releasing hormone and its analogs; or growth hormone and its analogs; or somatomedins including IGF-1 and IGF-2; or α-adrenergic agonists, such as clonidine; or serotonin 5-$HT_{1D}$ agonists, such as sumatriptan; or agents which inhibit somatostatin or its release, such as physostigmine and pyridostigmine.

As is well known to those skilled in the art, the known and potential uses of growth hormone are varied and multitudinous. The administration of the compounds employed in the present invention for the purpose of stimulating the release of endogenous growth hormone can have the same effects or uses as growth hormone itself. These varied uses of growth hormone may be summarized as follows: stimulating growth hormone release in elderly humans; treating growth hormone deficient adults; prevention of catabolic side effects of glucocorticoids; treatment of osteoporosis; stimulation of the immune system; acceleration of wound healing; accelerating bone fracture repair; treatment of growth retardation; treating acute or chronic renal failure or insufficiency; treatment of physiological short stature, including growth hormone deficient children; treating short stature associated with chronic illness; treatment of obesity and growth. retardation associated with obesity; treating growth retardation associated with Prader-Willi syndrome and Turner's syndrome; accelerating the recovery and reducing hospitalization of burn patients or following major surgery such as gastrointestinal surgery; treatment of intrauterine growth retardation, skeletal dysplasia, hypercortisonism, and Cushings syndrome; replacement of growth hormone in stressed patients; treatment of osteochondrodysplasias, Noonans syndrome, sleep disorders, Alzheimer's disease, delayed wound healing, and psychosocial deprivation; treatment of pulmonary dysfunction and ventilator dependency; attenuation of protein catabolic response after a major operation; treating malabsorption syndromes; reducing cachexia and protein loss due to chronic illness such as cancer or AIDS; accelerating weight gain and portion accretion in patients on total parenteral nutrition; treatment of hyperinsulinemia including nesidioblastosis; adjuvant treatment for ovulation induction and to prevent and treat gastric and duodenal ulcers; to stimulate thymic development and prevent the age-related decline of thymic function; adjunctive therapy for patients on chronic hemodialysis; treatment of immunosuppressed patients and to enhance antibody response following vaccination; improvement in muscle strength, mobility, maintenance of skin thickness, metabolic homeostasis, renal

homeostasis in the frail elderly; stimulation of osteoblasts, bone remodeling, and cartilage growth; treatment of neurological diseases such as peripheral and drug-induced neuropathy, Guillan-Barre syndrome, amyotrophic lateral sclerosis, multiple sclerosis, cerebrovascular accidents, and demyelinating diseases; stimulation of the immune system in companion animals and treatment of disorders of aging in companion animals; growth promotion in livestock; and stimulation of wool growth in sheep.

It will be understood by those skilled in the art that there are numerous compounds now being used in an effort to treat the diseases or therapeutic indications enumerated above. Combinations of these therapeutic agents, some of which have also been mentioned above, with the growth hormone secretagogues employed in this invention will bring additional, complementary; and often synergistic properties to enhance the growth promotant, anabolic, and desirable properties of these various therapeutic agents. In these combinations, the therapeutic agents and the growth hormone secretagogues of this invention may be independently present in dose ranges from one one-hundredth to one times the dose levels which are effective when these compounds and secretagogues are used singly.

The compounds of Formula I are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

The present invention also includes methods employing pharmaceutical compositions which contain, as the active ingredient, the compounds of Formula I associated with pharmaceutically acceptable carriers. In making the compositions of the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 5 to about 100 mg, more usually about 10 to about 30 mg, of the active ingredient. The term "unit dosage form" refers to physically discrete units suitable as unitary dosages dosages for human subjects and other mammals, each unit containing a predetermined quantity of active material calculated to produce the desired therapeutic effect, in association with a suitable pharmaceutical excipient.

The active compound is effective over a wide dosage range. For examples, dosages per day normally fall within the range of about 0.5 to about 30 mg/kg of body weight. In the treatment of adult humans, the range of about 1 to about 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for.administration throughout the day.

For preparing solid cmoposiions such as tablets the principal active ingredient is mixed with a pharmaceutical excipient to form a solid preformulation composition containing a homogeneous mixture of a compound of the present invention. When referring to these preformulation compositions as homogeneous, it is meant that the active ingredient is dipsersed evenly throughout the composition so that the composition may be readily subdivided into equally effective unit dosage forms such as tablets, pills and capsules. This solid preformulation is then subdivided into unit dosage forms of the type described above containing from 0.1 to about 500 mg of the active ingredient of the present invention.

The tablets or pills of the present invention may be coated or otherwise compounded to provide a dosage form

affording the advantage of prolonged action. For example, the tablet or pill can comprise an inner dosage and an outer dosage component, the latter being in the form of an envelope over the former. The two components can be separated by enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use of inert gases. Nebulized solutions may be breathed directly from the nebulizing device or the nebulizing device may be attached to a face mask, tent, or intermittent positive pressure breathing machine. Solution, suspension, or powder compsoitions may be administered, preferably orally or nasally, from devices which deliver the formulation in an appropriate manner.

The following examples illustrate the pharmaceutical compositions of the present invention.

Formulation Example 1

Hard gelatin capsules containing the following ingredients are prepared:

| Ingredient | Quantity (mg/capsule) |
|---|---|
| Compound of Example 51 | 30.0 |
| Starch | 305.0 |
| Magnesium stearate | 5.0 |

The above ingredients are mixed and filled into hard gelatin capsules in 340 mg quantities.

Formulation Example 2

A tablet formula is prepared using the ingredients below:

| Ingredient | Quantity (mg/tablet) |
|---|---|
| Compound of Example 66 | 25.0 |
| Cellulose, microcrystalline | 200.0 |
| Colloidal silicon dioxide | 10.0 |
| Stearic acid 5.0 | |

The components are blended and compressed to form tablets, each weighing 240 mg.

Formulation Example 3

A dry powder inhaler formulation is prepared containing the following components:

| Ingredient | Weight % |
|---|---|
| Compound of Example 17 | 5 |
| Lactose | 95 |

The active mixture is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
|---|---|
| Compound of Example 14 | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U. S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 150 mg.

Formulation Example 5

Capsules, each containing 40 mg of medicament are made as follows:

| Ingredient | Quantity (mg/capsule) |
|---|---|
| Compound of Example 13 | 40.0 mg |
| Starch | 109.0 mg |
| Magnesium stearate | 1.0 mg |
| Total | 150.0 mg |

The active ingredient, cellulose, starch, and magnesium stearate are blended, passed through a No. 20 mesh U. S. sieve, and filled into hard gelatin capsules in 150 mg quantities.

Formulation Example 6

Suppositories, each containing 25 mg of active ingredient are made as follows:

| Ingredient | Amount |
|---|---|
| Compound of Example 18 | 25 mg |
| Saturated fatty acid glycerides to | 2,000 mg |

The active ingredient is passed through a·No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 ml dose are made as follows:

| Ingredient | Amount |
|---|---|
| Compound of Example 43 | 50.0 mg |
| Xanthan gum | 4.0 mg |
| Sodium carboxymethyl cellulose (11%) Microcrystalline cellulose (89%) | 50.0 mg |

(continued)

| Ingredient | Amount |
|---|---|
| Sucrose | 1.75 g |
| Sodium benzoate | 10.0 mg |
| Flavor and Color | q.v. |
| Purified water to | 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Capsules, each containing 15 mg of medicament, are made as follows:

| Ingredient | Quantity (mg/capsule) |
|---|---|
| Compound of Example 58 | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U. S. sieve, and filled into hard gelatin capsules in 560 mg quantities.

Formulation Example 9

An intravenous formulation may be prepared as follows:

| Ingredient | Quantity |
|---|---|
| Compound of Example 91 | 250.0 mg |
| Isotonic saline | 1000 ml |

Formulation Example 10

A topical formulation may be prepared as follows:

| Ingredient | Quantity |
|---|---|
| Compound of Example 67 | 1-10 g |
| Emulsifying Wax | 30 g |
| Liquid Paraffin | 20 g |
| White Soft Paraffin | to 100 g |

The white soft paraffin is heated until molten. The liquid praffin and emulsifying wax are incorporated and stirred until dissolved. The compound of Example 67 is added and stirring is continued until dispersed. The mixture is then cooled until solid.

Formulation Example 11

Sublingual or buccal tablets, each containing 10 mg of active ingredient, may be prepared as follows:

| Ingredient | Quantity Per Tablet |
|---|---|
| Active Ingredient | 10.0 mg |
| Glycerol | 210.5 mg |
| Water | 143.0 mg |
| Sodium Citrate | 4.5 mg |
| Polyvinyl Alcohol | 26.5 mg |
| Polyvinylpyrrolidone | 15.5 mg |
| Total | 410.0 mg |

The glycerol, water, sodium citrate, polyvinyl alcohol, and polyvinylpyrrolidone are admixed together by continuous stirring and maintaining the temperature at about 90°C. When the polymers have gone into solution, the solution is cooled to about 50-55°C and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See, e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5,011,472, issued April 30, 1991, which is herein incorporated by refernce.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

## Claims

1. A compound of the formula

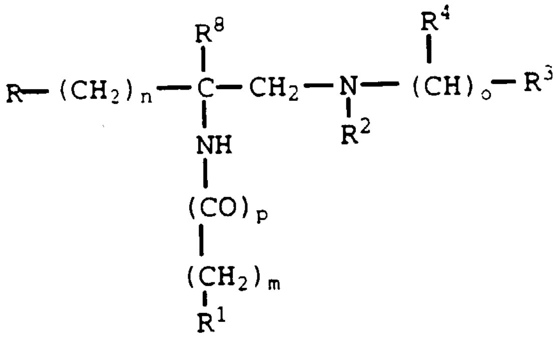

wherein

m is 0 or 1;

n is 0 or 1;

o is 0, 1, or 2;

p is 0 or 1;

R is phenyl, 2- or 3-indolyl, 2- or 3-indolinyl, benzothienyl, benzofuranyl, or naphthyl;
any one of which groups may be substituted with one or two halo, $C_1$-$C_3$ alkoxy, trifluoromethyl, $C_1$-$C_4$ alkyl, phenyl-$C_1$-$C_3$ alkoxy, or $C_1$-$C_4$ alkanoyl groups;

$R^1$ is trityl, phenyl, diphenylmethyl, phenoxy, phenylthio, hexamethyleneiminyl, piperazinyl, piperidinyl, pyrrolidinyl, morpholinyl, indolinyl, indolyl, benzothienyl, benzofuranyl, quinolinyl, tetrahydropyridinyl, isoquinolinyl, reduced quinolinyl, reduced isoquinolinyl, phenyl-($C_1$-$C_4$ alkyl)-, phenyl-($C_1$-$C_4$ alkoxy)-, quinolinyl-($C_1$-$C_4$ alkyl)-, isoquinolinyl-($C_1$-$C_4$ alkyl)-, reduced quinolinyl-($C_1$-$C_4$ alkyl)-, reduced isoquinolinyl-($C_1$-$C_4$ alkyl)-, benzoyl-($C_1$-$C_3$ alkyls, $C_1$-$C_4$ alkyl, or -NH-$CH_2$-$R^5$;
any one of which $R^1$ groups may be substituted with halo, $C_1$-$C_4$ alkyl, $C_1$-$C_4$ alkoxy, trifluoromethyl, amino, $C_1$-$C_4$ alkylamino, or di($C_1$-$C_4$ alkyl)amino; or any one of which $R^1$ groups may be substituted with phenyl, piperazinyl, $C_3$-$C_8$ cycloalkyl, benzyl, $C_1$-$C_4$ alkyl, piperidinyl, pyridinyl, pyrimidinyl, $C_2$-$C_6$ alkanoylamino, pyrrolidinyl, $C_2$-$C_6$ alkanoyl, or $C_1$-$C_4$ alkoxycarbonyl;
any one of which groups may be substituted with halo, $C_1$-$C_4$ alkyl, $C_1$-$C_4$ alkoxy, trifluoromethyl, amino, $C_1$-$C_4$ alkylamino, di($C_1$-$C_4$ alkyl)amino, or $C_2$-$C_4$ alkanoylamino;

or $R^1$ is amino, a leaving group, hydrogen, $C_1$-$C_4$ alkylamino, or di($C_1$-$C_4$ alkyl)amino;

$R^5$ is pyridyl, anilino-($C_1$-$C_3$ alkyl)-, or anilinocarbonyl;

$R^2$ is hydrogen, $C_1$-$C_4$ alkyl, arylsulfonyl, $C_1$-$C_4$ alkylsulfonyl, carboxy-($C_1$-$C_3$ alkyl)-, $C_1$-$C_3$ alkoxycarbonyl-($C_1$-$C_3$ alkyl)-, or -CO-$R^6$;

$R^6$ is hydrogen, $C_1$-$C_4$ alkyl, $C_1$-$C_3$ haloalkyl, phenyl, $C_1$-$C_3$ alkoxy, $C_1$-$C_3$ hydroxyalkyl, amino, $C_1$-$C_4$ alkylamino, di($C_1$-$C_4$ alkyl)amino, or $-(CH_2)_q-R^7$;

q is 0 to 3;

$R^7$ is phenoxy, phenylthio, piperazinyl, piperidinyl, pyrrolidinyl, morpholinyl, indolinyl, indolyl, benzothienyl, benzofuranyl, quinolinyl, isoquinolinyl, reduced quinolinyl, reduced isoquinolinyl, phenyl-($C_1$-$C_4$ alkyl)-, quinolinyl-($C_1$-$C_4$ alkyl)-, isoquinolinyl-($C_1$-$C_4$ alkyl)-, reduced quinolinyl($C_1$-$C_4$ alkyl)-, reduced isoquinolinyl-($C_1$-$C_4$ alkyl)-, benzoyl-$C_1$-$C_3$ alkyl;

any one of which $R^7$ groups may be substituted with halo, trifluoromethyl, $C_1$-$C_4$ alkoxy, amino, $C_1$-$C_4$ alkylamino, di($C_1$-$C_4$ alkyl)amino, or $C_2$-$C_4$ alkanoylamino;

or any one of which $R^7$ groups may be substituted with phenyl, piperazinyl, $C_3$-$C_8$ cycloalkyl, benzyl, piperidinyl, pyridinyl, pyrimidinyl, pyrrolidinyl, $C_2$-$C_6$ alkanoyl, $C_1$-$C_4$ alkyl, or $C_1$-$C_4$ alkoxycarbonyl;
any of which groups may be substituted with halo, trifluoromethyl, amino, $C_1$-$C_4$ alkoxy, $C_1$-$C_4$ alkyl, $C_1$-$C_4$ alkylamino, di($C_1$-$C_4$ alkyl)amino, or $C_2$-$C_4$ alkanoylamino;

or $R^7$ is carboxy, $C_1$-$C_4$ alkoxycarbonyl, $C_1$-$C_4$ alkylcarbonyloxy, amino, $C_1$-$C_4$ alkylamino, di($C_1$-$C_4$ alkyl)amino, $C_1$-$C_6$ alkoxycarbonylamino;
$R^8$ is hydrogen or $C_1$-$C_6$ alkyl;
$R^3$ is phenyl, phenyl-($C_1$-$C_6$ alkyl)-, $C_3$-$C_8$ cycloalkyl, $C_5$-$C_8$ cycloalkenyl, $C_1$-$C_8$ alkyl, naphthyl, $C_2$-$C_8$ alkenyl, or hydrogen;
any one of which groups except hydrogen may be substituted with one or two halo, $C_1$-$C_3$ alkoxy, $C_1$-$C_3$ alkylthio, nitro, trifluoromethyl, or $C_1$-$C_3$ alkyl groups;

and
$R^4$ is hydrogen or $C_1$-$C_3$ alkyl;
with the proviso that if $R^1$ is hydrogen or halo, $R^3$ is phenyl, phenyl-($C_1$-$C_6$ alkyls, $C_3$-$C_8$ cycloalkyl, $C_5$-$C_8$ cycloalkenyl, or naphthyl;
or a pharmaceutically acceptable salt or solvate thereof, for use in treating or preventing a physiological condition

which may be modulated by an increase in growth hormone.

2. A use as claimed in **Claim 1** employing a compound wherein R is phenyl, naphthyl, or 2- or 3-indolyl which group may be optionally substituted.

3. A use as claimed in **Claim 2** employing a compound of the formula

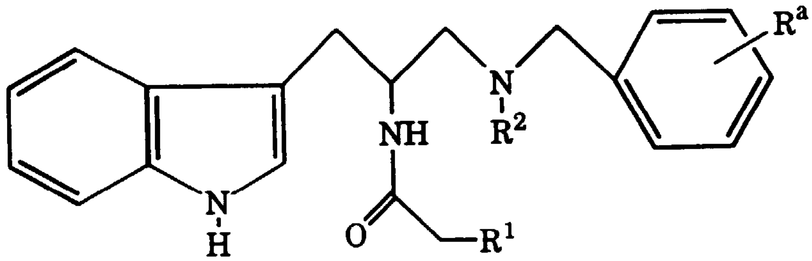

wherein $R^a$ is one, two or three substituents selected from the group consisting of halo, $C_1$-$C_3$ alkoxy, $C_1$-$C_3$ alkylthio, nitro, trifluoromethyl, and $C_1$-$C_3$ alkyl.

4. A use as claimed in **Claim 3** employing a compound wherein $R^1$ is piperazinyl, piperidinyl, substituted piperazinyl, or substituted piperidinyl.

5. A use as claimed in **Claim 4** employing a compound wherein $R^1$ is 1-(4-phenyl)piperazinyl.

6. A use as claimed in **Claim 5** employing (R) 1-[N-(2-methoxybenzyl)acetylamino]-3-(1H-indol-3-yl)-2-[N-(2-((4-phenyl)piperazin-1-yl)acetyl)amino]propane or (R) 1-[N-(2-chlorobenzyl)acetylamino]-3-(1H-indol-3-yl)-2-[N-(2-((4-phenyl)piperazin-1-yl)acetyl)amino]propane, or a pharmaceutically acceptable salt or solvate thereof.

7. A use as claimed in **Claim 4** employing a compound wherein $R^1$ is 1-(4-cyclohexyl)piperazinyl.

8. A use as claimed in **Claim 7** employing (R) 1-[N-(2-methoxybenzyl)acetylamino]-3-(1H-indol-3-yl)-2-[N-(2-((4-cyclohexyl)piperazin-1-yl)acetyl)amino]propane or (R) 1-[N-(2-chlorobenzyl)acetylamino]-3-(1H-indol-3-yl)-2-(N-(2-((4-cyclohexyl)piperazin-1-yl)acetyl)amino]propane, or a pharmaceutically acceptable salt or solvate thereof.

9. A use as claimed in **Claim 4** employing a compound wherein $R^1$ is 1-(4-phenyl)piperidinyl.

10. A use as claimed in **Claim 9** employing (R) 1-[N-(2-methoxybenzyl)acetylamino]-3-(1H-indol-3-yl)-2-[N-(2-((4-phenyl)piperidin-1-yl)acetyl)amino]propane or (R) 1-[N-(2-chlorobenzyl)acetylamino]-3-(1H-indol-3-yl)-2-[N-(2-((4-phenyl)piperidin-1-yl)acetyl)amino]propane, or a pharmaceutically acceptable salt or solvate thereof.

11. A use as claimed in **Claim 4** employing a compound wherein $R^1$ is 1-(4-cyclohexyl)piperidinyl.

12. A use as claimed in **Claim 11** employing (R) 1-[N-(2-methoxybenzyl)acetylamino]-3-(1H-indol-3-yl)-2-[N-(2-((4-cyclohexyl)piperidin-1-yl)acetyl)amino]propane or (R) 1-[N-(2-chlorobenzyl)acetylamino]-3-(1H-indol-3-yl)-2-[N-(2-((4-cyclohexyl)piperidin-1-yl)acetyl))amino]propane(R)-N-[2-[acetyl[(2-chlorophenyl)methyl]-amino]-1-(1H-indol-3-ylmethyl)ethyl)]-4-cyclohexyl-1-piperidineacetamide, or a pharmaceutically acceptable salt or solvate thereof.

13. A use as claimed in **Claim 4** employing a compound wherein $R^1$ is 1-[4-(1-piperidinyl)]piperidinyl.

14. A use as claimed in **Claim 13** employing (R) 1-[N-(2-methoxybenzyl)acetylamino]-3-(1H-indol-3-yl)-2-[N-(2-(4-(piperidin-1-yl)piperidin-1-yl)acetyl)amino]propane or (R) 1-[N-(2-chlorobenzyl)acetylanino]-3-(1H-indol-3-yl)-2-[N-(2-(4-(piperidin-1-yl)piperidin-1-yl)acetyl)amino]propane, or a pharmaceutically acceptable salt or solvate thereof.

15. A use as claimed in **Claim 14** employing the compound (R)-3-(1H-indol-3-yl)-1-[N-(2-methoxybenzyl)acetylamino]-2-[N-(2-(4-(piperidin-1-yl)piperidin-1-yl)acetyl)amino]propane dihydrochloride trihydrate.

**16.** A pharmaceutical formulation for use in treating or preventing a physiological condition which may be modulated by an increase in growth hormone, comprising a compound as claimed in any one of **Claims 1 to 15**, associated with one or more pharmaceutically acceptable carriers, diluents, or excipients therefor.

European Patent Office

# EUROPEAN SEARCH REPORT

Application Number
EP 96 30 5917

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.6) |
| X | WO-A-95 14017 (LILLY CO ELI) 26 May 1995 * page 126, line 19 - page 129, line 19 * * claims 1-14 * | 1-16 | A61K31/445 A61K31/495 |
| X | WO-A-93 01169 (MERCK SHARP & DOHME) 21 January 1993 * page 19, line 22 - page 21, line 17; claims 1-15 * | 1-3,16 | |
| A | | 4-15 | |
| X,P, D | WO-A-96 01819 (LILLY CO ELI) 25 January 1996 * page 45, line 5 - page 48, line 11; claims 1-3 * | 1-16 | |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| MUNICH | 22 November 1996 | Seegert, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)